(19) Europäisches Patentamt
European Patent Office
Office européen des brevets

(11) EP 2 136 853 B1

(12) **EUROPÄISCHE PATENTSCHRIFT**

(45) Veröffentlichungstag und Bekanntmachung des Hinweises auf die Patenterteilung:
**10.04.2013 Patentblatt 2013/15**

(21) Anmeldenummer: **08706776.5**

(22) Anmeldetag: **21.01.2008**

(51) Int Cl.:
***A61L 29/08*** (2006.01) ***A61L 29/16*** (2006.01)

(86) Internationale Anmeldenummer:
**PCT/DE2008/000096**

(87) Internationale Veröffentlichungsnummer:
**WO 2008/086794 (24.07.2008 Gazette 2008/30)**

(54) **MEDIZINPRODUKT ZUR BEHANDLUNG VON VERSCHLÜSSEN VON KÖRPERDURCHGÄNGEN UND ZUR PRÄVENTION DROHENDER WIEDERVERSCHLÜSSE**

MEDICAL PRODUCT FOR TREATING STENOSIS OF BODY PASSAGES AND FOR PREVENTING THREATENING RESTENOSIS

PRODUIT MÉDICAL DESTINÉ À TRAITER DES OCCLUSIONS DE VOIES CORPORELLES ET À PRÉVENIR LES RISQUES DE NOUVELLE FORMATION DE L'OCCLUSION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.01.2007 DE 102007003914**
**09.02.2007 DE 102007006557**
**21.03.2007 DE 102007013586**

(43) Veröffentlichungstag der Anmeldung:
**30.12.2009 Patentblatt 2009/53**

(60) Teilanmeldung:
**10075582.6 / 2 269 664**
**12168911.1 / 2 491 962**

(73) Patentinhaber: **Hemoteq AG**
**52146 Würselen (DE)**

(72) Erfinder:
- **HOFFMANN, Erika**
**52249 Eschweiler (DE)**
- **HORRES, Roland**
**52223 Stolberg (DE)**
- **FAUST, Volker**
**52080 Aachen (DE)**
- **SCHREIBER, Helmut**
**52146 Würselen (DE)**
- **VON HOLST, Armin**
**52066 Aachen (DE)**
- **HOFFMANN, Michael**
**52249 Eschweiler (DE)**

(74) Vertreter: **Arth, Hans-Lothar**
**ABK Patent Attorneys**
**Jasminweg 9**
**14052 Berlin (DE)**

(56) Entgegenhaltungen:
**WO-A-2004/028582 WO-A-2007/090385**
**US-A- 5 102 402**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kurzzeitig mit dem Organismus in Kontakt kommende Medizinprodukte wie beispielsweise Ballonkatheter, die mit mindestens einer Schicht, welche mindestens einen antiproliferativen, immunsuppressiven, antiangiogenen, antiinflammatorischen, fungiziden oder/und antithrombotischen Wirkstoff beschichtet sind, Verfahren zur Herstellung dieser wirkstofffreisetzenden Einführungshilfen und die Verwendung dieser Medizinprodukte zur Verhinderung von Wiederverschlüssen der betroffenen Körperdurchgänge.

Seit Ende der 80er Jahre des letzten Jahrhunderts haben sich zur Verhinderung der Restenose, also der Verhinderung eines Wiederverschlusses von Gefäßen, dem Körperdurchgang angepasste metallische rohrförmige Gefäßstützen immer mehr etabliert, die als Implantate von innen stützend gegen die Gefäßwände drücken. Die Weiterentwicklung dieser als Stents bekannten Implantate zum wirkstoffbeschichteten "Drug Eluting Stent" wird zur Zeit aufgrund positiver Ergebnisse bei der Minimierung der Restenoseraten im Vergleich zum unbeschichteten Stent mit Hochdruck verfolgt.

Diese Langzeitimplantate lösten immer mehr die seit den 60er Jahren durchgeführte PTCA (perkutane transluminale Angioplastie) ab und nehmen in der heutigen Zeit bereits den Großteil der durchgeführten Interventionen ein, da die Wiederverschlussraten eines unbeschichteten Stents in einigen Fällen geringer sind als die nach einer durchgeführten PTCA wiederkehrenden Verengungen.

Die beim Drug Eluting Stent realisierte und erfolgreiche Idee der Kombination von mechanischer und chemischer Prophylaxe wurde zur Geburtsstunde des Stents bereits an Ballonkathetern zur Verhinderung der Restenose von Koronararterien untersucht und in unterschiedlichen Varianten in Klinischen Studien angewendet.

Doch konnte sich der wirkstofftragende Ballonkätheter gegen den Stent nicht durchsetzen. Die Gründe liegen auf der Hand :

Bei der PTCA wird die verengte Stelle für eine kurze Zeit von 1-3 Minuten mit Hilfe des aufblasbaren Ballons am Ende des Katheters, wenn erforderlich mehr als zweimal wiederholt, aufgeweitet. Dabei müssen die Gefäßwände derart überdehnt werden, dass die Verengung behoben wird. Aus dieser Vorgehensweise resultieren Mikrorisse in den Gefäßwänden, die bis in die Adventitia reichen. Nach Entfernen des Katheters wird das verletzte Gefäß sich selbst überlassen, so dass dem Heilungsprozess in Abhängigkeit vom zugefügten Verletzungsgrad, der sich aus der Überdehnungsdauer, den Überdehnungswiederholungen und Überdehnungsgrad ergibt, mehr oder minder hochgradige Leistungen abverlangt werden. Dies zeigt sich in der hohen Wiederverschlussrate nach erfolgter PTCA.

Bei der Stentimplantation wird der Ballonkatheter als Transport- und Implantationshilfe eingesetzt, so dass es auch hier zu einer Überdehnung der Gefäßwand kommt, aber in diesem Fall wird diese Überdehnung nur für den Zeitraum der Stentdilatation benötigt. Sitzt der Stent an der richtigen Stelle unverrückbar fest, wird der Ballon wieder deflatiert und kann entfernt werden. Damit ist die Dauer der Überdehnung verkürzt und einmalig. Die Verringerung der Restenoserate zeigt, dass diese geringere Überdehnungszeit und der beim Stent ebenfalls verringerte Überdehnungsgrad trotz Einführung des Fremdmaterials in den Organismus bereits zu einer verminderten Quote in der Nachbehandlung führen kann. Diese vielversprechende Entwicklung ließ keinen nennenswerten Raum mehr für ein weiteres Interesse an der Optimierung der PTCA, da man mit dem Stent als permanentes Implantat einen hoffnungsvollen Träger der neuen möglichst restenosefreien Ära entdeckt zu haben glaubte und diese Technik bevorzugt behandelt hat und heute noch tut. Die PTCA wird nur in minder schweren Fällen angewendet und in besonders schweren Fällen einer Stentimplantation vorgeschaltet Das nächste Ziel in der Geschichte des Stents ist die 100%ige Verhinderung eines Wiederverschlusses. Hierzu hat die Suche nach der Kombination von idealem Wirkstoff und idealem möglichst bioabbaubaren Stent begonnen. Die Unterdrückung der zellulären Reaktionen in den ersten Tagen und Wochen wird dabei vorrangig mit Hilfe vorzugsweise antiproliferativer, immunsupressiver und/oder antiphlogistischer Wirkstoffe wie gleichermaßen wirkende Derivate/Analoga sowie Metaboliten erreicht. Dabei werden die Wirkstoffe und/oder Wirkstoffkombinationen in sinnvoller Weise zur Wundheilung bzw. den Wundheilungsverlauf unterstützend eingesetzt.

Die Verbesserungen, die in der zurückliegenden Zeit den Ballonkathetern widerfahren sind, bezogen und beziehen sich bislang vorrangig auf ihre Fähigkeiten, einen Stent präzise und sicher zu platzieren. Die PTCA als eigenständige Methode wurde von der Stentimplantation weitgehend verdrängt.

Doch bestehen bei der Anwendung der PTCA Vorteile gegenüber dem Stent, nicht zuletzt deshalb, weil sich auf diese Weise zu keinem Zeitpunkt nach Durchführung der Behandlung ein Fremdkörper als zusätzliche Belastung bzw. Initiator für Folgeerscheinungen wie sie auch die Restenose ist, im Organismus befindet. Deshalb gab und gibt es Anknüpfungen an die Ende der 80er Jahre durchgeführten Arbeiten eines wirkstofffreisetzenden Ballonkatheters.

So wurden beispielsweise verschiedenartige Ausführungsformen von Ballonkathetern beschrieben, deren mit der Umgebung in direktem Kontakt stehende Hülle über Löcher verfügt, durch die während der Dilatation unter Druck ein gelöster bzw. flüssiger Wirkstoff an die Gefäßwand gedrückt wird (z.B. in US5087244, US4994033, US4186745)

EP 0 383 429 A offenbart beispielsweise einen Ballonkatheter mit winzigen Löchern, durch die bei der Dilatation eine Heparinlösung an die Gefäßwand abgegeben wird.

Diverse Nachteile, wie die geringe Aufnahme des Wirkstoffes in die Gefäßwand, keine Kontrolle über die

Dosierung, Probleme mit dem Ballonmaterial etc. liessen diese Möglichkeit der fremdkörperfreien Behandlung von Stenosen in der Versuchsphase verbleiben. Die den Stents entsprechende Beschichtung von Ballons mit Wirkstoffen mit und ohne polymere Matrix führt ebenfalls zu Problemen, die zum einen in der Kürze des Kontaktes und der damit geringen Obertragbarkeit des Wirkstoffes von Katheter auf die Umgebung fußen und zum anderen in den nicht unerheblichen Schwierigkeiten, die Beschichtung auf dem Ballon vor und während der Dilatation unversehrt an den Zielort zu bringen.

Erst in jüngster Zeit bietet ein wirkstofffreisetzender Ballonkatheter eine Alternative zum Stent (CardioNews Letter 21.04.2006). Hierbei handelt es um einen in eine Lösung aus Paclitaxel und Röntgenkontrastmittel getauchten Ballonkatheter, der laut den Ergebnissen der nun einjährigen klinischen Studie im Vergleich zum unbeschichteten Ballonkatheter eine Erniedrigung der Restenoserate von 40 auf 9% erreicht hat. Ein solcher Ballonkatheter ist beispielsweise in WO2004028582A1 offenbart.

Auch wenn diese ersten Ergebnisse sich vielversprechend präsentieren, sind die typischen Probleme einer solchen Behandlung nicht ausgemerzt worden.

In jedem Fall ist die durch das Auftragen des Kontrastmittels erreichte optische Verfolgbarkeit vorteilhaft, doch bleibt die nach Durchführung der PTCA tatsächlich am Wirkort einsetzbare und aufgenommene Wirkstoffmenge individuell und unkontrolliert, da sich bereits während der Einführung des Ballonkatheters in der Blutbahn von der Leiste ausgehend bis zum Herzen ein nicht zu quantifizierender Teil der Beschichtung vom Ballon löst. Zudem bröckeln auch während der Dilatation des Ballons weitere Stücke der Beschichtung ab und werden von der Oberfläche weg im Blutstrom mitgerissen. Dies hat zur Folge, dass ein Teil der auf dem Ballonkatheter gebrachten Wirkstoffgehaltes nicht an die erkrankte Stelle gelangt, sondern lediglich als ineffektive, intravenöse Gabe angesehen werden können. Die Menge des verloren gehenden Anteils ist nicht kontrollierbar und steht damit nicht für eine optimale Versorgung an der erkrankten Stelle kalkulierbar zur Verfügung. Was auf dem Ballonkatheter verbleibt, muss also genügen, um eine erfolgsversprechende Therapie zu leisten, wobei sich umgekehrt die Frage stellt, wie viel des Wirkstoffes kommt tatsächlich am Ziel an und wird von der Gefäßwand aufgenommen.

Somit gilt es, die mit diesem Ballonkatheter aufgezeigte Möglichkeit der stentfreien Restenosebehandlung auf neue effektive und kontrollierbare Wege zu bringen.

Ferner besitzen die herkömmlich angewendeten Tauchbeschichtungsverfahren sowie Sprühbeschichtungsverfahren für Katheterballons den großen Nachteil, dass nie genau bestimmt werden kann, wieviel Wirkstoff tatsächlich auf die Ballonoberfläche aufgetragen worden ist, was dazu führt, dass grundsätzlich eine deutliche Überdosierung stattfindet. Zudem wird es aus regulatorischen und zulassungsrechtlichen Gründen immer wichtiger, wohldefinierte Ballonbeschichtungen bereitzustellen, worin die Wirkstoffmenge genau bestimmt wurde. Die herkömmlichen Verfahren, wobei der Katheterballon zumeist mehrmals in eine Beschichtungslösung getaucht wurde oder der Ballon sich in einem Sprühstrom oder Sprühnebel aus Beschichtungslösung befand, liefern keine reproduzierbaren Ergebnisse, so dass die Aufbringung einer definierten Wirkstoffmenge nicht möglich war.

Die Aufgabe der vorliegenden Erfindung besteht darin, Beschichtungsverfahren für Ballonkatheter bereitzustellen, wobei die Menge an aufgetragener Beschichtung und damit die Menge an aufgetragenem Wirkstoff genau bestimmt werden kann.

Eine weitere Aufgabe der vorliegenden Erfindung ist es, wirkstofffreisetzende Ballonkatheter und ähnliche kurzfristig im Körper einsetzbare Medizinprodukte bereitzustellen, welche schon bei kurzer Kontaktzeit eine kontrollierte und optimale Wirkstoffübertragung an und in die Gefäßwand gewährleisten, so dass der Heilungsprozess einen positiven Verlauf nimmt.

Hierzu muss gewährleistet sein, dass zum einen der Wirkstoff nicht bereits auf dem Wege zum Zielort vorzeitig durch Körperflüssigkeit vom Medizinprodukt gespült wird oder spätestens beim Expandieren abbröckelt und lediglich eine undefinierte bzw. ungenügende Menge an Wirkstoff das Ziel erreicht. Zum anderen muss die stark limitierte Kontaktzeit ausreichen, damit der Wirkstoff in vorgesehener Dosierung vom Ballonkatheter auf bzw. in die Gefäßwand übertragen werden kann.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Erfindungsgemäß wird die Aufgabe durch spezielle Beschichtungsverfahren für Katheterballons gelöst, welche den Katheterballon mit einer definierten Menge eines pharmakologischen Wirkstoffs beschichten, wobei das Beschichtungsverfahren eine Beschichtungsvorrichtung mit einer Volumenmesseinrichtung zur Abgabe einer messbaren Menge einer Beschichtungslösung mittels einer Abgabevorrichtung gezielt auf die Oberfläche des Katheterballons verwendet.

Als Volumenmesseinrichtung kann jede beliebige Vorrichtung dienen, welche in der Lage ist, eine abgemessene Menge Beschichtungslösung bereitzustellen oder die Menge an abgegebenen Beschichtungslösung zu messen oder anzuzeigen. Volumenmesseinrichtungen sind daher im einfachsten Fall Skalen, skalierte Pipetten, skalierte Büretten, skalierte Behälter, skalierte Kavitäten als auch Pumen, Ventile, Spritzen oder andere kolbenförmige Behälter, welche in der Lage sind eine abgemessene Menge an Beschichtungslösung bereitzustellen oder zu befördern oder auszugeben.

Somit dient die Volumenmesseinrichtung nur dazu, entweder eine bestimmte Menge einer Beschichtungslösung bereitzustellen oder abzugeben oder eine abgegebene Menge an Beschichtungslösung zu messen und/oder anzuzeigen. Die Volumenmesseinrichtung dient somit dazu die von der Abgabevorrichtung auf die Katheterballonoberfläche übertragene Menge an Beschichtungslösung und damit an Wirkstoff zu bestimmen bzw. zu messen.

Die Beschichtungslösung enthält zumindest einen pharmakologischen Wirkstoff zusammen mit mindestens einem Transportvermittler, Citratester, Kontrastmittel, Polymer, Polysaccharid, Peptid, Nukleotid, Öl, Fett, Wachs, Fettsäure, Fettsäureester, Hydrogel, Salz, Lösungsmittel, pharmakologisch verträglichen Hilfsstoff oder einer Mischung der vorgenannten Stoffe. Die möglichen Bestandteile der Beschichtungslösung sind hierin eingehend beschrieben.

Das wichtigste bei der Beschichtungsvorrichtung ist jedoch die Abgabevorrichtung, welche als Düse, Vielzahl von Düsen, Faden, Netz aus Fäden, ein Stück Textil, Lederstreifen, Schwamm, Kugel, Spritze, Nadel, Kanüle oder als Kapillare ausgestaltet sein kann. Je nachdem, welche Ausgestaltung die Abgabevorrichtung besitzt, ergeben sich etwas abgewandelte Beschichtungsverfahren, welche alle auf dem Grundprinzip beruhen, eine messbare oder vorbestimmte aber bekannte Menge an Wirkstoff auf die Katheterballonoberfläche zu übertragen, so dass sich eine Beschichtung mit definierter Wirkstoffkonzentration oder Wirkstoffmenge ergibt und man reproduzierbare Beschichtungen mit geringen Abweichungen zueinander bereitstellen kann, was die bisher eingesetzten Tauch- oder Sprühverfahren nicht vermögen. Zur Unterscheidung der Verfahren werden hierin verschiedene Begriffe benutzt, nämlich Spritzenverfahren, Pipettierverfahren, Kapillarverfahren, Faltensprühverfahren, Schleppverfahren, Fadenschleppverfahren oder Rollverfahren, welche die bevorzugten Ausführungsformen der vorliegenden Erfindung darstellen.

Nicht nur ein erfindungsgemäßes Verfahren sondern auch eine erfindungsgemäße Vorrichtung ergibt sich bei der Verwendung einer Kugel als Abgabevorrichtung. Das zugehörige Verfahren wird hierin als Rollverfahren bezeichnet und die zugehörige Vorrichtung besitzt einen Kugelkopf mit einer Zuleitung für eine Beschichtungslösung auf den Kugelkopf. Über eine Steuerung vorzugsweise optische Steuerung wird der Kugelkopf auf die Oberfläche des Katheterballons aufgesetzt. Über ein Ventil oder aufgrudn des Druckes von der Ballonoberfläche auf den Kugelkopf fließt die Beschichtungslösung aus einer Kavität oder einer Volumenmesseinrichtung aus und fließt auf den Kugelkopf. Der Kugelkopf wird über die Oberfläche des Katheterballons gerollt und fährt somit die Oberfläche des Katheterballons ab, wobei die auf den Kugelkopf gegebene Beschichtungslösung vom Kugelkopf auf die Oberfläche des Katheterballons übertragen wird.

Mit dieser Vorrichtung und mit diesem Rollverfahren lassen sich Katheterballons vollständig oder auch nur teilweise im deflatierten oder inflatierten Zustand beschichten. Z.B. kann im inflatierten oder teilweise inflatierten Zustand ein Katheterballon im Bereich der aufgeweiteten Falten gezielt abgefahren und beschichtet werden, wobei die Beschichtung nach erfolgter Deflation (d.h. zusammenfalten) innerhalb der Falten liegt, so dass man auf diese Weise auch eine gezielte Beschichtung der Falten bewerkstelligen kann. Damit der Kugelkopf den Ballon bzw. das Ballonmaterial nicht beschädigt, ist diese vorzugsweise aus einem gummiartigen Material wie z.B. Kautschuk oder anderen Polymeren.

Auf die einzelnen bevorzugten Beschichtungsverfahren wird weiter unten ausführlich eingegangen.

Die vorliegende Erfindung betrifft insbesondere beschichtete Katheterballons, welche eine wirkstofffreisetzende Beschichtung aufweisen.

Als Katheterballons können die herkömmlichen Katheterballons, Bifurkationsballons als auch Faltenballons oder Spezialballons eingesetzt werden.

Unter dem Begriff "Katheterballons" bzw. "herkömmliche Katheterballons" werden solche dilatierbare Katheterballons bezeichnet, welche in der Regel dazu dienen, mittels Dilatation einen Stent zu platzieren. Ferner werden so auch nicht dilatierbare Katheterballons für die Stentplatzierung bezeichnet, welche für selbstexpandierende Stents geeignet sind und zur Verhinderung der vorzeitigen Stentexpansion eine entfernbare Schutzhülle über dem Stent tragen.

Expandierbare und wieder komprimierbare Katheterballons mit einer Schutzhülle wie bei den nicht-dilatierbaren Katheterballons für selbstexpandierende Stents werden jedoch in der Regel ohne Stent eingesetzt, um die auf dem Katheterballon befindliche Beschichtung vor frühzeitiger Ablösung zu schützen.

Bifurkationsballons bezeichnet Katheterballons zur Behandlung einer Verzweigung eines Gefäßes insbesondere eines Blutgefäßes. Derartige Ballons können zwei Arme aufweisen oder aus zwei miteinander verbundenen oder aus zwei getrennten Ballons bestehen, welche gleichzeitig oder nacheinander zur Behandlung einer Gefäßgabelung bzw. zur Platzierung eines Stents oder zweier Stents in einer Gefäßgabelung oder in unmittelbarer Nähe zu einer Gefäßgabelung eingesetzt werden.

Als "Faltenballons" werden Ballons bezeichnet, wie sie beispielsweise in EP 1189553 B1, EP 0519063 B1, WO 03/059430 A1 und WO 94123787 A1 beschrieben sind und welche "Falten" im komprimierten Zustand des Ballons aufweisen, die sich bei der Expansion des Ballons zumindest teilweise öffnen.

Als Spezialballons werden Ballons mit Poren, insbesondere mit Mikroporen bezeichnet, welche bei der Expansion oder bei dem Anlegen von Druck den Durchtritt von Flüssigkeiten und Lösungen erlauben. Ein solcher Ballon mit Mikroöffnungen ist in EP 0 383 429 A offenbart. Ferner werden unter dem Begriff "Spezialballon" auch Ballons mit speziell ausgestalteter Oberfläche bezeichnet wie beispielsweise der in WO 02/043796 A2 beschriebene Katheterballon mit Mikronadeln oder der in WO 03/026718 A1 offenbart Katheterballon mit einer mirkorauen oder nanorauen Oberfläche

zur Einlagerung von Wirkstoffen mit oder ohne Trägersubstanzen.

Der Begriff "Ballon" oder "Katheterballon" bezeichnet grundsätzlich jede expandierbare und wieder komprimierbare sowie temporär implantierbare medizinische Vorrichtung, welche in der Regel zusammen mit einem Katheter verwendet wird.

Die erfindungsgemäßen beschichteten Ballons können ohne Stent als auch mit gekrimptem Stent eingesetzt werden. Ihr Einsatz bschränkt sich hierbei nicht nur auf eine Erstbehandlung von stenotischem Gefässen, sondern sind ebenfalls besonders gut geeignet, eine auftretende Re-Stenose (z.B. In-stent-Restenose) erfolgreich zu bekämpfen und eine wiederholte Verengung zu verhindern.

Der Katheterballon kann aus den gängigen Materialien, insbesondere Polymeren bestehen, wie sie weiter unter beschrieben werden und insbesondere aus Polyamid, wie z.B. PA 12, Polyester, Polyurethan, Polyacrylaten, Polyethern usw.

Der Stent kann ebenfalls aus den gängigen Materialien wie beispielsweise medizinischem Edelstahl, Titan, Chrom, Vanadium, Wolfram, Molybdän, Gold, Nitinol, Magnesium, Eisen, Legierungen der vorgenannten Metalle als auch aus polymerem Material und vorzugsweise resorbierbarem polymerem Material wie z.B. Chitosan, Heparanen, Polyhydroxybutyrate (PHB), Polyglyceriden, Polylactiden und Copolymeren der vorgenannten Stoffe.

Vorzugsweise werden die erfindungsgemäßen beschichteten Katheterballons ohne aufgesetzten Stent verwendet, jedoch ist eine Verwendung mit gekrimptem Stent ebenfalls möglich. Wird neben dem beschichteten Ballon ein darauf gekrimpter Stent verwendet, so kann der Stent unbeschichtet (bare stent) oder ebenfalls beschichtet sein, wobei der Stent eine andere Beschichtung und auch einen anderen Wirkstoff als die Beschichtung des Katheterballons aufweisen kann.

Der Begriff "Beschichtung" soll nicht nur eine Beschichtung der Oberfläche des Katheterballons sondern auch eine Befüllung oder Beschichtung von Falten, Kavitäten, Poren, Mikronadeln oder anderen befüllbaren Räumen auf oder zwischen oder in dem Ballonmaterial umfassen.

Die Beschichtung kann in einem oder mehren Schritten aufgebracht werden, einschichtig oder mehrschichtig sein, aus einem Material oder einer Mischung verschiedener Substanzen bestehen und enthält vorzugsweise einen oder auch mehrere Wirkstoffe. Als Wirkstoffe bzw. Wirkstoffkombinationen eignen sich antiinflammatorische, cytostatische, cytotoxische, antiproliferative, anti-mikrotubuli, antiangiogene, antirestenotische (anti-Restenose), antifungizide, antineoplastische, antimigrative, athrombogene und antithrombogene Substanzen.

Als antiinflammatorische, cytostatische, cytotoxische, antiproliferative, anti-mikrotubuli, antiangiogene, antirestenotische, antifungizide antineoplastische, antimigrative, athrombogene und antithrombogene Wirkstoffe können bevorzugt eingesetzt werden: Vasodilatoren, Sirolimus (Rapamycin), Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, 8-□-Ergoline, Dimethylergoline, Agroclavin, 1-Allylisurid, 1-Allyltergurid, Bromergurid, Bromocriptin (Ergotaman-3',6',18-trione, 2-bromo-12'-hydroxy-2'-(1-methylethyl)-5'-(2-methylpropyl)-, (5'alpha)-), Elymoclavin, Ergocristin (Ergotaman-3',6',18-trione, 12'-hydroxy-2'-(1-methylethyl)-5'-(phenylmethyl)-, (5'-alpha)-), Ergocristinin, Ergocornin (Ergotaman-3',6',18-trione, 12'-hydroxy-2',5'-bis(1-methylethyl)-, (5'-alpha)-), Ergocorninin, Ergocryptin (Ergotaman-3',6',18-trione, 12'-hydroxy-2'-(1-methylethyl)-5'-(2-methylpropyl)-, (5'alpha)- (9Cl)), Ergocryptinin, Ergometrin, Ergonovin (Ergobasin, INN: Ergometrin, (8beta(S))-9,10-Didehydro-N-(2-hydroxy-1-methylethyl)-6-methylergoline-8-carboxamid), Ergosin, Ergosinin, Ergotmetrinin, Ergotamin (Ergotaman-3',6',18-trione, 12'-hydroxy-2'-methyl-5'-(phenylmethyl)-, (5'-alpha)- (9Cl)), Ergotaminin, Ergovalin (Ergotaman-3',6',18-trione, 12'-hydroxy-2'-methyl-5'-(1-methylethyl)-, (5'alpha)-), Lergotril, Lisurid (CAS-Nr.: 18016-80-3, 3-(9,10-Didehydro-6-methylergolin-8alpha-yl)-1,1-diethylharnstoff), Lysergol, Lysergsäure (D-Lysergsäure), Lysergsäureamid (LSA, D-Lysergsäureamid), Lysergsäurediethylamid (LSD, D-Lysergsäurediethylamid, INN: Lysergamid, (8ß)-9,10-Didehydro-N,N-diethyl-6-methyl-ergoline-8-carboxamid), Isolysergsäure (D-Isolysergsäure), Isolysergsäureamid (D-Isolysergsäureamid), Isolysergsäurediethylamid (D-Isolysergsäurediethylamid), Mesulergin, Metergolin, Methergin (INN: Methylergometrin, (8beta(S))-9,10-Didehydro-N-(1-(hydroxymethyl)propyl)-6-methyl-ergoline-8-carboxamid), Methylergometrin, Methysergid (INN: Methysergid, (8beta)-9,10-Didehydro-N-(1-(hydroxymethyl)propyl)-1,6-dimethyl-ergoline-8-carboxamid), Pergolid ((8ß)-8-((Methylthio)methyl)-6-propyl-ergolin), Protergurid und Tergurid, Celecoxip, Thalidomid, Fasudil®, Cyclosporine, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon ($\alpha$-MSH),

aktiviertes Protein C, IL1-β-inhibitor, Thymosin α-1, Fumarsäure und deren Ester, Calcipotriol, Tacalcitol, Lapachol, ß-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1, 11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A und B, Paclitaxel und dessen Derivate wie 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor $X_a$-Inhibitor Antikörper, Interleukininhibitoren, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vasiprost, Interferon α, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1, B2, B6 und B12, Folsäure, Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-AII, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze, Hydrate, Solvate, Enantiomere, Racemate, Enantiomerengemische, Diastereomerengemische; Metaboliten, Prodrugs und Mischungen der vorgenannten Wirkstoffe.

Grundsätzlich können beliebige Wirkstoffe als auch Wirkstoffkombinationen eingesetzt werden, wobei jedoch Paclitaxel und Paclitaxel-Derivate, Taxane, Docetaxel sowie Rapamycin und Rapamycin-Derivate wie z.B. Biolimus A9, Pimecrolimus, Everolimus, Zotarolimus, Tacrolimus, Fasudil und Epothilone bevorzugt sind und insbesondere bevorzugt sind Paclitaxel und Rapamycin.

Paclitaxel ist unter dem Markennamen Taxol® und dem chemischem Namen [2aR-[2a,4,4a.6,9

(R*,S*),11,12,12a,12b]] - (Benzoylamino) - hydroxybenzolpropionsäure-6,12b-bis-(acetyloxy)-12-(benzoyloxy)-2a-3, 4, 4a, 5, 6, 9, 10, 11, 12, 12a, 12b-dodecahydro-4,11-dihydroxy-4a,8,13,13-tetramethyl-5-oxo-7,11-methano-1H-cyclodeca [3,4] benz[1,2-b] oxet-9-yl-ester) bekannt.

Rapamycin ist auch unter Rapamun oder dem International Nonproprietary Name (INN) Sirolimus sowie dem IUPAC-Namen [3S-[3R*[E(1S*,3S*,4S*)],4S*,5R*,8S*,9E,12R*,14R*, 15S*,16R*,18S*,19S*,26aR*]]-5,6,8,11,12,13,14, 15,16,17,18,19,24,25,26,26a-hexadeca-hydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-c][1,4]-oxaazacyclo-tricosin-1,7,20,21(4H,23H)-tetron-monohydrat bekannt.

Unter "Produgs" wird eine Vorstufe der pharmakologisch aktiven Verbindung verstanden, welche unter physiologischen Bedingungen in die aktive Verbindung umgewandelt wird.

Diese Wirkstoffe oder Wirkstoffkombinationen gelangen vorzugsweise über einen Transportvermittler oder als ihr eigener Transportvermittler in einer genügenden Konzentration während der limitierten Kontaktzeit des Kurzzeitimplantates an den Wirkort.

Wie bereits erwähnt, besteht ein großes Problem bei den Ausführungsformen des Standes der Technik darin, bei einer Dilatationszeit von maximal 1 Minute und eventuell mehrerer Wiederholungen der Dilatation nach einer gewissen Pause und vorzugsweise maximal 45 Sekunden und insbesondere bevorzugt von maximal 30 Sekunden genügend Wirkstoff auf den stenotischen oder re-stenotischen oder thrombotischen Gefäßabschnitt zu übertragen, so dass auch bei einer Dilatation ohne einen Stent zu setzen, eine Wiederverengung oder ein Wiederverschluss des Gefäßabschnitts verhindert wird. Da bei höheren Kontaktzeiten, d.h. Dilatationszeiten das Herzinfarktrisiko steigt, bleibt somit nur sehr wenig Zeit für die Übertragung des oder der Wirkstoffe auf die bzw. in die Gefäßwand. Zudem ist bei dem sogenannten "biological stenting" ohne Stent auch eine mehrmaliges Expandieren und Komprimieren des Katheterballons, um zwischenzeitlich zumindest einen geringen Blutfluß zu gewährleisten, kritisch, da der Wirkstoff größtenteils bereits bei der ersten Expansion des Katheterballons freigesetzt wird und weitere Dilatationen nicht mehr zu einer nennenswerten Wirkstoffübertragung auf die Gefäßwand beitragen können.

Somit bedarf es besonderer Beschichtungen, welche in relativ kurzer Zeit kontrolliert relativ viel Wirkstoff auf die Gefäßwand und/oder in die Gefäßwand übertragen.

**Transportvermittler**

Zur Steigerung der Wirkstoffübertragung werden bevorzugt sogenannte Transportvermittler bzw. Transportbeschleuniger eingesetzt, welche jedoch auch gleichzeitig den Wirkstoff darstellen können.

Von besonderem Interesse sind die erfindungsgemäßen Ausführungsformen, welche niedermolekulare chemische Verbindungen als Transportvermittler enthalten, die die Aufnahme von Wirkstoffen in die Gefäßwand beschleunigen bzw. erleichtern, so dass der vorhandene Wirkstoff bzw. die Wirkstoffkombination während des kurzzeitigen Kontaktes kontrolliert und in der vorgesehenen Dosierung durch die Zellmembran in das Zellinnere geschleust werden kann.

Dabei kann der Transportbeschleuniger auch als Träger fungieren. Hierbei sind verschiedene Möglichkeiten gegeben: die Bindung zwischen Wirkstoff und Träger besteht bereits und wird nach Eintritt in die Zelle gelöst oder sie wird an der Aussenseite der Membran für die Zeit des Membrandurchganges gebildet und anschliessend wieder gelöst oder Träger und Wirkstoff bilden eine Einheit, die auch im Zellinneren besteht, allerdings ohne die Effektivität des Wirkstoffes negativ zu beeinträchtigen.

Derartige Eigenschaften besitzen Substanzen, die entweder direkt mit der Lipiddoppelschicht der Zellmembran wechselwirken, mit Rezepturen auf der Zellmembran interagieren, über Membrantransportproteine, die als Carrier oder als Kanal (Ionenpumpe) wirken, in das Zellinnere gelangen, wo sie das Membranpotential und damit die Membranpermeabilität der Zellen verändern. Dadurch wird die Aufnahme eines Wirkstoffs in die Zellen erleichtert bzw. beschleunigt.

In erster Linie steht die Fähigkeit von Substanzen durch eine Membran in die Zelle zu diffundieren in direktem Zusammenhang mit der Grösse der Substanz. Kleinere Moleküle sind leichter durchgängig als größere. Moleküle, die weniger Wasserstoffbrücken-bindungen eingehen, diffundieren dementsprechend ebenfalls schneller als Moleküle, die bereitwillig Wasserstoffbrücken ausbilden. Ebenso ist die Polarität des Moleküls von Bedeutung. Unter Berücksichtigung dieser Sachverhalte lässt sich eine Reihe von synthetischen, semisynthetischen und nativen Substanzen nutzen, die Permeabilität einer Zellmembran derart zu verändern, dass das Eindringen eines Wirkstoffes in optimaler Weise erfolgt.

Zu solcherart nutzbaren Verbindungen gehören beispielsweise die Vasodilatoren, zu denen körpereigene Substanzen wie die Kinine, z:B. Brädykinin, Kallidin, Histamin, und die NOS-Synthase, die aus L-Arginin das vasodilatatorisch wirkende NO freisetzt, zählen. Substanzen pflanzlichen Ursprungs wie der nachweislich vasodilatatorisch, wirkenden Extrakt des Gingko biloba, DMSO, Xanthone, Flavonoide, Terpenoide, pflanzliche und tierische Farbstoffe, Lebensmittelfarben, NO-freisetzende Substanzen wie z.B. das Pentaerythrityltetranitrat (PETN), Kontrastmittel und Kontrastmittelanaloga zählen ebenfalls zu dieser Kategorie.

Es ergeben sich somit zwei auch miteinander kombinierbare Möglichkeiten den Transport eines oder mehrerer

Wirkstoffe in die Zellen zu unterstützen:

1. Der Transportbeschieuniger bzw. -vermittler bewirkt eine sofortige durch die Kontaktzeit mit dem Medizinprodukt begrenzte Überführung der Wirkstoffe in die Zelle.
2. Der Transportbeschleuniger bzw. vermittler haftet nach Entfernung des Medizinproduktes in Kombination mit dem Wirkstoff und eventuell mit einem die Haftung unterstützenden Träger (bzw. Reservoir) an den Zellwänden. Dadurch kann die Wirkstoffdiffusion in die Zellen retardiert und dosiskontrolliert erfolgen.

Transportvermittler, Wirkstoff bzw. Wirkstoffkombination als auch eine mögliche Matrix können adhäsiv oder/und kovalent, partiell oder vollflächig auf dem Medizinprodukt aufgebracht sein:

1. Transportvermittler und Wirkstoff haften adhäsiv und/oder kovalent auf dem Medizinprodukt oder auf einer auf dem Medizinprodukt adhäsiv oder kovalent aufgebrachten Matrix.
2. Transportvermittler und Wirkstoff sind kovalent aneinander gebunden und haften adhäsiv auf dem Medizinprodukt oder auf einer Matrix, die adhäsiv oder kovalent auf dem Medizinprodukt aufgebracht ist.
3. Transportvermittler und Wirkstoff sind kovalent aneinander gebunden und haften kovalent auf dem Medizinprodukt oder auf einer Metrix, die adhäsiv oder kovalent auf dem Medizinprodukt aufgebracht ist.

Neben den oben genannten Substanzen sind weitere für die Transportvermittlung geeignete Substanzen : Carbocromen-HCl, Cinnarizin, Dihydralazinsulfat, Dipyridamol, Etofyllin, Isosorbiddinitrat (Lactosever), Nicotinsäure, Propanolol, Nifedipin, Pentoxifyllin, Prenylaminlactat, Tolazolin-HCl, Acetylcholin, Phosphatidylcholin, Insulin Glargine, Gentiacaulenin und Gentiakochianin, Thieno[3,2-c]pyridin und Derivate Benzothiadiazine z.B. Hydrochlorothiazid, Euxanthon, Garcinone E, Gentisin, Euxanthinsäure, Isogentisin, Gentisein, Mangiferin und Homomangiferin, 2-Pyrrolidon, Citrate wie Acetyltributyl- und triethylcitrat, Tributyl- und Triethylcitrat, Benzoesäurebenzylester, Phtalate wie Dibutyl- und triethylphtalat, Fettsäureester wie Isopropylmyristat und -palmitat, Triacetin, Anthocyane wie Pelargonidin, Cyanidin, Delphidin, Paeonidin, Petunidin, Malvidin, Catechine, sowie deren Derivate und Metaboliten.

Die Kombination von membrangängigem Transportvermittler und Wirkstoff kann in verschiedenen Ausführungsformen realisiert werden:

1. Transportvermittler und Wirkstoff sind identisch
2. Transportvermittler und Wirkstoff sind nicht identisch, unterstützen sich in ihrer Wirkung
3. Der Transportvermittler hat keinen Einfluss auf die Wirkung des beigefügten Wirkstoffes und dient ausschliesslich als Transportvehikel

Insbesondere sind Citrate und Citratester bevorzugte Bestandteile für eine Beschichtung bzw. der Beschichtungslösung. Es hat sich gezeigt, dass Citrate und Citratester die Anhaftung der an das Gewebe abgegebenen Beschichtung begünstigen und den Eintritt des oder der Wirkstoffe in das Gewebe und in die Zellen fördern.
Citrate haben folgende allgemeine Struktur.

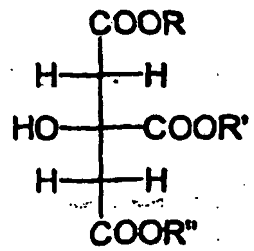

worin
R, R' und R" unabhängig voneinander Wasserstoff oder einen linearen oder verzweigten, gesättigten oder ungesättigten, mit mindestens einer funktionellen Gruppe substituierten oder unsubstituierten Alkylrest, Arylalkylrest oder Cycloalkylrest bedeuten.

Als funktionelle Gruppe sind folgende Reste möglich:

-H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -SH, $-SCH_3$, $-SC_2H_5$, $-NO_2$, -F, -Cl, -Br, -I, $-COCH_3$, $-COC_2H_5$, - $COC_3H_7$, -CO-cyclo-$C_3H_5$, $-COCH(CH_3)_2$, -COOH, $-COOCH_3$, $-COOC_2H_5$, -$COOC_3H_7$, -COO-cyclo-$C_3H_5$, $-COOCH(CH_3)_2$, $-OOC-CH_3$, -OOC-C2$H_5$, - OOC-$C_3H_7$, -OOC-cyclo-$C_3H_5$, -OOC-$CH(CH_3)_2$, $-CONH_2$, $-CONHCH_3$, - $CONHC_2H_5$, $-CONHC_3H_7$, -CONH-cyclo-$C_3H_5$, $-CONH[CH(CH_3)_2]$, $-CON(CH_3)_2$,

$-CON(C_2H_5)_2$, $-CON(C_3H_7)_2$, $-CON(cyclo\text{-}C_3H_5)_2$, $-CON[CH(CH_3)_2]_2$, $-NHCOCH_3$, $-NHCOC_2H_5$, $-NHCOC_3H_7$, $-NHCO\text{-}cyclo\text{-}C_3H_5$, $-NHCO\text{-}CH(CH_3)_2$, $-NHCO\text{-}OCH_3$, $-NHCO\text{-}OC_2H_5$, $-NHCO\text{-}OC_3H_7$, $-NHCO\text{-}O\text{-}cyclo\text{-}C_3H_5$, -NHCO-OCH(CH3)$_2$, $-NHCO\text{-}OC(CH_3)_3$, $-NH_2$, $-NHCH_3$, $-NHC_2H_5$, $-NHC_3H_7$, $-NH\text{-}cyclo\text{-}C_3H_5$, $-NHCH(CH_3)_2$, -NHC (CH3)$_3$, $-N(CH_3)_2$, $-N(C_2H_5)_2$, $-N(C_3H_7)_2$, - N(cyclo-$C_3H_5)_2$, $-N[CH(CH_3)_2]_2$, $-SO_2CH_3$, $-SO_2C_2H_5$, $-SO_3H$, $-SO_3CH_3$, - $SO_3C_2H_5$, $-OCF_3$, $OC_2F_5$, $-NH\text{-}CO\text{-}NH_2$, $-NH\text{-}C(=NH)\text{-}NH_2$, $-O\text{-}CO\text{-}NH_2$, . $-O\text{-}CO\text{-}NHCH_3$, $-O\text{-}CO\text{-}N(CH_3)_2$, -O-CO-$N(C_2H_5)_2$, $-CH_2F$, $-CHF_2$, $-CF_3$, $-CH_2Cl$, $-CH_2Br$, $-CH_2\text{-}CH_2F$, $-CH_2\text{-}CF_3$, $-CH_2\text{-}CH_2Cl$, $-CH_2\text{-}CH_2Br$, $-CH_3$, $-C_2H_5$, - $C_3H_7$, $-CH(CH_3)_2$, $-C(CH_3)_3$, $-C_4H_9$, $-CH_2\text{-}CH(CH_3)_2$, $-CH(CH_3)\text{-}C_2H_5$, $-C_5H_{11}$, - $C_8H_{13}$, $-C_7H_{15}$, $-C_8H_{17}$, -cyclo-$C_3H_5$, -cyclo-$C_4H_7$, -cyclo-$C_5H_9$, -cyclo-$C_6H_{11}$, - Ph, $-CH_2\text{-}Ph$, $-CH=CH_2$, $-CH_2\text{-}CH=CH_2$, $-C(CH_3)=CH_2$, -CH=CH-$CH_3$, $-C_2H_4\text{-}CH=CH_2$, $-CH=C(CH_3)_2$, $-C\equiv CH$, $-C\equiv C\text{-}CH_3$, $-CH_2\text{-}C\equiv CH$.

Bevorzugt sind die vorgenannten Alkylgruppen, substituierten Alkylgruppen sowie Diester und insbesondere Triester der Zitronensäure.

**Polymere Matrix**

Neben den nichlpolymeren Substanzen für eine Matrix zur Aufnahme eines oder mehrerer Wirkstoffe sind natürlich auch die bekannten polymeren Substanzen einsetzbar.

Als Matrix können biokompatible Substanzen verwendet werden, die als minimale Forderung im Vergleich zum unbeschichteten Implantat die Eigenschaften und Verwendung des Implantates nicht negativ beeinflussen. Die Matrix wird hierin auch als Träger, Trägersystem, polymerer Träger oder wirkstoffenthaltende Beschichtung bezeichnet.

Folgende biokompatiblen bioabbaubaren oder/und biostabilen Polymeren lassen sich bevorzugt für die Beschichtung des Kurzzeitimplantates verwenden :

Als biologisch stabile und nur langsam biologisch abbaubare Polymere können genannt werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Polyethersulfone, Epoxyharze, ABS-Harze, EPDM-Gummis, Siliconpräpolymere, Silicone wie Polysiloxane, Polyvinylhalogene und Copolymere, Celluloseether, Cellulosetriacetate, Chitosan, Chitosanderivate, polymerisierbare Öle wie z.B. Leinöl und Copolymere und/oder Mischungen derselben. Ferner lässt sich in einem dem Beschichtungsschritt vorgelagerten Schritt eine hämokompatible Schicht adhäsiv oder vorzugsweise kovalent auf die unbeschichtete Oberfläche des Medizinproduktes bringen oder mittels Quervernetzung z.B. mit Glutardialdehyd auf der Oberfläche des Medizinproduktes immobilisieren. Eine solcherart die Blutgerinnung nicht aktivierende Schicht ist sinnvoll, da dadurch die Hämokompatibilität der Medizinproduktoberfläche erhöht wird und die Thrombosegefahr gesenkt wird. Besonders sinnvoll ist dieser Beschichtungsschritt vor allem, wenn das Kurzzeitimplantat nur partiell beschichtet werden soll. Der nicht mit Wirkstoff beschichtete Bereich verfügt damit vorteilhafterweise über eine die Gerinnung nicht aktivierende, athrombogene Oberfläche und gewährleistet damit eine erheblich höhere Sicherheit während und nach Kontakt des Medizinproduktes mit dem Blut.

Die vorzugsweise hämokompatible Schicht wird aus folgenden bevorzugten Stoffen hergestellt: Heparin nativen Ursprungs als auch regioselektiv hergestellte Derivate unterschiedlicher Sulfatierungsgrade und Acetylierungsgrade im Molekulargewichtsbereich von des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycolcalix, Oligosaccharide, Polysaccharide, vollständig desulfatiertes und N-reacetyliertes Heparin; desulfatiertes und N-reacetyliertes Heparin, N-carboxymethyliertes und/oder partiell N-acetyliertes Chitosan, Polyacrylsäure, Polyetheretherketone, Polyvinylpyrrolidon, und/oder Polyethylenglykol sowie Mischungen dieser Substanzen.

Als biologisch abbaubare oder resorbierbare Polymere können beispielsweise verwendet werden: Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat); Poly(DTE-co-DT-carbonat), Poly(Bisphenol A-iminocarbonat), Polyorthoester, Polyglycol-säuretrimethylcarbonate, Polytrimethylcarbonate, Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, . Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierte Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen.

Zusätzlich kann die Ballonoberfläche mit oder ohne Stent mit einer athrombogenen bzw. inerten bzw. biokompatiblen Oberfläche oder allgemein mit einer Beschichtung und insbesondere einer polymeren oder nicht-polymeren Beschichtung versehen werden. Zur Erzeugung einer hemokompatiblen bzw. blutverträglich Oberfläche auf dem Katheterballon können die hierin genannten Oligosaccharide, Polysaccharide und insbesondere die beschriebenen Heparin-Derivate und Chitosan-Derivate vorzugsweise gemäß allgemeiner Formel Ia und Ib eingesetzt werden.

Insbesondere bevorzugte Polymere sind Polysulfone, Polyethersulfone, Silicone, Chitosan, Polyacrylate, Polyamide, Polyetheramide, Polyurethane, Polylactide, Polyglycolide, Copolymere der Polylactide und Polyglycolide, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polyester, PEG, Hyaluronsäure, Heparansulfat, Heparin, Chondroitinsulfat, Dextran und beta-Cyclodextrine.

**Ballon mit gekrimptem Stent**

Eine weitere bevorzugte Ausführungsform der vorliegenden Erfindung umfasst einen Katheterballon mit gekrimptem Stent.

Bei dieser Ausführungsform ergeben sich insbesondere 4 Varianten, welche je nach zu behandelnder Gefäßverengung ausgewählt und eingesetzt werden können.

Variante [A] stellt einen Katheterballon mit gekrimptem nicht resorbierbarem und unbeschichtetem Stent dar.

Bei Variante [B] ist der nicht-resorbierbare Stent mit einem Wirkstoff-freisetzenden Trägersystem beschichtet.

Variante [C] umfasst einen resorbierbaren unbeschichteten Stent und Variante [D] ist ein Katheterballon mit einem resorbierbaren wirkstofffreisetzenden Stent.

Variante [A]:

Da ein wirkstofffreisetzendes System, in der Regel eine wirkstofffreisetzende Beschichtung auf einem Stent nicht immer erwünscht ist und in einigen Fällen das Problem von Spätthrombosen auftreten kann, bietet die Variante [A] ein ideales System einen stark verengten Körperdurchgang wie beispielsweise Gallenwegen, Speiseröhre, Harnwegen, Bauchspeicheldrüse, Nierenwege, Lungenwege, Luftröhre, Dünndarm sowie Dickdarm und insbesondere Blutgefäße mit einem dauerhaften Stent offen zu halten, welcher keine Beschichtung aufweist, wobei dennoch auf eine Wirkstoffapplikation nicht verzichtet werden muss.

Der Katheterballon gemäß Variante [A] ist mit einer reinen Wirkstoffschicht oder einem Träger enthaltend einen Wirkstoff beschichtet und bei der Dilatation wird zum einen der Stent gesetzt und zum anderen mindestens über die gesamte Länge des Stents und vorteilhafter Weise noch darüber hinaus ein Wirkstoff appliziert, der für ein kontrolliertes Einwachsen und eine Verhinderung der Überwucherung des Stents mit vor allem glatten Muskelzellen sorgt. Als Wirkstoff oder Wirkstoffgemisch können die oben genannten Wirkstoffe und insbesondere Paclitaxel und/oder Rapamycin eingesetzt werden.

Vorzugsweise wird der Katheterballon mit Wirkstoff ohne oder mit Trägersystem so beschichtet, dass die Ballonbeschichtung über beide Stentenden hinausreicht und vorzugsweise 10-20% der Gesamtlänge des Stents über ein Ende des Stents hinausreicht. Dadurch wird bei der Dilatation der Wirkstoff auch auf den Gefäßbereich an beiden Enden des Stents übertragen, wo der Stent nicht mehr hinreicht und es wird Wirkstoff ganzflächig auf die Gefäßwand übertragen, welche zwischen den sich aufweitenden bzw. aufgeweiteten Stentstreben liegt.

Diese Ausführungsform hat den Vorteil, dass die Stentoberfläche keinen Wirkstoff aufweist, der die Zellen insbesondere die glatten Muskelzellen inhibiert oder abtötet, welche direkt mit der Stentoberfläche in Verbindung treten. Hingegen wird in die Zwischenräume zwischen den Stentstreben genügend Wirkstoff appliziert, so dass gerade das zu schnelle Überwachsen des Stents, welches in den Zwischenräumen der Stentstreben beginnt und sich in das Innere des Stents fortsetzt und zur In-Stent-Restenose führt, eingedämmt bzw. auf ein kontrolliertes Maß reduziert wird.

Während ein wirkstoffbeschichteter Stent den Wirkstoff nur von seiner Oberfläche und nicht aus den Zwischenräumen der Stentstreben oder an den Enden des Stent bzw. dem darüber hinausgehenden Bereich und zudem gerade auf das anliegende Gewebe abgibt, welches gar nicht inhibiert oder abgetötet werden soll, wird gemäß Variante [A] der Wirkstoff genau dort appliziert, wo er benötigt wird. Bevorzugt ist ferner, wenn der Katheterballon am distalen und proximalen Ende noch einige mm über das Ende des Stents hinaus beschichtet ist, so dass die Wirkstoffabdeckung der Gefäßwand einige mm über das Ende des Stents hinausgeht, um auch die Endbereiche des im Gefäß liegenden Stents ausreichend mit Wirkstoff zu versorgen.

Somit wird vorzugsweise der Katheterballon mit dem Wirkstoff mit oder ohne Träger beschichtet und danach ein unbeschichteter Stent auf den Ballon gekrimpt.

Zur Variante [B] kann man hingegen gelangen, wenn man einen nicht resorbierbaren Stent wie bei Variante [A] auf einen Ballon krimpt und danach Stent und Ballon mit einem Wirkstoff beschichtet.

Der Begriff "nicht resorbierbar" bedeutet, dass der Stent ein Dauerimplantat darstellt, welches unter physiologischen Bedingungen nicht oder nur sehr langsam aufgelöst wird. Derartige Stents sind beispielsweise aus medizinischem Edelstahl, Titan, Chrom, Vanadium, Wolfram, Molybdän, Gold, Nitinol, Magnesium, Zink, Eisen, Legierungen der vorgenannten Metalle sowie Keramiken oder auch biostabilen Polymeren.

Beschichtet an einen Katheterballon mit gekrimptem Stent gleichzeitig, so wird vorzugsweise eine Lösung des reinen Wirkstoffs in einem Lösungsmittel verwendet, welches den Katheterballon möglichst wenig angreift dennoch vorzugsweise benetzend ist und zudem dünnflüssig genug, um zwischen die Streben des gekrimpten Stent im komprimierten Zustand zu fließen.

Diese Ausführungsform eignet sich für eine spontane Freisetzung (Spontanrelease) von relativ viel Wirkstoff, da die Zwischenräume der Stentstreben und die Zwischenräume zwischen der inneren Oberfläche des Stents und der Oberfläche des Katheterballons als Wirkstoffreservoir dienen.

Der Unterschied zur Variante [A] besteht vor allem in der applizierbaren Menge an Wirkstoff, da sich gemäß der vorher beschriebenen Methode eine deutlich größere: Menge eines Wirkstoffs oder Wirkstoffgemisches auf den Stent und Katheterballon aufbringen lässt.

Als Beschichtungslösung eignen sich für hydrophobe Wirkstoffe wie z.B. Paclitaxel Lösungen aus z.B. Dimethylsulfoxid (DMSO) Chloroform, Ethanol, Aceton, Methylacetat und Hexan und ihren Gemischenoder z.B. von Rapamycin in Essigsäureethylester, Methanol / Ethanol-Gemischen, Ethanol/Wasser-Gemischen oder in Ethanol. Selbstverständlich können auch andere Wirkstoffe verwendet werden.

Es ist auch möglich, einen Träger in die Wirkstofflösung zu geben, wobei jedoch polymere Träger eher selten eingesetzt werden, wenn der Katheterballon zusammen mit dem gekrimpten Stent beschichtet werden. Falls ein Trägersystem verwendet werden soll, so eignen sich eher nicht-polymere Träger wie beispielsweise Kontrastmittel oder Kontrastmittelanaloga als auch biologisch verträgliche organische Stoffe, die die Beschichtungseigenschaften verbessern und die Aufnahme von Wirkstoff in das Gefäß erhöhen wie beispielsweise Aminosäuren, Zucker, Vitamine, Saccharide, 2-Pyrrolidon, Acetyltributyl- und triethylcitrat, Tributyl- und Triethylcitrat, Benzoesäurebenzylester, Triethyl- und Dimethylphtalat, Fettsäureester wie Isopropylmyristat und -palmitat, Triacetin und dergleichen. Als ebenfalls gut geeignet eignen sich Mischungen aus diesen verschiedenenen Substanzen. So zeigt sich z.B. die Mischung aus dem Polysaccharid Carrageenan, Lecithin und Glycerin als äusserst geeignetes Wirkstoffträgersystem. Auch physiologisch verträgliche Salze können als Matrix zur Einlagerung des Wirkstoffs verwendet werden.

Auch bei dieser Variante wird vorzugsweise der Ballon über die vom Stent bedeckte Oberfläche hinausgehend beschichtet. Vorzugsweise beträgt der über das Ende des Stents hinausgehende beschichtete Bereich des Ballons nicht mehr als 20% der Gesamtlänge des Stents, vorzugsweise nicht mehr als 15% und insbesondere bevorzugt nicht mehr als 10% der Gesamtlänge des Stents.

In der Regel ist bei Variante [A] als auch Variante [B] eine vollflächige Beschichtung vorteilhaft, d.h. der Katheterballon gemäß Variante [A] oder Stent und Katheterballon gemäß Variante [B] werden vollflächig mit einer Beschichtung versehen.

Die Varianten [A] und [B] können ferner noch dahingehend ausgestaltet werden, dass die Beschichtung mit Wirkstoff nicht gleichmäßig erfolgt, sondern man einen Gradienten verwendet, d.h. ein Konzentrationsgefälle an Wirkstoff auf der Ballon- bzw Ballon- und Stentoberfläche erzeugt. So kann beispielsweise in der Mitte des Katheterballons eine größere Konzentration an Wirkstoff aufgebracht werden oder an einem oder beiden Enden des Katheterballons oder in der Mitte und an einem oder beiden Enden.

Zudem kann auch nur an einer Stelle oder einem Teil des Katheterballons eine höhere Wirkstoffkonzentration aufgebracht werden als auf der restlichen Oberfläche. So muss beispielsweise den Stentenden besondere Aufmerksamkeit vor allem in der Frühphase nach der Implantation zukommen, da diese Übergangsbereiche vermehrt risikobehaftet sind. Beliebige Variationen sind hier denkbar.

Bei Variante [C] und Variante [D] handelt es sich um zukünftig wohl immer wichtiger werdende Ausführungsformen, da beide Ausführungsformen keine Dauerimplantate darstellen.

Beide Varianten verwenden biologisch abbaubare, d.h. bioresorbierbare Stents. Solche unter physiologischen Bedingungen degradierbare Stents werden im Körper des Patienten innerhalb von einigen Wochen bis zu einem oder

zwei Jahren vollständig abgebaut.

Biologisch abbaubare Stents bestehen aus Metallen wie beispielsweise Magnesium, Calcium oder Zink oder auch aus organischen Verbindungen wie beispielsweise Polyhydroxybutyrat, Chitosan oder Kollagen.

Ein bioresorbierbarer Metallstent aus überwiegend Magnesium ist in dem europäischen Patent EP 1 419 793 B1 offenbart. Die deutsche Offenlegungsschrift beschreibt Stents aus Magnesiumlegierungen und Zinklegierungen. Bioresorbierbare Stents aus Magnesium, Calcium, Titan, Zirkon, Niob, Tantal, Zink oder Silizium oder aus Legierungen oder Mischungen der vorgenannten Stoffe wird in der deutschen Offenlegungsschrift DE 198 56 983 A1 offenbart. Explizite Beispiele werden zu Stents aus einer Zink-Calcium-Legierung offenbart.

Weitere bioresorbierbare Metallstents aus Magnesium, Titan, Zirkon, Niob, Tantal, Zink und/oder Silizium als Komponente A und Lithium, Natrium, Kalium, Calcium, Mangan und/oder Eisen als Komponente B werden in der europäischen Patentanmeldung EP 0 966 979 A2 beschrieben. Explizite Beispiele werden zu Stents aus einer Zink-Titan-Legierung mit einem Titangewichtsanteil von 0,1 bis 1 % und einer Zink-Calcium-Legierung mit einem Gewichtsverhältnis von Zink zu Calcium von 21:1 offenbart.

Ein biologisch degradierbarer Stent aus der organischen Verbindung Polyhydroxybutyrat (PHB) sowie anderen Polyhydroxyalkanoaten wird in den US-Patenten US 6,548,569 B1, US 5,935,506, US 6,623,749 B2, US 6,838,493 B2 sowie US 6,867,247 B2 offenbart.

Das US-Patent US 6,245,103 B1 nennt ferner Polydioxanone, Polycaprolactone, Polygluconate, Polymilchsäure-Polyethylenoxid-Copolymere, modifizierte Cellulose, Collagen, Poly(hydroxybutyrate), Polyanhydride, Polyphosphoester sowie Polyaminosäuren als weitere geeignete bioabbaubare Stentmaterialien.

Das US-Patent US 6,991,647 B2 führt des weiteren Polyglycolsäure, Polylactide, Polyphosphatester und Poly-ε-Caprolactone als mögliche bioabbaubare organische Polymere an.

Grundsätzlich können biodegradierbare Stents aus folgenden Substanzen oder Gemischen der folgenden Substanzen hergestellt werden:

> Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierte Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere der vorgenannten Substanzen.

Bei Variante [C] wird nun ein solcher bioresorbierbarer Stent aus Metall oder organischen Polymeren auf einen beschichteten Katheterballon gekrimpt.

Ähnlich wie bei Variante [A] erfolgt die Beschichtung des Katheterballons. Varianten [C] und [D] haben den Vorteil, dass der Stent sich nach einer Zeitdauer von wenige Wochen bis zu ca. 18 Monaten vollständig auflöst und somit kein dauerhafter Fremdkörper im Patienten zurückbleibt, der chronische Entzündungen verursachen könnte. Über den beschichteten Katheterballon wird genügend Wirkstoff bei der Dilatation appliziert, dass der Stent zuerst einmal kontrolliert einwachsen kann und nach dem Einwachsen erst beginnt, sich derart zu zersetzen, dass keine Fragmente durch das Gefäß bzw. durch die Blutbahn weggespült werden können.

Bei Variante [D] kann der Wirkstoff oder die Wirkstoffkombination auf die Stentoberfläche als reine Wirkstoffschicht aufgetragen werden oder in einer nicht-polymeren Matrix wie beispielsweise einem Kontrastmittel, Kontrastmittelgemisch oder Kontrastmittelanalogon auf der Stentoberfläche eingebettet werden oder sich in einem polymeren Träger wie beispielsweise einem der oben genannten biologisch abbaubaren Polymeren auf der Stentoberfläche befinden und/oder auch in das biologisch abbaubare Stentmaterial selbst eingelagert werden.

Dadurch ergeben sich gerade bei der Variante [D] eine Fülle von Möglichkeiten, einen oder mehrere Wirkstoffe auf oder in den biologisch degradierbaren Stent aufzubringen oder einzulagern. Natürlich besteht auch die Möglichkeit, in das biologisch abbaubare Stentmaterial, also in den Stent selbst einen oder mehrere Wirkstoffe einzubringen und

diesen Stent zusätzlich mit einem Wirkstoff zu beschichten oder mit einem polymeren oder nicht-polymeren Träger enthaltend einen oder mehrere Wirkstoffe zu beschichten. Ferner kann der Stent oder die wirkstoffenthaltende Schicht wiederum mit einer biologisch abbaubaren Barriereschicht oder hemokompatiblen Schicht versehen werden, so dass Zweischichtsysteme oder auch Mehrschichtsysteme mögliche Ausführungsformen darstellen.

Zudem sind auch Wirkstoffkombinationen dahingehend denkbar, dass eine Wirkstoffkombination in den Stent oder auf den Stent aufgetragen wird oder eine Wirkstoffkombination dadurch entsteht, dass sich in dem Stent ein anderer Wirkstoff befindet als auf dem Stent.

Die Varianten [B] und [D] bieten ferner die Möglichkeit der Applikation einer Wirkstoffkombination dadurch, dass sich auf dem Katheterballon ein anderer Wirkstoff befindet als auf oder in dem Stent.

Auf dem Katheterballon wird vorzugsweise ein Wirkstoff aufgetragen, welcher seine Wirkung innerhalb weniger Stunden oder Tage nach der Dilatation entfaltet, wobei auf dem Stent oder in dem biologisch abbaubaren Stent ein zweiter Wirkstoff in einer anderen Konzentration aufgebracht oder eingebracht werden kann, der insbesondere eine Langzeitwirkung entfaltet und während der Zeit des biologischen Abbaus des Stents freigesetzt wird.

Insbesondere ist bevorzugt, wenn sich auf dem Katheterballon eine cytotoxische Dosis eines Wirkstoffs befindet und auf dem Stent und/oder in dem biologisch abbaubaren Stent eine cytostatische Dosis desselben oder eines anderen Wirkstoffs enthalten ist.

Eine besonders bevorzugte Ausführungsform enthält Paclitaxel auf dem Katheterballon in einer cytotoxischen Dosis und in einer polymeren Beschichtung auf einem Metallstent oder in einer biologisch abbaubaren Beschichtung auf dem bioresorbierbaren Stent in einer cytostatischen Konzentration.

Eine weitere insbesondere bevorzugte Ausführungsform ist eine Kombination von Paclitaxel in einer cytotoxischen oder einer cytostatischen Dosis auf dem Katheterballon und eine vorzugsweise cytostatische Dosis von Rapamycin auf oder in dem biologisch abbaubaren Stent.

Diese letztgenannten Kombinationen ermöglichen eine Kombinationstherapie mit einem schnell freigesetzten Wirkstoff in vorzugsweise hoher und/oder cytotoxischer Konzentration und einem langsam freigesetzten Wirkstoff in vorzugsweise niedriger und/oder cytostatischer Konzentration.

Bei den verwendeten biologisch stabilen (nicht-resorbierbaren) als auch den biologisch abbaubaren Stents ist es bevorzugt, den Stent mit einer blutverträglichen Basisbeschichtung zu versehen. Dies ist insbesondere bei nicht-resorbierbaren Stents vorteilhaft, da diese als Langzeitimplantate dauerhaft hemokompatibel sein sollten. Diese hemokompatible Beschichtung befindet sich vorzugsweise als unterste Schicht oder auch als einzige Schicht auf der Stentoberfläche und kann adhäsiv aus auch vorzugsweise kovalent gebunden sein und maskiert die Stentoberfläche, d.h. die gesamte Stentoberfläche (Innenoberflächen, Außenoberfläche und Strebenzwischenräume) bestenfalls vollständig.

Diese hemokompatible Basisbeschichtung gewährleistet, dass mit Abklingen des Wirkstoffeinflusses und Abbau der Matrix keine Reaktionen auf die bestehende Fremdoberfläche mehr einsetzen, die langzeitlich ebenfalls zu einem Wiederverschluß des Blutgefäßes führen können. Die den Stent unmittelbar bedeckende hämokompatible Schicht besteht bevorzugt aus Heparin nativen Ursprungs als auch synthetisch hergestellter Derivate unterschiedlicher Sulfatierungsgrade und Acteylierungsgrade im Molekulargewichtsbereich von den für die antithrombotische Wirkung verantwortlichen Pentasaccharides bis zum Standardmolekulargewicht des käuflichen Heparins, Heparansulfaten und dessen Derivate, Oligo- und Polysaccharide der Erythrocytenglycolcalix, die in perfekter Weise die athrombogene Oberfläche der Erythrocyten wiedergeben, da hier im Gegensatz zum Phosphorylcholin der tatsächliche Kontakt von Blut und Erythrocytenoberfläche stattfindet, Oligosacchariden, Polysacchariden, vollständig desulfatiertem und N-reacetyliertem Heparin, desulfatiertem und N-reacetyliertem Heparin, N-carboxymethyliertem und/oder partiell N-acetyliertem Chitosan, Polyacrylsäure, Polyvinylpyrrolidon, und Polyethylenglykol und/oder Mischungen dieser Substanzen. Diese Stents mit einer hämokompatiblen Beschichtung werden hergestellt, indem man herkömmliche in der Regel unbeschichtete Stents bereitstellt und bevorzugt kovalent eine hämokompatible Schicht aufbringt, welche nach Wirkstofffreigabe und damit nach Abklingen des Wirkstoffeinflusses und Abbau der Matrix die Oberfläche des Implantates permanent maskiert. Daher wird diese hämokompatible Beschichtung auch direkt auf die Stentoberfläche aufgetragen.

Somit betrifft eine bevorzugte Ausführungsform der vorliegenden Erfindung einen Stent beliebigen Materials, dessen Oberfläche durch die Auftragung der Glycocalixbestandteile von Blutzellen, Esotheizellen oder Mesothelzellen maskiert wird. Die Glycocalix ist die äußere Schicht von beispielsweise Blutzellen, Esothelzenen oder Mesothelzellen aufgrund derer diese Zellen blutverträglich (hämokompatibel) sind. Die Bestandteile dieser äußeren Schicht (Glycocalix) von Blutzellen, Esothelzellen und/oder Mesothelzellen wird von der Zelloberfläche vorzugsweise enzymatisch abgetrennt, von den Zellen separiert und als Beschichtungsmaterial für die Stents verwendet. Zu diesen Glycocalixbestandteilen zählen unter anderem Oligosaccharid-, Polysaccharid - und Lipid-Anteile der Glykoproteine, Glykolipide und Proteoglykane sowie Glykophorine, Glykosphingolipide, Hyaluronsäuren, Chondroitinsulfate, Dermatansulfate, Heparansulfate als auch Keratansulfate. Verfahren für die Isolierung und Verwendung dieser Stoffe als Beschichtungsmaterial sind ausgiebig in dem europäischen Patent EP 1 152 778 B1 der Firmengründer der Hemoteq AG, Herrn Dr. Michael Hoffmann und Herrn Dipl.-Chem. Roland Horres, beschrieben.

Die kovalente Anbindung erfolgt wie beim Hemoparin (siehe bei den Beispielen Beispiel Nr 9, 14)

Weitere bevorzugte Ausführungsformen weisen eine unterste direkt auf der Ballonoberfläche aufgebrachte hämokompatible Beschichtung aus desulfatiertem und N-reacetyliertem Heparin und/oder N-carboxymethyliertem und/oder partiell N-acetyliertem Chitosan. Diese Verbindungen sowie die Glycocalixbestandteile haben sich in etlichen Studien bereits als sehr gute hämokompatible Beschichtung erwiesen und machen die Oberfläche blutverträglich nachdem die darüberliegenden Wirkstoff - und/oder Trägerschichten abgespalten oder biologisch abgebaut worden sind. Derartige besonders bevorzugte Materialien für die Beschichtung der Stentoberfläche sind in dem europäischen Patent Nr. EP 1 501 565 B1 der Firma Hernoteq AG offenbart. Auf diese untere hämokompatible Schicht werden eine oder mehrere Wirkstoffschichten und/oder wirkstofffreie oder wirkstoffenthaltende Träger- oder Polymerschichten aufgebracht.

Peptide, Nukleotide, Saccharide

Als Matrixmaterialien eignen sich ferner sehr gut Peptide, Proteine, Nukleotide sowie Saccharide, welche zum einen Wirkstoffe einlagern können und zum anderen eine gewisse Affinität zur Zellwand aufweisen und nach der Übertragung auf die Zellwand biologisch abgebaut werden können.

Als Beispiele solcher Verbindungen können, Chitosan, Chitin, Glykosaminoglykane wie Heparin, Dermatansulfate, Heparansulfate, Chondroitinsulfat und Hyaluronsäure, Collagen, Carrageenan, Agar-Agar, Johannisbrotkernmehl, Fibrin, Cellulose, Rayon, Peptide mit 50 bis 500 Aminosäuren, Nukleotide mit 20 bis 300 Basen sowie Saccharide mit 20 bis 400 Zuckerbausteinen. Derartige Trägerstoffe haben eine gewisse Affinität zu biologischem Gewebe und können bei der kurzzeitigen Dilatation für eine ausreichende Wirkstoffübertragung auf die Gefäßwand sorgen.

Bevorzugt sind Polysaccharide mit Molekulargewichten von 2kD bis 400kD auf, bevorzugt von 5kD bis 150kD, mehr bevorzugt von 10kD bis 100kD und insbesondere bevorzugt von 30kD bis 80kD. Die bevorzugten Oligo- und/oder Polysaccharide zeichnen sich dadurch aus, dass sie den Zuckerbaustein N-Acylglucosamin oder N-Acylgalactosamin in großer Menge enthalten. Dies bedeutet, dass 40 bis 60%,

bevorzugt 45 - 55% und insbesondere bevorzugt 48 - 52% der Zuckerbausteine N-Acylglucosamin oder N-Acylgalactosamin sind und im wesentlichen die restlichen Zuckerbausteine jeweils einen Carboxylrest aufweisen. Die Oligo- und/oder Polysaccharide bestehen somit gewöhnlich zu über 95%, bevorzugt zu über 98%, aus nur zwei Zuckerbausteinen, wobei ein Zuckerbaustein einen Carboxylrest und der andere einen N-Acylrest trägt.

Ein Zuckerbaustein der bevorzugten Oligo- und/oder Polysaccharide ist N-Acylglucosamin bzw. N-Acylgalactosamin., bevorzugt N-Acetylglucosamin bzw. N-Acetylgalactosamin, und bei dem anderen handelt es sich um Uronsäuren, bevorzugt um Glucuronsäure und Iduronsäure.

Bevorzugt sind Oligo- und/oder Polysaccharide, welche im wesentlichen aus dem Zucker Glucosamin bzw. Galactosamin bestehen, wobei im wesentlichen die Hälfte der Zuckerbausteine eine N-Acylgruppe trägt, bevorzugt eine N-Acetylgruppe, und die andere Hälfte der Glucosaminbausteine eine über die Aminogruppe direkt oder über eine oder mehrere Methylenylgruppen gebundene Carboxylgruppe trägt. Bei diesen an die Aminogruppe gebundenen Carbonsäurereste handelt es sich bevorzugt um Carboxymethyl- oder Carboxyethylgruppen. Ferner sind Oligo- und/oder Polysaccharide bevorzugt, welche im wesentlichen zur Hälfte, d.h. zu 48 - 52%, bevorzugt zu 49 - 51% und insbesondere bevorzugt zu 49,5 - 50,5%, aus N-Acylglucosamin bzw. N-Acylgalactosamin, bevorzugt aus N-Acetylglucosamin oder N-Acetylgalactosamin und im wesentlichen zur anderen Hälfte aus einer Uronsäure, bevorzugt Glucuronsäure und Iduronsäure, bestehen. Besonders bevorzugt sind die Oligo- und/oder Polysaccharide, welche eine im wesentlichen alternierende Abfolge (d.h. abgesehen von der statistischen Fehlerrate bei der alternierenden Verknüpfung) der beiden Zuckerbausteine aufweisen. Die Rate der Fehlverknüpfungen sollte unter 1%, bevorzugt unter 0.1% liegen.

Überraschenderweise hat sich gezeigt, daß sich für die erfindungsgemäßen Verwendungen insbesondere im wesentlichen desulfatiertes und im wesentlichen N-acyliertes Heparin sowie partiell N-carboxyalkyliertes und N-acyliertes Chitosan als auch desulfatiertes und im wesentlichen N-acyliertes Dermatansulfat, Chondroitinsulfat und auch in der Kettenlänge reduzierte Hyaluronsäure besonders gut eignen. Insbesondere sind für die hemokompatible Beschichtung N-acetyliertes Heparin sowie partiell N-carboxymethyliertes und N-acetyliertes Chitosan geeignet.

Die durch "im wesentlichen" definierten Desulfatierungs- und Acylierungsgrade wurden bereits weiter oben definiert. Der Begriff "im wesentlichen" soll verdeutlichen, daß statistische Abweichungen zu berücksichtigen sind. Eine im wesentlichen alternierende Abfolge der Zuckerbausteine besagt, daß in der Regel keine zwei gleichen Zuckerbausteine aneinander gebunden sind, schließt aber eine derartige Fehlverknüpfung nicht vollkommen aus. Entsprechend bedeutet "im wesentlichen zur Hälfte" annähernd 50%, läßt aber geringe Schwankungen zu, da gerade bei biosynthetisch hergestellten Makromolekülen nie der Idealfall erreicht wird und immer gewisse Abweichungen berücksichtigt werden müssen, da Enzyme nicht perfekt arbeiten und bei der Katalyse mit einer gewissen Fehlerrate zu rechnen ist. Im Falle des natürlichen Heparins liegt hingegen eine streng alternierende Abfolge von N-Acetylglucosamin- und Uronsäureeinheiten vor.

Als besonders gut an der Zellwand haftend hat sich beispielsweise auch eine Mischung aus Carrageenan mit Phospatidylcholin und Glycerin erwiesen. Deratige Mischungen von Polysacchariden mit membrangängigen Substanzen können als Matrix für den Wirkstoff oder die Wirkstoffmischung an der Aussenseite der Zellmembran haftend für die kontrollierte

Wirkstoffübertragung in das Zellinnere über einen wesentlich längeren Zeitraum sorgen, als der kurzzeitige Kontakt des Medizinproduktes mit der Gefässwand erlaubt.

Des weiteren wird ein Verfahren zur hemokompatiblen Beschichtung von Oberflächen offenbart, welche für den direkten Blutkontakt bestimmt sind. Bei diesem Verfahren wird eine natürliche und/oder künstliche Oberfläche bereitgestellt und die oben beschriebenen Oligo- und/oder Polysaccharide werden auf dieser Oberfläche immobilisiert.

Die Immobilisierung der Oligo- und/oder Polysaccharide auf diesen Oberflächen kann mittels hydrophober Wechselwirkungen, van der Waals Kräften, elektrostatischer Wechselwirkungen, Wasserstoffbrücken, ionischer Wechselwirkungen, Quervernetzung der Oligo- und/oder Polysaccharide und/oder durch kovalente Bindung an die Oberfläche bewirkt werden. Bevorzugt ist die kovalente Verknüpfung der Oligo - und/oder Polysaccharide mehr bevorzugt die kovalente Einzelpunktverknüpfung (sideon Bindung) und insbesondere bevorzugt die kovalente Endpunktverknüpfung (end-on Bindung).

Unter "im wesentlichen die restlichen Zuckerbausteine" ist zu verstehen, dass 93% der restlichen Zuckerbausteine, bevorzugt 96% und insbesondere bevorzugt 98% der restlichen 60% - 40% der Zuckerbausteine einen Carboxylrest tragen.

Somit sind vor allem die Kurzimplantate bevorzugt mit dieser hämokompatiblen Schicht aus den vorgenannten Heparin-Derivaten, Chitosan-Derivaten und/oder Oligo- bzw. Polypeptiden auszustatten, von denen im Verlaufe der Kontaktzeit eine Erhöhung der Biokompatibiltät erforderlich werden kann, wie sich beispielsweise bei einem nicht vollständig sondern partiell mit Wirkstoff beschichteten Kurzzeitimplantat für den unbeschichteten Bereich bereits über die gesamte Kontaktzeit als Vorteil erweit, wenn die nicht wirkstoffbeschichtete Oberfläche eine verbesserte Biokompatibilität aufweist. Ebenso sinnvoll ist die hämokompatible Schicht anzuwenden, wenn im Verlaufe der Kurzzeitimplantataufenthaltes im Organismus die unbeschichtete Materialoberfläche partiell oder ganz freigelegt wird.

Zur Verbesserung der Haftung derartiger Trägerstoffe lassen sich die Peptide, Proteine, Pronucleotide, Nukleotide sowie Saccharide miteinander vernetzen, was beispielsweise mittels Glutaraldehyd durchgeführt werden kann.

**Öle und Fette als Trägersubstanzen**

Neben den oben erwähnten biostabilen und bioabbaubaren Polymeren als Matrix für Transportvermittler und Wirkstoffe können auch physiologisch verträgliche Öle, Fette, Lipide, Lipoide und Wachse eingesetzt werden. WO 03/022265 A1 beschreibt ölige Formulierungen von Paclitaxel, welche ebenfalls eingesetzt werden können. Besonders bevorzugt sind jedoch aushärtbare bzw. autopolymerisierbare Öle und Fette.

Solche Öle, Fette und Wachse, die als Trägersubstanzen oder als wirkstofffreie Schichten, insbesondere Deckschichten eingesetzt werden können, eignen sich Stoffe, die sich durch folgende allgemeine Formeln darstellen lassen:

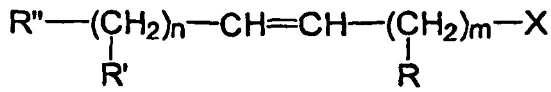

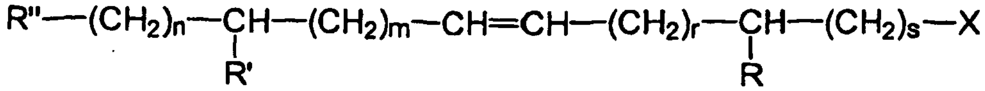

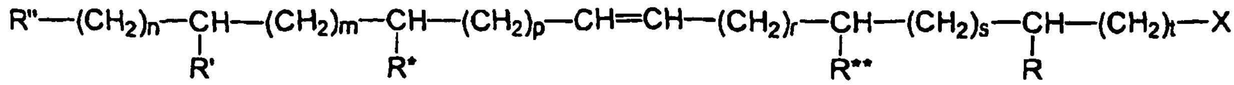

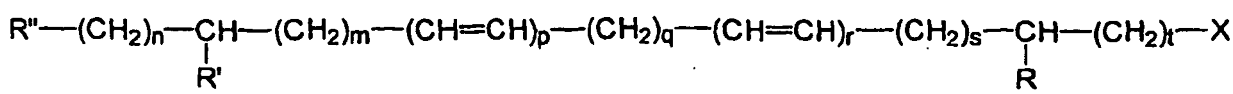

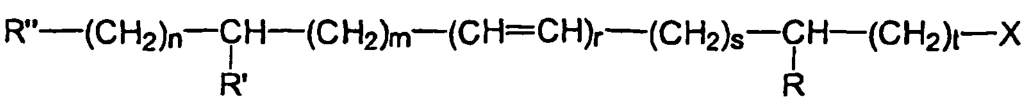

worin

R, R', R", R* und R** unabhängig voneinander für Alkyl-, Alenyl-, Alkinyl-, Heteroalkyl-, Cycloalkyl-, Heterocyclylreste mit 1 bis 20 Kohlenstoffatomen, Aryl-, Arylalkyl-, Alyklaryl- , Heteroarylreste mit 3 bis 20 Kohlenstoffatomen oder für funktionelle Gruppen stehen und bevorzugt folgende Reste bedeuten: -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, - OPh, $-OCH_2$-Ph, $-OCPh_3$, -SH, $-SCH_3$, $-SC_2H_5$, $-NO_2$, -F, -Cl, -Br, -I, -CN, -OCN,

-NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, $-COOCH_3$, $-COOC_2H_5$, - $COOC_3H_7$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-cyclo-$C_3H_5$, -OOC-$CH(CH_3)_2$, -OOC-$C(CH_3)_3$, - $CONH_2$, $-CONHCH_3$, $-CONHC_2H_5$, $-CONHC_3H_7$, -CON $(CH_3)_2$, $-CON(C_2H_5)_2$, - $CON(C_3H_7)_2$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, - $N[CH(CH_3)_2]_2$, $-N[C(CH_3)_3]_2$, $-SOCH_3$, $-SOC_2H_5$, $-SOC_3H_7$, $-SO_2CH_3$, $-SO_2C_2H_5$, $-SO_2C_3H_7$, $-SO_3H$, $-SO_3CH_3$, $-SO_3C_2H_5$, $-SO_3C_3H_7$, $-OCF_3$, $-OC_2F_5$, -O-$COOCH_3$, -O-$COOC_2H_5$, -O-$COOC_3H_7$, -O-COO-cyclo-$C_3H_5$, -O-$COOCH(CH_3)_2$, -O-$COOC(CH_3)_3$, -NH-CO-$NH_2$, -NH-CO-$NHCH_3$, -NH-CO-$NHC_2H5$, -NH-CO-$N(CH_3)_2$, -NH-CO-$N(C_2H_5)_2$, -O-CO-$NH_2$, -O-CO-$NHCH_3$, -O-CO-$NHC_2H_5$, -O-CO-$NHC_3H_7$, -O-CO-$N(CH_3)_2$, -O-CO-$N(C_2H_5)_2$, -O-CO-$OCH_3$, -O-CO-$OC_2H_5$, -O-CO-$OC_3H_7$, -O-CO-O-cyclo-$C_3H_5$, -O-CO-$OCH(CH_3)_2$, -O-CO-$OC(CH_3)_3$, $-CH_2F$, $-CHF_2$, $-CF_3$, $-CH_2Cl$, $-CH_2Br$, $-CH_2I$, - $-CH_2$-$CH_2F$, $-CH_2$-$CHF_2$, $-CH_2$-$CF_3$, $-CH_2CH_2Cl$, $-CH_2$-$CH_2Br$, $-CH_2$-$CH_2I$, $-CH_3$, $-C_2H_5$, $-C_3H_7$, -cyclo-$C_3H_5$, $-CH(CH_3)_2$, $-C(CH_3)_3$, $-C_4H_9$, $-CH_2$-$CH(CH_3)_2$, -CH $(CH_3)$-$C_2H_5$, -Ph, $-CH_2$-Ph, $-CPh_3$, $-CH=CH_2$, $-CH_2$-$CH=CH_2$, $-C(CH_3)=CH_2$, -CH=CH-$CH_3$, $-C_2H_4$-$CH=CH_2$, $-CH=C(CH_3)_2$, -C≡CH, -C≡C-$CH_3$, $-CH_2$-C≡CH;

X für eine Estergruppe oder Amidgruppe und insbesondere für -O-alkyl, -O-CO-alykl, -O-CO-O-alkyl, -O-CO-NH-alkyl, -O-CO-N-dialkyl, -CO-NH-alkyl, -CO-N-dialkyl, -CO-O-alkyl, -CO-OH, -OH;

m, n, p, q, r, s und t unabhängig voneinander ganze Zahlen von 0 bis 20, bevorzugt von 0 bis 10 bedeuten.

Die Bezeichnung "alkyl" beispielsweise bei -CO-O-alkyl bedeutet vorzugsweise eine der für die vorgenannten Reste R, R' usw. genannten Alkylreste, z.B. $-CH_2$-Ph. Die Verbindungen der vorgenannten allgemeinen Formeln können auch in Form ihrer Salze, als Racemate oder Diastereomerengemische, als reine Enantiomeren oder Diastereomeren sowie als Gemische oder Oligomere oder Copolymere oder Blockcopolymere vorliegen. Ferner können die vorgenannten Verbindungen im Gemisch mit anderen Substanzen wie den biostabilen und biodegradierbaren Polymeren und insbesondere im Gemisch mit den hierin genannten Ölen und/oder Fettsäuren eingesetzt werden. Bevorzugt sind derartige Gemische und Einzelsubstanzen, welche zur Polymerisation, insbesondere zur Autopolymerisation, geeignet sind.

Die zur Polymerisation insbesondere Autopolymerisation geeigneten Substanzen umfassen unter anderem Öle, Fette, Lipide, Fettsäuren sowie Fettsäureester, welche im folgenden näher beschrieben werden.

Bei den Lipiden handelt es sich vorzugsweise um einfach oder mehrfach ungesättigte Fettsäuren und/oder Mischungen aus diesen ungesättigten Fettsäuren in Form ihrer Triglyceride und/oder in nicht glyceringebundener, freier Form.

Vorzugsweise werden die ungesättigten Fettsäuren aus der Gruppe ausgewählt, welche Ölsäure, Eicosapentaensäure, Timnodonsäure, Docosahexaensäure, Arachidonsäure, Linolsäure, α-Linolensäure, γ-Linolensäure sowie Mischungen der vorgenannten Fettsäuren umfasst. Diese Mischungen umfassen insbesondere Mischungen der reinen ungesättigten Verbindungen.

Als Öle werden bevorzugt Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle eingesetzt. Insbesondere geeignet sind Mischungen der reinen ungesättigten Verbindungen.

Fischöl und Lebertran enthalten hauptsächlich Eicosapentaensäure (EPA C20:5) und Docosahexaensäure (DHA C22:6) neben wenig alpha-Linolensäure (ALA C18:3). Bei allen drei Fettsäuren handelt es sich um Omega-3-Fettsäuren, die im Organismus als bedeutende biochemische Bausubstanz für zahlreiche Zellstrukturen benötigt werden (DHA und EPA), zum Beispiel sind sie wie bereits erwähnt, wesentlich für den Aufbau und Bestand der Zellmembran (Sphingolipide, Ceramide, Ganglioside) verantwortlich. Omega-3-Fettsäuren finden sich nicht nur in Fischöl, sondern auch in Pflanzenölen. Weitere ungesättigte Fettsäuren, wie die Omega-6-Fettsäuren, sind in Ölen pflanzlicher Herkunft vorhanden, die hier teils einen höheren Anteil als in tierischen Fetten ausmachen. Daher werden verschiedene Pflanzenöle wie Leinöl, Walnussöl, Flachsöl, Nachtkerzenöl mit entsprechend hohem Gehalt an essentiellen Fettsäuren als besonders hochwertige und wertvolle Speiseöle empfohlen. Vor allem Leinöl stellt ein wertvolle Lieferant von Omega-3- und Omega-6-Fettsäuren dar und ist als hochwertiges Speiseöl seit Jahrzehnten bekannt.

Als an der Polymerisationsreaktion teilnehmende Substanzen sind die Omega-3 als auch die Omega-6-Fettsäuren bevorzugt sowie sämtliche Substanzen, welche mindestens einen Omega-3 und/oder Omega-6-Fettsäurerest tragen. Derartige Substanzen zeigen auch eine gute Befähigung zur Autopolymerisation.

Die Fähigkeit auszuhärten, d.h. die Fähigkeit zur Autopolymerisation, ist in der Zusammensetzung der auch als trocknende Öle bezeichneten Öle begründet und geht auf den hohen Gehalt an essentiellen Fettsäuren, genauer auf die Doppelbindungen der ungesättigten Fettsäuren zurück. An der Luft werden mit Hilfe des Sauerstoffs an den Doppelbindungsstellen der Fettsäuremoleküle Radikale gebildet, die die radikalische Polymerisation initiieren und propagieren, so dass die Fettsäuren unter Verlust der Doppelbindungen untereinander vernetzen. Durch die Aufhebung der Doppelbindung im Fettmolekül steigt der Schmelzpunkt und die Vernetzung der Fettsäuremoleküle bewirkt eine zusätzliche Härtung. Es entsteht ein hochmolekulares Harz, dass als flexibler Polymerfilm die medizinische Oberfläche gleichmäßig bedeckt.

Die Autopolymerisation wird auch als Seibstpolymerisation bezeichnet und kann beispielsweise durch Sauerstoff, insbesondere Luftsauerstoff initiiert werden. Diese Autopolymerisation kann auch unter Lichtausschluss durchgeführt werden. Eine weitere Möglichkeit besteht in der Initiierung der Autopolymerisation durch elektromagnetische Strahlung, insbesondere Licht. Eine weitere aber weniger bevorzugt Variante stellt die Autopolymerisation ausgelöst durch chemische Zerfallsreaktionen dar, insbesondere Zerfallsreaktionen der zu polymerisierenden Stoffe.

Je mehr Mehrfachbindungen der Fettsäurerest aufweist, desto höher ist der Vernetzungsgrad. Somit ist die Menge an Substanzen, welche aktiv an der Polymerisationsreaktion teilnehmen um so geringer, je höher die Dichte an Mehrfachbindungen in einem Alkylrest (Fettsäurerest) als auch in einem Molekül ist.

Der Gehalt an aktiv an der Polymerisationsreaktion teilnehmenden Substanzen in Bezug auf die Gesamtmenge aller auf die Oberfläche des Medizinproduktes aufgetragenen Substanzen beträgt mindestens 25 Gew.-%, bevorzugt 35 Gew.-%, weiter bevorzugt 45 Gew.-% und insbesondere bevorzugt 55 Gew.-%.

Die folgende Tabelle 1 zeigt eine Aufstellung der Fettsäurebestandteile in verschiedenen Ölen, welche bei der vorliegenden Erfindung bevorzugt eingesetzt werden.

Tabelle 1

| Ölsorte | Ölsäure (C 18:1) Omega-9 | Linolsäure (C 18:2) Omega-6 | Linolensäure (C 18:3) Omega-3 | Eicosapentaensäure (C 20:5) Omega-3 | Docosaexaensäure (C 22:6) Omega-3 |
|---|---|---|---|---|---|
| Olivenöl | 70 | 10 | 0 | 0 | 0 |
| Maisöl | 30 | 60 | 1 | 0 | 0 |
| Leinöl | 20 | 20 | 60 | 0 | 0 |
| Lebertran | 25 | 2 | 1 | 12 | 8 |
| Fischöl | 15 | 2 | 1 | 18 | 12 |

Die bei der erfindungsgemäßen Beschichtung eingesetzten Öle bzw. die Mischungen der Öle enthalten einen Anteil von mindestens 40 Gew.-% an ungesättigten Fettsäuren, bevorzugt einen Anteil von 50 Gew.-%, weiter bevorzugt einen Anteil von 60 Gew.-%, noch weiter bevorzugt einen Anteil von 70 Gew.-% und insbesondere bevorzugt einen Anteil von 75 Gew.-% an ungesättigten Fettsäuren. Sollten kommerziell erhältliche Öle, Fette oder Wachse verwendet werden, welche einen geringeren Anteil als 40 Gew.-% an Verbindungen mit mindestens einer Mehrfachbindung enthalten, so können ungesättigte Verbindungen in dem Maße zugesetzt werden, dass der Anteil an ungesättigten Verbindungen auf über 40 Gew.-% ansteigt. Bei einem Anteil von weniger als 40 Gew.-% sinkt die Polymerisationsgeschwindigkeit zu stark ab, so dass gleichmäßige Beschichtungen nicht mehr gewährleistet werden können.

Die Eigenschaft zur Polymerisation macht vor allem die Lipide mit hohen Anteilen mehrfach ungesättigter Fettsäuren zu hervorragenden Substanzen für die vorliegende Erfindung.

So besitzt die Linolsäure (Octadecadiensäure) zwei Doppelbindungen und die Linolensäure (Octadecatriensäure) drei Doppelbindungen. Eicosapentaensäure (EPA C20:5) hat fünf Doppelbindungen und Docosahexaensäure (DHA C22:6) besitzt sechs Doppelbindungen in einem Molekül. Mit der Anzahl der Doppelbindungen steigt auch die Bereitschaft zur Polymerisation.

Diese Eigenschaften der ungesättigten Fettsäuren und deren Mischungen sowie deren Neigung zur Autopolymerisation lässt sich zur biokompatiblen und flexiblen Beschichtung von medizinischen Oberflächen, speziell von Stents mit z.B. Fischöl, Lebertran oder Leinöl nutzen (siehe Beispiele 13-18).

Linolsäure wird auch als cis-9, cis-12-Octadecadiensäure (chemische Nomenklatur) oder als $\Delta$9,12-Octadecadiensäure oder als Octadecadiensäure (18:2) bzw. Octadecadiensäure 18:2 (n-6) bezeichnet (biochemische bzw. physiologische Nomenklatur). Bei Octadecadiensäure 18:2 (n-6) ist n die Anzahl an Kohlenstoffatomen und die Zahl "6" gibt die Position der letzten Doppelbindung an. 18:2 (n-6) ist somit eine Fettsäure mit 18 Kohlenstoffatomen, zwei Doppelbindungen und einem Abstand von 6 Kohlenstoffatomen von der letzten Doppelbindung bis zur äußeren Methylgruppe.

Bevorzugt werden für die vorliegende Erfindung die folgenden ungesättigten Fettsäuren als Substanzen eingesetzt, welche an der Polymerisationsreaktion teilnehmen bzw. Substanzen, welche diese Fettsäuren enthalten oder Substanzen, welche den Alkylrest dieser Fettsäuren, also ohne die Carboxylatgruppe (-COOH), enthalten.

**Tabelle 1: Monoolefinische Fettsäuren**

| Systematischer Name | Trivialname | Kurzform |
|---|---|---|
| cis-9-Tetradecensäure | Myristoleinsäure | 14:1(n-5) |
| cis-9-Hexadecensäure | Palmitoleinsäure | 16:1(n-7) |
| cis-6-Octadecensäure | Petroselinsäure | 18:1(n-12) |

(fortgesetzt)

| Systematischer Name | Trivialname | Kurzform |
|---|---|---|
| cis-9-Octadecensäure | Ölsäure | 18:1(n-9) |
| cis-11-Octadecensäure | Vaccensäure | 18:1(n-7) |
| cis-9-Eicosensäure | Gadoleinsäure | 20:1(n-11) |
| cis-11-Eicosensäure | Gondoinsäure | 20:1(n-9) |
| cis-13-Docosensäure | Erucinsäure | 22:1(n-9) |
| cis-15-Tetracosensäure | Nervonsäure | 24:1(n-9) |
| t9-Octadecensäure | Elaidinsäure | |
| t11-Octadecensäure | t-Vaccensäure | |
| t3-Hexadecensäure | | trans-16:1 n-13 |

Tabelle 2: Polyungesättigte Fettsäuren

| Systematischer Name | Trivialname | Kurzform |
|---|---|---|
| 9,12-Octadecadiensäure | Linolsäure | 18:2(n-6) |
| 6,9,12-Octadecatriensäure | γ-Linolsäure | 18:3(n-6) |
| 8,11,14-Eicosatriensäure | Dihomo-γ-linolensäure | 20:3(n-6) |
| 5,8,11,14-Eicosatetraensäure | Arachidonsäure | 20:4(n-6) |
| 7,10,13,16-Docosatetraensäure | - | 22:4(n-6) |
| 4,7,10,13,16-Docosapentaensäure | - | 22:5(n-6) |
| 9,12,15-Octadecatriensäure | α-Linolensäure | 18:3(n-3) |
| 6,9,12,15-Octadecatetraensäure | Stearidonsäure | 18:4(n-3) |
| 8,11,14,17-Eicosatetraensäure | - | 20:4(n-3) |
| 5,8,11,14,17-Eicosapentaensäure | EPA | 20:5(n-3) |
| 7,10,13,16,19-Docosapentaensäure | DPA | 22:5(n-3) |
| 4,7,10,13,16,19-Docosahexaensäure | DHA | 22:6(n-3) |
| 5,8,11-Eicosatriensäure | Meadsäure | 20:3(n-9) |
| 9c 11t 13t Eleostearinsäure | | |
| 8t 10t 12c Calendinsäure | | |
| 9c 11t 13c Catalpicsäure | | |
| 4, 7, 9, 11, 13, 16, 19 Docosaheptadecansäure | Stellaheptaensäure | |
| | Taxolsäure | all-cis-5,9-18:2 |
| | Pinolensäure | all-cis-5,9,12-18:3 |
| | Sciadonsäure | all-cis-5,11,14-20:3 |

Tabelle 3: Acetylenische Fettsäuren

| Systematische Name | Trivialname |
|---|---|
| 6-Octadecinsäure | Taririnsäure |
| t11-Octadecen-9-insäure | Santalbin- oder Ximeninsäure |

(fortgesetzt)

| Systematische Name | Trivialname |
|---|---|
| 9-Octadecinsäure | Stearolinsäure |
| 6-Octadecen-9-insäure | 6,9-Octadeceninsäure~ |
| t10-Heptadecen-8-insäure | Pyrulinsäure |
| 9-Octadecen-12-insäure | Crepenynsäure |
| t7,t11-Octadecadiene-9-insäure | Heisterinsäure |
| t8,t10-Octadecadiene-12-insäure | - |
| 5,8,11,14-Eicosatetrainsäure | ETYA |

Nach der Durchführung der beschriebenen Polymerisation der einen linearen oder verzweigten und einen substituierten oder unsubstituierten Alkylrest mit mindestens einer Mehrfachbindung enthaltenden Substanzen, wird eine Oberfläche eines Medizinproduktes erhalten, welche zumindest teilweise mit einer polymeren Schicht versehen ist. Im Idealfall entsteht eine homogene gleichmäßig dicke polymere Schicht auf der gesamten äußeren Oberfläche des Stents oder eines Katheterballons mit oder ohne aufgesetztem Stent. Diese polymere Schicht auf der Oberfläche des Stents oder des Katheterballons mit oder ohne Stent besteht aus den polymerisierbaren Substanzen und schließt die nicht aktiv an der Polymerisationsreaktion teilnehmenden Substanzen und/oder Wirkstoffe und/oder Rapamycin in der Polymermatrix ein. Vorzugsweise ist der Einschluss so ausgestaltet, dass die nicht an der Polymerisation teilnehmenden Substanzen, vor allem Rapamycin und Paclitaxel und zusätzliche Wirkstoffe aus der Polymermatrix hinausdiffundieren können.

Der biokompatible Überzug aus den polymerisierten Substanzen sorgt für die notwendige Blutverträglichkeit des Stents oder des Katheterballons mit oder ohne Stent und stellt gleichzeitig einen geeigneten Träger für einen Wirkstoff wie Paclitaxel oder Rapamycin dar. Ein hinzugefügter Wirkstoff (oder Wirkstoffkombination), der über die Gesamtoberfläche des Stents und/oder Katheterballons gleichmäßig verteilt ist, bewirkt, dass der Bewuchs der Oberfläche mit Zellen, insbesondere glatten Muskel- und Endothelzellen, in einer kontrollierten Weise abläuft. Es findet somit keine rasche Besiedlung und Überwucherung einer Stentoberfläche mit Zellen statt, was zu einer Restenose führen könnte, wohingegen der Bewuchs der Stentoberfläche mit Zellen aber auch nicht durch eine hohe Medikamentenkonzentration vollkommen unterbunden wird, was die Gefahr einer Thrombose mit sich bringt. Diese Kombination beider Effekte verleiht der erfindungsgemäßen Oberfläche eines Medizinprodukts, insbesondere der Oberfläche eines Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 12 Monaten, bevorzugt über 1 - 2 Monate nach der Implantation.

Ein herkömmlicher Katheterballon wird vorzugsweise in einem ersten Schritt mit einem Gleitmittel wie z.B. Graphit oder einem Stearat beschichtet und danach vorzugsweise mittels eines Spritzverfahrens mit einer viskosen Mischung eines Öls oder Fetts und eines Wirkstoffs wie z.B. Rapamycin oder Paclitaxel beschichtet. Falls erforderlich kann danach eine geringe Aushärtung durch Autopolymerisation initiiert durch Sauerstoffmoleküle oder durch Strahlung und/oder Radikalbildner erfolgen. Dadurch ergibt sich auf der Katheterballonoberfläche eine glatte Oberfläche, welche in der Regel keines weiteren Schutzes vor frühzeitigen Ablösung bedarf. Der Katheterballon wann in der vorliegenden Form bis zur verengten Gefäßstelle vorgeschoben werden und dort kann die Übertragung der Beschichtung auf die Gefäßwand mittels Dilatation des Ballons durchgeführt werden, wobei das Gleitmittel direkt auf der Ballonoberfläche die Ablösung der öligen Beschichtung unterstützt.

**Liposomale Formulierungen**

Weitere bevorzugte Ausführungsformen der vorliegenden Erfindung betreffen liposomale Formulierungen von Wirkstoffen für die Beschichtung von Katheterballons mit oder ohne Stents.

Die liposomalen Formulierungen werden vorzugsweise hergestellt, indem in einem ersten Schritt der Wirkstoff (z.B. Paclitaxel oder Rapamycin) bzw. die Wirkstoffkombination in einem wässrigen Medium oder Puffermedium gelöst und anschließend mit Lösungen, die membranbildende Substanzen enthalten, in Kontakt gebracht wird. Bei diesem Verfahren ergeben sich hohe Einschlussraten von mindestens 30% bis zu 95%.

Membranbildende Substanzen sind geladene amphiphile Verbindungen bevorzugt Alkylcarbonsäuren, Alkylsulfonsäuren, Alkylamine, Alkylammoniumsalze, Phosphorsäureester mit Alkoholen, natürliche sowie synthetische Lipide, wie Phosphatidylglycerol (PG), Phosphatidylserin (PS), Derivate des Phophatidylethanolamins (PE-Derivate) sowie des Cholesterols, Phosphatidsäure, Phosphatidylinositol, Cardiolipin, Sphingomyelin, Ceramid in seinen natürlichen, halbsynthetischen oder synthetischen Formen, Stearylamin und Stearinsäure, Palmitoyl-D-glucuronid und/oder geladene Sphingolipide, wie z. B. Sulfatid.

Als neutrale membranbildende Substanzen fungieren an sich bekannte Komponenten wie z. B. Phosphatidylcholin (PC), Phophatidylethanolamin (PE), Steroide, vorzugsweise Cholesterol, komplexe Lipide und/oder neutrale Sphingolipide.

Die Gewinnung der Liposomen aus der wässrigen Lösung erfolgt ebenfalls nach an sich bekannten Techniken so z. B. durch Dialyse, Ultrafiltration, Gelfiltration, Sedimentation oder Flotation. Die Liposomen besitzen einen durchschnittlichen Durchmesser von 10 bis 400 nm.

Derartige liposomale Formulierungen lassen sich auch bevorzugt in die Falten eines Faltenballons einbringen.

Magnetische Partikel enthaltende Beschichtung

Eine weitere erfindungsgemäße Beschichtung von Katheterballons enthält magnetische und/oder endozytosefähige Partikel vorzugsweise mit einem durchschnittlichen Partikeldurchmesser im Nanometer- bis Mikrometer-Bereich wie sie z.B. in DE 197 26 282 A offenbart sind.

Es ist bekannt, dass Nanopartikel von Zellen durch Endozytose aufgenommen werden können. Ein Verfahren zur Herstellung von zellgängigen Nanopartikeln ist in DE 197 26 282.1 genannt. Die Aufnahme der Nanopartikel kann durch in-vitro Untersuchungen an hochreinem Zellmaterial untersucht werden. In der DE 199 12 798 C1 sind Methoden genannt, mit deren Hilfe man beliebige Zellen aus Gewebematerial in Kultur bringen kann. Durch diese Methoden ist es möglich, die Partikel chemisch so zu gestalten, dass eine hohe Aufnahme in bestimmte Zelltypen stattfindet. So wird in DE 100 59 151 A eine Ankopplung von Substanzen wie beispielsweise auch Paclitaxel und Rapamycin an die Partikel durch ionische Wechselwirkung verfolgt, wobei das Konjugat im Gewebe angereichert werden soll.

Bei den magnetischen Partikeln kann eine Beschichtung vorzugsweise aus monomeren Aminosilanen wie z.B. 3-Aminopropyltriethoxysilan, 2-Aminoethyl-3-aminopropyltrimethoxysilan, Trimethoxysilylpropyldiethylentriamin oder N-(6-Aminohexyl)-3-aminopropyltrimethoxysilan bestehen, welche zur Erzielung der erforderlichen Stabilität nach bekannten Verfahren polykondensiert werden. Ein geeignetes Verfahren ist beispielsweise in DE 196 14 136 A oder DE 195 15 820 A beschrieben.

Ferner ist bekannt, dass sich derartige magnetische Partikel mittels eines von außen angelegten Magnetfeldes lokal anreichern lassen (DE 100 59 151 A) oder sich die Zielfindungseigenschaften auf chemischem Wege beispielsweise durch die Ankopplung von Antikörpern steigern lassen (DE 44 28 851 A1, EP 0516252 A2). Mehrschalige Teilchen zur Einschleusung von Partikel-Wirkstoff-Konjugaten in Zellen, insbesondere Tumorzellen werden in der Patentschrift WO 98/58673 A beschrieben. Ferner kann durch das Anlegen eines äußeren magnetischen Wechselfeldes auch eine Erwärmung der Partikel z.B. aufgrund von Hysteresewärme auf beispielsweise 45°C und mehr erreicht werden.

Die Nanopartikel selbst bestehen aus einem magnetischen Material, vorzugsweise einem ferromagnetischen, antiferromagnetischen, ferrimagnetischen, antiferrimagnetischen oder superparamagnetischen Material, insbesondere aus superparamagnetischen Eisenoxiden oder aus reinem Eisen, welches mit einer Oxidschicht versehen ist. Die Nanopartikel bestehen vorzugsweise aus Eisenoxiden und insbesondere aus Magnetit ($Fe_3O_4$), Maghemit ($\gamma$-$Fe_2O_3$) oder Mischungen dieser beiden Oxide. Allgemein können die bevorzugten Nanopartikel durch die Formel $FeO_x$ wiedergegeben werden, worin X eine Zahl von 1 bis 2 bedeutet. Die Nanopartikel weisen vorzugsweise einen Durchmesser von weniger als 500 nm auf. Vorzugsweise besitzen die Nanopartikel einen durchschnittlichen Durchmesser von 15 nm oder liegen vorzugsweise in dem Größenbereich von 1-100 nm und insbesondere bevorzugt im Bereich von 10-20 nm.

Neben den magnetischen Materialien der Formel $FeO_x$, worin X eine Zahl im Bereich von 1,0 bis 2,0 ist, sind erfindungsgemäß auch Materialien der allgemeinen Formel $M(II)Fe_2O_4$ mit M = Co, Ni, Mn, Zn, Cu, Cd, Ba oder andere Ferrite einsetzbar. Der Gehalt an von Eisenatomen verschiedenen Metallatomen beträgt vorzugsweise nicht mehr als 70 Metallatom-%, insbesondere nicht mehr als 35 Metallatom-%. Bevorzugt bestehen die Nanopartikel jedoch zu über 98 Gew.-% aus Eisenoxid, welches sowohl Fe(III) als auch Fe(II) in einem Verhältnis von vorzugsweise 1 zu 1 bis 1 zu 3 enthält. Ferner eignen sich auch Silica- oder Polymerpartikel, in die magnetische Materialien wie beispielsweise die hierin genannten magnetischen Materialien eingelagert und/oder angebunden sind.

Die verwendeten Nanopartikel-Kerne können auch aus nichtmagnetischen Materialien bestehen. In Frage kommen z.B. Nanopartikel aus Polymeren (z.B. PLGA, Polyacrylamid, Polybutylcyanoacrylat), Metallen sowie aus sämtlichen oxidischen Materialien (z.B. MgO, CaO, $TiO_2$, $ZrO_2$, $SiO_2$, $Al_2O_3$). Erfindungsgemäß eignet sich jedes Material, dass sich mit den oben beschriebenen Methoden mit tumorspezifischen Hüllen beschichten lässt, da die Endozytosefähigkeit nicht vom Kern der Partikel, sondern von der Hülle abhängt.

An diese Nanopartikel können therapeutisch wirksame Substanzen gebunden werden, wobei eine kovalente Bindung als auch adsorptive Bindungen und ionische Bindungen möglich sind.

Erfindungsgemäß basieren die aktivierbaren Nanopartikel-Wirkstoff-Konjugate bevorzugt auf magnetischen eisenhaltigen Kernen, die von einer oder mehreren kolloidalen Hüllen oder Beschichtungen umgeben sind, welche eine Möglichkeit zur Ankopplung der Wirkstoffe über funktionelle Gruppen bieten. Der Kern besteht dabei vorzugsweise aus

Magnetit oder Maghemit. Die primäre Funktion der Hüllen ist es, eine kolloidale Verteilung im wässrigen Medium zu erreichen und die Nanopartikel vor Agglomerationen zu schützen. Mehrschalig umhüllte Partikel, wie sie in WO 98/58673 beschrieben werden, sind prinzipiell als Basis für die aktivierbare Nanopartikel-Wirkstoff-Konjugate geeignet, da das biologische Verhalten solcher Partikel durch Überbeschichtungen mit Polymeren einstellbar und eine Ankopplung der Wirkstoffe an funktionelle Gruppen der Primärhülle möglich ist.

Eine weitere Beschichtung der aktivierbaren Nanopartikel-Wirkstoff-Konjugate (z.B. durch Polymere), wie sie in Patentschrift WO 98/58673 A beschrieben wird, ist ebenfalls möglich, und kann genutzt werden, um die biologischen Eigenschaften der Partikel-Wirkstoff-Konjugate zu verbessern.

Erfindungsgemäß werden somit Katheterballons mit einer Beschichtung versehen, welche magnetische und/oder endozytosefähige Partikel enthält. Die Beschichtung kann des weiteren vorzugsweise ein oder mehrere Polymere umfassen, worin die magnetischen und/oder endozytosefähigen Partikel zusammen mit dem Wirkstoff wie z.B. Paclitaxel oder Rapamycin oder der Wirkstoffkombination eingelagert werden. Zudem ist es auch möglich eine Mischung aus einem Kontrastmittel oder Kontrastmittelanalogon zusammen mit einem Wirkstoff und den magnetischen und/oder endozytosefähigen Partikeln auf die Ballonoberfläche mit oder ohne gekrimpten Stent aufzubringen. Des weiteren kann eine Lösung oder Dispersion aus magnetischen und/oder endozytosefähigen Partikeln und dem Wirkstoff in einem vorzugsweise leicht flüchtigen Lösungsmittel wie z.B. Aceton, Methanol, Ethanol, Tetrahydrofuran (THF), Methylenchlorid, Chloroform, Ether, Petrolether, Essigsäureethylester und -methylester, Cyclohexan, Hexan, und anderen organischen Lösungsmitteln mit Siedepunkten unterhalb von 100°C hergestellt werden, welche dann vorzugsweise im Sprühverfahren auf den Katheterballon mit oder ohne gekrimpten Stent aufgetragen wird.

Der Wirkstoff kann wie oben erwähnt adhäsiv oder auch kovalent an die Außenhülle der magnetischen und/oder endozytosefähigen Partikel gebunden werden oder die magnetischen und/oder endozytosefähigen Partikel werden zusammen mit dem Wirkstoff oder Wirkstoffgemisch (z.B. Rapamycin und/oder Paclitaxel) in Mikrokapseln oder liposomalen Formulierungen eingeschlossen und in dieser Form auf die Ballonoberfläche aufgebracht.

Derartige Schichten aus Wirkstoff und magnetischen und/oder endozytosefähigen Partikeln können selbstverständlich mit einer weiteren schützenden als auch die Freisetzung kontrollierenden Schicht überzogen werden.

Als Außenschichten für die Beschichtung von Katheterballons eignen sich insbesondere hart bei der Dilatation aufbrechende Schichten oder Beschichtungen, welche dem Ballon eine besonders gute Gleitfähigkeit verleihen und nur eine geringen Wechselwirkung bzw. Gleitreibung gegenüber der Gefäßwand aufweisen.

Bei einer besonderes bevorzugten Ausführungsform umfassend eine Beschichtung enthaltend magnetische und/oder endozytosefähige Partikel werden diese Partikel bzw. wird die Beschichtung, welche diese Partikel enthält auf der Ballonoberfläche durch ein äußeres Magnetfeld fixiert. Bei einer anderen Ausführungsform wird im Inneren des Katheterballons oder in dessen Außenhülle ein umpolbarer Magnet wie z.B. ein Elektromagnet angeordnet, der während des Einführens des Katheters die gegensätzlich gepolten magnetischen Partikel anzieht und somit an die Ballonoberfläche fest bindet. Bei der Dilatation des Ballons wird dann der im Ballon befindliche Magnet umgepolt und stößt die gleichpoligen bzw. gleich magnetisierten Partikel ab, wodurch die magnetischen Partikel in die Gefäßwand und in die einzelnen Zellen insbesondere die glatten Muskelzellen gedrückt werden.

Diese Ausführungsform gewährleistet eine feste Anhaftung der magnetischen Partikel während des Einführens des Katheterballons aufgrund von Magnetismus, sei es durch ein äußeres lokales Magnetfeld oder bevorzugt durch ein im Katheterballon erzeugtes Magnetfeld und führt zudem zu einer quantitativen Abstoßung der magnetischen Teilchen und Übertragung auf das umliegende Gewebe bei der Expansion des Katheterballons.

Bei diesem Verfahren genügen sehr kurze Dilatationszeiten des Katheterballons von weniger als 30 Sekunden, vorzugsweise 5 - 20 Sekunden, weiter bevorzugt 5 - 10 Sekunden und insbesondere bevorzugt drei bis 6 Sekunden.

Da der oder die Wirkstoffe durch Adsorption fest an die magnetischen Partikel oder durch kovalente Verknüpfung eventuell auch über einen Linker an die Oberfläche der magnetischen Partikel oder durch Einlagerung in eine Oberflächenbeschichtung auf den magnetischen Kernen der Partikel somit auch fest mit den magnetischen Partikeln verbunden sind, ist der Wirkstoffverlust während der Einführung des Katheters ebenfalls sehr gering.

Zudem können die magnetischen Partikel mit einer Beschichtung versehen werden, welche eine besonders hohe Affinität zu glatten Muskelzellen besitzt und eine geringere Affinität zu Endothelzellen, so dass über die Beschichtung der magnetischen Mikro- bzw. Nanopartikel zudem gesteuert werden kann, dass bevorzugt glatte Muskelzellen abgetötet oder in ihrer Proliferation gehemmt werden, während die Endothelzellen weitgehend verschont bleiben, was sich bei der Restenoseprophylaxe und Restenosebehandlung sehr positiv auswirkt. Ferner kann über die Menge an aufgebrachtem Wirkstoff gesteuert werden, ob z.B. Paclitaxel eher eine zytotoxische Wirkung oder eine zytostatische Wirkung ausübt.

Da die Wirkstoffe relativ fest jedoch in der Regel nicht irreversibel an die magnetischen Partikel gebunden sind, werden die Wirkstoffe samt der Magnetpartikel in die Zellen, vorzugsweise die glatten Muskelzellen, eingeschleust und entfalten in der Zelle ihre Wirkung, was zu einer deutlichen Wirkungssteigerung des Wirkstoffs führt.

**Hydrogel**

Bei einer anderen erfindungsgemäßen Ausführungsform wird auf den Katheterballon mit oder ohne Stent ein Hydrogel aufgetragen, welches mindestens einen der oben genannten Wirkstoffe, vorzugsweise Paclitaxel oder Rapamycin oder deren Derivate, enthält.

Vorzugsweise wird eine solche Hydrogelbeschichtung durch einen äußeren Schutzmantel wie er für selbstexpandierende Nitinol-Stents verwendet wird, so lange vor dem Kontakt mit Blut geschützt, bis sich der Katheterballon an der verengten Gefäßstelle befindet. Dort wird die Schutzhülle entfernt und das Hydrogel beginnt bei Kontakt mit Blut zu quellen. Die Expansion des Katheterballons überträgt den Großteil der Hydrogelschicht auf die Gefäßwand und verbleibt dort als ein kurzzeitiges Wirkstoffreservoir, welche bis zur Auflösung der Hydrogelschicht innerhalb weniger Tage bis Wochen weiterhin Wirkstoff z.B. Paclitaxel oder Rapamycin an die Gefäßwand abgibt.

**Salze mit Wirkstoff**

Eine besonders bevorzugte Ausführungsform der vorliegenden Erfindung ist eine Beschichtung eines Katheterballons vorzugsweise ohne Stent mit einer Lösung oder Dispersion eines Wirkstoffs vorzugsweise Paclitaxel oder Rapamycin oder deren Derivate und insbesondere Paclitaxel zusammen mit einem oder mehreren physiologisch verträglichen Salzen.

Als Salze können Verbindungen enthaltend Natriumkationen, Calcium-, Magnesium-, Zink-, Eisen- oder Lithiumkationen zusammen mit Sulfat-, Chlorid-, Bromit-, Iodid-, Phosphat-, Nitrat-, Citrat- oder Acetatanionen eingesetzt werden.

Dieser Lösung, Dispersion oder Aufschlämmung wird der Wirkstoff oder die Wirkstoffkombination zugesetzt. Als Lösungsmittel dient vorzugsweise Wasser eventuell mit Cosolventien. Der Salzgehalt sollte relativ hoch liegen.

Der Katheterballon wird mittels Tauchverfahren oder Sprühverfahren oder Pinselverfahren oder Spritzenverfahren mit dieser wirkstoffenthaltenden Salzlösung beschichtet und danach getrocknet, so dass sich eine feste Salzkruste auf dem Katheterballon ergibt. Als Salze können zudem auch ionische Kontrastmittel eingesetzt werden oder ionische Kontrastmittel können den oben genannten Salzen zugesetzt werden.

Ziel ist es, auf dem Katheterballon eine weitgehend durchgehende Beschichtung aus festem Material, d.h. Salz zu erzeugen, worin der Wirkstoff eingeschlossen ist. Diese Salzkruste wird dann entweder mittels einer schützenden Deckschicht oder einer entfernbaren Schutzhülle wie sie die selbstexpandierenden Stents verwendet wird versehen, um sie vor vorzeitiger Ablösung zu schützen. Eine dritte Variante besteht darin, einen Faltenballon zu verwenden und diese Salzmischung gezielt unter die Falten des Katheterballon einzubringen.

Die Salzbeschichtung ist sehr hygroskopisch und besitzt daher eine hohe Affinität zum Gefäßgewebe. Bei der Dilatation wird die Schutzhülle entfernt oder die äußere schützende Barriereschicht platzt auf oder bei Verwendung eines Faltenballons öffnen sich die Falten und drücken die salzige Beschichtung gegen die Gefäßwand.

Die Salzbeschichtung klebt dann regelrecht an der Gefäßwand, wo sie mehrere Aufgaben erfüllt. Zum einen führt die lokale sehr hohe Salzkonzentration zu einem hohen isotonischen Druck, der Zellen zum Platzen bringt und zum anderen löst die hohe Salzkonzentration auch harte Plaque und andere Ablagerungen im Gefäß auf und setzt zudem noch den Wirkstoff frei, welche insbesondere die Proliferation der glatten Muskelzellen unterbindet.

Nach wenigen Stunden bis zu ein paar Tagen ist je nach Menge die auf die Gefäßwand übertragene Salzbeschichtung vollständig aufgelöst.

**Beschichtungsverfahren**

Ferner betrifft die vorliegende Erfindung Verfahren zur Beschichtung von Katheterballons mit oder ohne gekrimptem Stent.

Das Kurzzeitimplantat wird entweder mit einer Lösung der aufzutragenden Stoffe inklusive dem Wirkstoff oder dem Wirkstoffgemisch in Sprüh-, Tauch-, Pinsel-, Spritzen-, Schlepp-, Roll- oder Pipettierverfahren oder Elektrospinning vollständig oder partiell oder in Kombination mit einer Matrix vollständig oder teilweise beschichtet.
Als Lösungsmittel werden leichtflüchtige organische Verbindungen verwendet wie beispielsweise Dichlormethan, Chloroform, Ethanol, Aceton, Heptan, n-Hexan, DMF, DMSO, Methanol, Propanol, Tetrahydrofuran (THF), Methylenchlorid, Ether, Petrolether, Acetonitril, Essigsäureethyl- und methylester, Cyclohexan und entsprechende Mischungen. Je nach Beschichtungsmaterial (z:B. Hydrogele oder wasserisl. Wirkstoffe)
kann auch die Anwesenheit von Wasser erwünscht sein.

Allgemein ist bei der Wahl des Lösungsmittel es vor allem wichtig, dass es das Material des Kurzzeitimplantates nicht auflöst bzw. unbrauchbar macht bzw. die Kontaktzeit des Lösungsmittels so gering ist, dass keine Schädigung entstehen kann.

Die Matrix besteht aus einem den Implantat-Ansprüchen genügendes synthetisches, semisynthetisches oder

natürliches, biostabiles oder bioabbaubares, biokompatibles Polymer bzw. Polymermischung, Präpolymeren, polymerisierbaren Substanzen wie ungesättigte Fettsäuren, Micellen- bzw. Liposombildenden Substanzen, die Wirkstoffe einkapseln. Geeignete Polymere sind oben erwähnt. Hiermit lässt sich zusätzlich eine Depotwirkung und Dosierungserhöhung erzielen.

Der Katheterballon lässt sich entweder im expandierten oder im gefalteten Zustand beschichten, teilweise oder vollständig beschichten oder zusammen mit einem aufgesetzten Stent beschichten.
Die Beschichtung ist im Sprüh-, Tauch-, Pinsel-, Spritzen-, Schlepp-, Roll- und/oder Pipettierverfahren durchführbar. Das Pipettier-, Schlepp-, Roll- oder Spritzenverfahren eignet sich vor allem für die Anwendung auf einem gefalteten Katheterballon oder einem Faltenballon, da man mit diesem Verfahren gezielt die Wirkstofflösung oder die Mischung der aufzutragenden Substanzen in die Falten bzw. unter die Falten bringen kann. Dabei ist wichtig, dass durch diese partielle Beschichtung keine Beeinträchtigung der Funktionalität verbunden ist. So dürfen die Falten beim Expandieren beispielsweise nicht zusammenkleben und damit einer Expansion entgegenwirken. Desgleichen sollte der nominale Druck auf den Ballon nicht über den maximalen Wert erhöht werden müssen, um Adhäsionskräfte der Beschichtung in den Falten entgegenwirken zu können. Ungleichmäßige Expansion ist ebenfalls zu vermeiden. Die Beschichtung darf die Expansionseigenschaften des Ballonkatheters keinesfalls negativ beeinträchtigen.

Ferner kann der Katheterballon zusammen mit einem gekrimpten Stent beschichtet werden oder ein unbeschichteter Stent (bare stent) als auch ein bereits beschichteter Stent kann auf den beschichteten Katheterballon gekrimpt werden, so dass man ein System aus einem beispielsweise schnell vom Katheterballon freigesetzten Wirkstoff und einem langsam aus der Beschichtung des Stents freigesetzten Wirkstoff erhalten kann.

In Kombination mit einem Stent, der seinerseits beschichtet und einen Wirkstoff abgeben kann, ist ein wirkstofffreisetzender Ballonkatheter von besonderem Vorteil in der Frühphase des Heilungsprozesses, da nur auf diese Art und Weise der vollflächige Kontakt mit der zu behandelnden Stelle zustande kommt und Wirkstoff vollflächig in die erkrankte Gefäßwand gelangt. Der gesamte erkrankte Bereich wird bei Kontakt mit der Ballonkatheterfläche mit Wirkstoff versehen, während der Stent mit einer möglichst geringen Oberfläche nur eine geringe Gefäßwandoberfläche abdeckt.
Im gleichen Masse sollte der Vorteil auch für die Stentrandbereiche gegeben sein, die immer wieder Problematiken aufweisen. Ein Katheterballon, der auch in den Randbereichen des Stents Wirkstoff abgeben kann, sorgt für eine optimale Versorgung des Gefäßes bis in die Problemzonen eines Stents.

Die Salzlösungen sowie die Zusammensetzungen enthaltend Kontrastmittel oder auch die Zusammensetzungen mit Salzen und Kontrastmitteln sind besonders gut geeignet, um Faltenballons oder Katheterballons mit unebener, aufgerauter, poröser oder mikrostrukturierter Oberfläche zu beschichten oder eben diese Mischungen in bzw. unter die Falten der Faltenballons einzubringen.

Die Katheterballons mit besonders beschaffener Oberfläche werden bevorzugt im Sprühverfahren oder Pipettierverfahren beschichtet. Beim Sprühverfahren wird der Katheterballon rotierend aufgehängt und durch Anlegen eines leichten Vakkums wird die Ballonkatheterform stabilisiert. Beispielsweise lässt sich auf diese Weise verhindern, dass sich die Falten eines Faltenballons beim Drehen umklappen bzw. seitlich wegrutschen und damit die Beschichtung nicht lokal gezielt durchgeführt werden kann.
Derart fixiert wird der Ballonkatheter mehrfach kurzzeitig besprüht, wobei er zwischenzeitlich trocknet. Falls erwünscht, wird eine äußere schützende Schicht oder Barriereschicht ebenfalls bevorzugt im Sprühverfahren aufgebracht. Gleiches gilt für reine Wirkstoffschichten aus beispielsweise Paclitaxel oder Rapamycin, welche ebenfalls bevorzugt im Sprühverfahren aufgetragen werden.
Das Pipettierverfahren ist besonders gut zur Beschichtung eines Ballonkatheters geeignet. Hierbei wird der drehbar fixierte Ballonkatheter (mit oder ohne Stent) mit Hilfe einer feinen Düse, die mit einer Kapillaren verlängert ist und durch die die Beschichtungslösung in Längsrichtung auf den Ballonkatheter austritt, beschichtet.

Beim Spritzenverfahren oder Pipettierverfahren zur vorzugsweisen Befüllung der Falten eines Faltenballons wird eine feine Düse oder Kanüle unter die Falte geschoben und die einzubringende Mischung wird in die Falte gespritzt, wobei vorzugsweise die Düse oder Kanüle entlang der Falte bewegt wird oder bei ortfester Düse oder Kanüle der Faltenballon in Längsrichtung der Falte bewegt wird. Dieses Verfahren ermöglicht eine sehr präzise und genaue Beschichtung einer jeden einzelnen Falte bzw. des gesamten Faltenballons. Ein eventuell verwendetes Lösungsmittel verdunstet oder wird im Vakuum entfernt.

Hat die einzubringende Mischung oder Lösung eine Konsistenz, so dass sie in die Falten hineinfließen kann, dann wird der Faltenballon mit einer Falte nach oben horizontal gelagert oder vorzugsweise um 5 bis 25 Grad geneigt, so dass die Spritze oder Düse am unteren Ende des Faltenballons an der Faltenöffnung angesetzt werden kann und die Mischung eigenständig in die Falte hineinfließt und diese voll ausfüllt.

Bei diesen Salzlösungen mit hohem Salzgehalt wird vorzugsweise Wasser als Lösungsmittel eingesetzt, weil Wasser das Ballonmaterial nicht angreift und beschädigt. Sobald die Mischung eine Konsistenz hat, dass sie nicht mehr aus der Falte herausfließen kann, wird der Faltenballon gedreht und die nächste Falte wird befüllt bis in der Regel alle 4 bis 6 Falten des Ballons befüllt sind. Faltenballons werden bevorzugt im komprimierten Zustand beschichtet, wobei einige spezielle Ausführungsformen von Faltenballons auch im expandierten Zustand beschichtet werden können.

Ein solches Beschichtungsverfahren umfasst die Schritte

a) Bereitstellen eines Faltenballons
b) Platzierung einer Falte des Ballons in einer horizontalen oder bis zu 25 Grad geneigten Position,
c) Ansetzen der Spritzenöffnung an die dem Kopfende des Ballons zugewandten Faltenöffnung,
d) Ausführung einer Relativbewegung von Spritzenöffnung und Faltenbation in Längsrichtung der Falte,
e) Befüllung der Falte während des Bewegungsvorgangs mit einer Mischung aus einem Wirkstoff und einem Salz und/oder einem ionischen Kontrastmittel in einem geeigneten Lösungsmittel,
f) sofern erforderlich Trocknung der in der Falte befindlichen Mischung bis zu einem Grad, der ein Auslaufen der Mischung aus der Falte verhindert,
g) Drehung des Ballons um 360° dividiert durch die Anzahl der Falten,
h) Wiederholung der Schritte b) bis g) bis alle Falten befüllt sind und
i) Trocknung der Mischungen in den Falten bis sich die Mischung verfestigt.

Werden dünnflüssigere Mischungen eingesetzt, so wird bei Schritt c) die Spritzenöffnung an das Fußende angesetzt und ohne Relativbewegung gemäß Schritt d) die Falte vorwiegend aufgrund von Kapillarkräften befüllt.

Die vorliegende Erfindung betrifft ferner ein Verfahren zur Offenhaltung von verengten Gefäßdurchgängen insbesondere von kardiovaskulären Gefäßen mittels Kurzzeitdilatation. Bei diesem Verfahren wird innerhalb von maximal 50 Sekunden, bevorzugt maximal 40 Sekunden, weiter bevorzugt maximal 30 Sekunden und insbesondere bevorzugt maximal 20 Sekunden ein Katheterballon ohne Stent expandiert und wieder auf einen Durchmesser kleiner als der 1,5-fache Ausgangsdurchmesser im komprimierten Zustand komprimiert, wobei während dieses Vorgangs das Gefäß nur bis maximal 10% seiner Durchmessers im unverengten Zustand überdehnt wird und pro $mm^2$ Ballonoberfläche mindestens 20% des enthaltenen Wirkstoffs abgegeben und größtenteils auf die Gefäßwand übertragen werden.

Dabei findet die Übertragung des Wirkstoffs vorzugsweise nicht in reiner Form statt sondern in einer Matrix, welche zumindest innerhalb einer Stunde nach erfolgter Dilatation noch als Wirkstoffreservoir wirkt und weiteren Wirkstoff an die Gefäßwand abgibt, bevor sie ausgelöst oder abgebaut worden ist.

Dieses Verfahren zeichnet sich also dadurch aus, in möglichst kurzer Zeit eine möglichst große Wirkstoffmenge lokal und gezielt auf die Gefäßwand einer verengten Gefäßstelle zu übertragen und innerhalb der nachfolgenden 30 bis 60 Minuten bis zu max. 3 Tagen für ein lokales Wirkstoffreservoir zu sorgen, welches danach aufgelöst oder abgebaut ist.

Für dieses Verfahren haben sich vor allem Wirkstoffe, die entzündungshemmende und antiproliferative Eigenschaften in sich vereinigen, als besonders geeignet erwiesen (siehe Wirkstoffliste S. 6-9). Hierzu zählen z.B. Colchicin, Angiopeptin, aber vor allem Rapamycin und seine Derivate, des weiteren zeigen sich weitere hydrophobe Wirkstoffe, insbesondere Paclitaxel und Paclitaxel-Derivate als sehr geeignet.

Ein weiteres erfindungsgemäßes Verfahren betrifft die Beschichtung von Katheterballons mit öligen polymerisierbaren Substanzen. Dieses Verfahren umfasst die Schritte:

a) Bereitstellen eines Katheterballons,
b) Bereitstellen eines Gemisches, welches zu mindestens 50 Gew.-% aus öligen Substanzen mit mindestens einer Mehrfachbindung bestehen und mindestens einen Wirkstoff enthalten,
c) Auftragen eines Gleitmittels auf die Oberfläche des Katheterballons, welches weitgehend das Anhaften der öligen Substanzen auf der Oberfläche des Katheterballons verhindert,
d) Auftragen der öligen Mischung auf das Gleitmittel bzw. die Gleitmittelschicht auf dem Katheterballon,
e) Rotation des Katheterballons während des Beschichtungsschritts d),
f) Initiierung der Polymerisation mittels Licht, Sauerstoff oder Radikalstartern bis hin zu einer nicht harten aber elastischen Polymerschicht,
g) eventuell Wiederholung der Beschichtungsschritte d) bis f).

Die erfindungsgemäßen Faltenbeschichtungsverfahren oder Faltenbefüllungsverfahren sind das Pipettierverfahren auch als Kapillarverfahren bezeichnet, das Spritzverfahren und das Sprühverfahren auch als Faltensprühverfahren bezeichnet, um den Unterschied zum unselektiven Sprühverfahren für den gesamten Katheterballon zu verdeutlichen.

Somit betrifft die vorliegende Erfindung Verfahren zur Beschichtung oder Befüllung der Falten eines Katheterfaltenballons auf folgende Weise:

a) eine wirkstoffenthaltende Zusammensetzung am distalen oder proximalen Ende einer Falte des Katheterfaltenballons abgegeben und die Falte aufgrund von Kapillarkräften befüllt wird; oder
b) eine Spritze, welche einen kontinuierlichen Fluß einer wirkstoffenthaltenden Zusammensetzung abgibt, relativ zum Katheterfaltenballon entlang der Falte bewegt wird; oder

c) eine Vielzahl in Reihe befindlicher Abgabeöffnungen unter die Falte des Faltenballons geschoben werden und gleichzeitig aus der Vielzahl von Abgabeöffnungen eine wirkstoffenthaltende Zusammensetzung in die Falte abgegeben wird.

Vorteilhaft ist, dass diese Beschichtungs- oder Befüllungsverfahren vorzugsweise im komprimierten oder deflatierten Zustand oder maximal 10% inflatierten Zustand des Katheterballons durchgeführt werden. Unter dem Begriff "10% inflatierten Zustand" soll verstanden werden, dass der Katheterballon 10% der Inflation, d.h. der Aufdehnung bis zur bei der Dilatation geplanten Maximalausdehnung erfahren hat. Wird die bei der Dilatation vorgesehene Aufdehnung mit 100% bezeichnet und der deflatierte Zustand mit 0% angesetzt, so ergibt sich eine 10%ige Inflation gemäß folgender Formel:

**(Durchmesser des Katheterballons im deflatierten Zustand)**

**+**

**(Durchmesser des Katheterballons im inflatierten Zustand – Durchmesser des Katheterballons im deflatierten Zustand) / 10**

Ferner können gemäß den erfindungsgemäßen Verfahren mehrere oder alle Falten gleichzeitig beschichtet oder befüllt werden und die Beschichtung oder Befüllung kann gezielt erfolgen. Eine gezielte Befüllung der Falten oder gezielte Beschichtung der Falten soll heißen, dass nur die Falten befüllt oder beschichtet werden und die Oberfläche des Katheterfaltenballons außerhalb der Falten nicht beschichtet wird.

Eine bevorzugt verwendete Zusammensetzung aus Wirkstoff, Lösungsmittel und Matrix wie z.B. Kontrastmittel hat die Konsistenz einer Paste, Gels einer dickflüssigen Masse oder einer viskosen Dispersion oder Emulsion oder eines zähen Breis.

Diese Zusammensetzung hat den Vorteil, dass sie nicht polymerisiert und während des Beschichtungsvorgangs ihre Konsistenz beibehält. Diese Paste oder (hoch)viskose Masse oder dickflüssige Aufschlämmung wird mittels einer Spritzvorrichtung, vorzugsweise einer Düse wie in Fig. 1 gezeigt unter Druck in die Falten eingebracht.

Die Düse kann sofern nötig, die Ballonfalten aufweiten und gezielt die durch die Falten gebildeten Hohlräume befüllen. Die Faltenballons weisen in der Regel 4 oder mehr Falten auf, welche einzeln befüllt werden.

Als besonders vorteilhaft hat sich erwiesen, nach Befüllung einer oder mehrerer oder aller Falten den Faltenballon in Richtung der Öffnungen der Falten zu rotieren. Diese Drehung bewirkt, dass die dickflüssige Paste vollständig und gleichmäßig in den Falten verteilt wird und eventuelle Lufteinschlüsse freigesetzt werden. Nach der Drehung des Faltenballons kann eine weitere Befüllung von bereits befüllten oder leeren Falten erfolgen.

Während und/oder nach der Rotation trocknet die Zusammensetzung in den Falten entweder bei Atmosphärendruck oder unter vermindertem Druck. Trocknung oder Aushärtung der Zusammensetzung erfolgt durch die Entfernung des mindestens einen Alkohols durch Verdunstung. Die getrocknete Zusammensetzung hat eine poröse Konsistenz und löst sich sehr leicht von der Ballonoberfläche bei der Dilatation ab. Der Alkohol als Lösungsmittel wurde bis auf die üblichen Rückstände entfernt und das Kontrastmittel bildet eine poröse Matrix für den Wirkstoff und ist zudem in der Lage, nach Dilatation des Faltenballons den Wirkstoff schnell und in großer Konzentration freizusetzen. Zudem hat das erfindungsgemäße Verfahren den Vorteil, sehr materialschonend zu arbeiten, da nur die Falten beschichtet oder befüllt werden und somit sich kein Wirkstoff auf der äußeren Ballonoberfläche befindet, der bei der Einführung des Katheters verloren geht.

**Allgemeine Beschreibung der Beschichtungsverfahren**

Pipettierverfahren - Kapillarverfahren

Dieses Verfahren umfasst die folgenden Schritte:

a) Bereitstellung eines zusammengefalteten, komprimierten Katheterballons,
b) Bereitstellung einer Beschichtungsvorrichtung mit einem zur punktförmigen Abgabe der Beschichtungslösung befähigten Auslaß,
c) Ansetzen des zur punktförmigen Abgabe der Beschichtungslösung befähigten Auslaß am proximalen oder am distalen Ende einer Falte des Katheterballons,
d) Abgabe einer definierten Menge der Beschichtungslösung über den Auslaß an das proximale oder distale Ende einer Falte, und
e) Befüllung der Falte aufgrund Kapillarwirkung mit der Beschichtungslösung.

Optional kann noch der Schritt f) für die Trocknung folgen:

f) Trocknung der Beschichtungslösung in der Falte, wobei während der Trocknung der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie innerhalb von 5 Minuten, vorzugsweise 2 Minuten aufgrund der Kapillarkräfte oder zusätzlich unter Ausnutzung der Schwerkraft in die Falte gezogen wird und die Falte weitgehend vollständig befüllt.

Spritzverfahren oder Spritzenverfahren:

Dieses Verfahren umfasst die folgenden Schritte:

a) Bereitstellung eines zusammengefalteten, komprimierten Katheterballons,
b) Bereitstellung einer Beschichtungsvorrichtung mit mindestens einer Düse oder mindestens einem spritzenförmigen Auslaß,
c) Ansetzen der Düse oder des Auslasses am proximalen oder am distalen Ende einer Falte des Katheterballons,
d) Bewegen der Düse oder des Auslasses relativ zur Falte entlang der Falte, und
e) Abgabe eines definierten Flusses an Beschichtungslösung pro Zeit und pro zurückgelegter Strecke.

Optional kann noch der Schritt f) für die Trocknung folgen:

f) Trocknung der Beschichtungslösung in der Falte oder gleichmäßige Verteilung der Beschichtung in der Falte, wobei der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie mittels kleiner Düsen oder kleiner Auslassöffnungen in die Falte gefüllt werden kann.

Sprühverfahren oder Faltensprühverfahren:

Dieses Verfahren umfasst die folgenden Schritte:

a) Bereitstellung eines zusammengefalteten, komprimierten Katheterballons,
b) Bereitstellung einer Beschichtungsvorrichtung mit einer Vielzahl in Reihe befindlicher Abgabeöffnungen,
c) Einführen der Vielzahl in Reihe befindlicher Abgabeöffnungen unter eine Falte des Katheterballons,
d) gleichzeitige Abgabe einer definierten Menge einer Beschichtungslösung aus den Abgabeöffnungen in die Falte, und
e) Trocknung der Beschichtungslösung in den Falten.

Optional kann noch der Schritt f) für die Trocknung folgen:

f) Trocknung der Beschichtungslösung in der Falte oder gleichmäßige Verteilung der Beschichtung in der Falte, wobei der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie mittels kleiner Düsen oder kleiner Auslassöffnungen in die Falte gefüllt werden kann.

Schteppverfahren oder Tropfenschleppverfahren:

Dieses Verfahren umfasst die folgenden Schritte:

a) Bereitstellung eines Katheterballons im gefalteten, teilweise inflatierten oder vollständig inflantierten Zustand,
b) Bereitstellung einer Beschichtungsvorrichtung mit einer Abgabevorrichtung,
c) Bildung eines Tropfens aus Beschichtungslösung an der Abgabevorrichtung,
d) Schleppen des Tropfens über die zu beschichtende Oberfläche des Katheterballons, ohne dass die Abgabevorrichtung selbst die Oberfläche des Katheterballons berührt, und
e) Nachdosieren der Beschichtungslösung, so dass der Tropfen im wesentlichen seine Größe beibehält.

Dieses elegante und besonders schonende Verfahren für den Katheterballon benutzt einen Tropfen der Beschichtungslösung, welcher, ohne dass die Abgabevorrichtung die Ballonoberfläche berührt, über die Ballonoberfläche bewegt oder gezogen wird, indem sich Abgabevorrichtung und damit Tropfen und Ballonoberfläche relativ zueinander bewegen.

Die Beschichtungslösung wird dabei derart nachdosiert, dass der Tropfen seine Größe im wesentlichen beibehält und die Verbindung zwischen Abgabevorrichtung und Ballonoberfläche aufrechterhält. Über eine Volumenmesseinrichtung lässt sich nach der Beschichtung die abgegebene Menge an Beschichtungslösung genau bestimmen und damit die auf dem Ballon befindliche Menge an Wirkstoff.

<u>Fadenschleppverfahren:</u>

Dieses Verfahren umfasst die folgenden Schritte:

a) Bereitstellung eines Katheterballons im gefalteten, teilweise inflatierten oder vollständig inflantierten Zustand,
b) Bereitstellung einer Beschichtungsvorrichtung mit einer Abgabevorrichtung in Form eines Fadens, Schwammes, Lederstreifens oder Textilstückes,
c) Bereitstellung einer Beschichtungslösung,
d) Tränkung der Abgabevorrichtung mit der Beschichtungslösung,
e) Übertragung der Beschichtungslösung von der Abgabevorrichtung auf die zu beschichtende Oberfläche des Katheterballons, und
f) Nachdosieren der Beschichtungslösung, so dass eine gleichmäßige Abgabe der Beschichtungslösung von der Abgabevorrichtung auf die zu beschichtende Oberfläche des Katheterballons erfolgt.

Dieses ebenfalls sehr elegante Verfahren ist auch sehr schonend für die Ballonoberfläche, da die Abgabevorrichtugn zwar die Ballonoberfläche berührt jedoch derart ausgestaltet ist, dass sie die Ballonoberfläche nicht beschädigen kann. Die Abgabevorrichtung wird über die Ballonoberfläche durch relative Bewegung von Ballon zur Abgabevorrichtung gezogen oder geschleppt und gibt dabei eine bestimmte Menge an Beschichtungslösung ab. Mittels einer Volumenmesseinrichtung kann nach der Beschichtung genau bestimmt werden, wieviel Beschichtungslösung auf die Ballonoberfläche übertragen worden ist, woraus sich die genaue Menge an Wirkstoff auf der Ballonoberfläche ergibt.

<u>Kugelschreiberverfahren oder Rollverfahren:</u>

Dieses Verfahren umfasst die folgenden Schritte:

a) Bereitstellung einer Beschichtungsvorrichtung mit einem Kugelkopf zur Übertragung der Beschichtungslösung auf die Oberfläche des zu beschichtenden Katheterballons,
b) Bereitstellung einer Beschichtungslösung mit Zugang zum Kugelkopf,
c) Aufsetzen den Kugelkopfes der Beschichtungsvorrichtung auf die zu beschichtende Oberfläche des Katheterballons,
d) Ausübung eines Druckes auf den Kugelkopf der Beschichtungsvorrichtung zwecks Ermöglichung des Ausflusses der Beschichtungslösung, und
e) Abfahren der zu beschichtenden Oberfläche des Katheterballons mit dem Kugelkopf unter Übertragung der Beschichtungslösung auf die zu beschichtende Oberfläche des Katheterballons.

Bei diesem ebenfalls recht eleganten Verfahren rollt die Abgabevorrichtung aufgrund relativer Bewegung von Katheterballon zur Abgabevorrichtung über die Ballonoberfläche und gibt dabei eine mittels Volumenmesseinrichtung bestimmbare Menge an Beschichtungslösung über einen Kugelkopf auf die Ballonoberfläche ab.

Im folgenden wird auf die erfindungsgemäßen Beschichtungs- und Befüllungsverfahren genauer eingegangen.

<u>Pipettierverfahren oder Kapillarverfahren:</u>

Bei diesem Verfahren wird eine Pipette oder Spritze oder eine andere zur punktförmigen Abgabe der wirkstoffenthaltenden Zusammensetzung befähigte Vorrichtung eingesetzt.

Unter dem Begriff "wirkstoffenthaltende Zusammensetzung" oder "Beschichtungslösung" wie hierin verwendet wird das Gemisch aus Wirkstoff und Lösungsmittel und/oder Hilfsstoff und/oder Träger verstanden, also eine tatsächliche

Lösung, Dispersion, Suspension oder Emulsion aus einem Wirkstoff oder einem Wirkstoffgemisch und mindestens einem weiteren Bestandteil ausgewählt aus den hierin genannten Lösungsmitteln, Ölen, Fettsäuren, Fettsäureester, Aminosäuren, Vitaminen, Kontrastmitteln, Salzen und/oder membranbildenden Substanzen. Der Begriff "Lösung" soll ferner verdeutlichen, dass es sich um ein flüssiges Gemisch handelt, welches jedoch auch gelartig, zähflüssig bzw. pastös (dickviskos oder hochviskos) sein kann.

Die Pipette oder Spritze oder Auslass oder die andere zur punktförmigen Abgabe der wirkstoffenthaltenden Zusammensetzung befähigte Vorrichtung wird mit der Zusammensetzung befüllt und deren Auslass vorzugsweise am proximalen oder am distalen Ende einer Falte angesetzt. Die austretende Zusammensetzung wird aufgrund von Kapillarkräften in die Falte und entlang der Falte gezogen bis das gegenüberliegende Ende der Falte erreicht ist.

Der Katheterballon befindet sich im komprimierten, d.h. deflatierten Zustand. Eine auch nur teilweise oder geringfügige Inflation des Katheterballons ist in der Regel nicht erforderlich um die Falten ein wenig zu öffnen. Dennoch kann die Befüllung der Falten bei einer geringfügigen Inflation des Katheterballons von maximal 10% des bei der Dilatation vorgesehenen Durchmessers erfolgen. Die Befüllung der Falten kann zudem bei einer leichten Aufweitung der Falten erfolgen indem 100 kPa (1 bar) Überdruck, vorzugsweise 50 kPa (0,5 bar) Überdruck angelegt werden, um die Falten leicht aufzuweiten.

Bei diesem Verfahren ist es wichtig, dass die wirkstoffenthaltende Zusammensetzung dünnflüssig genug ist, um die entsprechenden Kapillarkräfte zu entwickeln.

Als Zusammensetzungen werden insbesondere Lösungen von einem Wirkstoff oder Wirkstoffgemisch in einem Alkohol oder Alkoholgemisch bevorzugt.

Die Kapillarkräfte sollten derart stark sein, dass sich eine Falte einer Länge von 10 mm innerhalb von 5 bis 80 Sekunden, vorzugsweise innerhalb von 15 bis 60 Sekunden und insbesondere bevorzugt innerhalb von 25 bis 45 Sekunden vollständig füllt.

Ist die Zusammensetzung bzw. Lösung zu viskos, kann es zudem von Vorteil sein, den Katheterballon mit der zu befüllenden Falte nach oben aus der horizontalen Lage um maximal 45°, vorzugsweise maximal 30° zu neigen und dadurch auch die Schwerkraft mit zu nutzen. In der Regel erfolgt die Befüllung einer Falte mittels Kapillarkräften jedoch im horizontalen Zustand des Katheterballons mit der zu befüllenden Falte nach oben. Die Spritze oder Pipette oder die andere zur punktförmigen Abgabe der wirkstoffenthaltenden Zusammensetzung befähigte Vorrichtung wird vorzugsweise am proximalen oder am distalen Ende der Falte spitz in Richtung des Faltenverlaufs in einem Winkel von 10° bis 65°, vorzugsweise 20° bis 55°, weiter bevorzugt in einem Winkel von 27° bis 50° und insbesondere bevorzugt in einem Winkel von 35° bis 45° gemessen von der Horizontalen an die Falte angesetzt. Die Befüllung der Falte erfolgt dann von dem höherliegenden Ende einer Falte her, so dass die Beschichtungslösung ein Gefälle vorfindet und zusätzlich zur Kapillarwirkung auch die Schwerkraft ausgenutzt wird.

Grundsätzlich besteht natürlich auch die Möglichkeit, die Spritze oder Pipette oder die andere zur punktförmigen Abgabe der wirkstoffenthaltenden Zusammensetzung befähigte Vorrichtung in der Mitte der Falten oder an einem beliebigen anderen zwischen dem distalen und proximalen Ende liegenden Punkt anzusetzen, so dass sich die Falte aufgrund von Kapillarkräften gleichzeitig in Richtung des proximalen und des distalen Endes befüllt, jedoch haben sich die Ansatzpunkte am Ende der Falte als bevorzugt erwiesen.

Hat die Zusammensetzung zum Befüllen der Falten bzw. der vorliegenden Falte das gegenüberliegende Ende erreicht, so stoppt der Substanzfluß in der Regel von selbst und die Spritze oder Pipette oder die andere zur punktförmigen Abgabe der wirkstoffenthaltenden Zusammensetzung befähigte Vorrichtung kann entfernt werden.

Damit nun kein größerer Tropfen an wirkstoffenthaltender Zusammensetzung am Ansatzpunkt der Spritze oder Pipette oder der anderen zur punktförmigen Abgabe der wirkstoffenthaltenden Zusammensetzung befähigten Vorrichtung zurückbleibt, hat sich als vorteilhaft erwiesen, die Spritze oder Pipette oder die andere Abgabevorrichtung bereits zu entfernen, bevor die wirkstoffenthaltende Zusammensetzung vollständig bis an das andere Ende der Falte gelangt ist. Dadurch wird auch noch die restliche am Ansatzpunkt der Spritze oder der Pipette oder der anderen Abgabevorrichtung verbliebene wirkstoffenthaltende Zusammensetzung in die Falte hineingezogen, so dass keine Beschichtungszusammensetzung oder besser Befüllungszusammensetzung außerhalb der Falte verbleibt.

Vorzugsweise wird die Spritze oder Pipette oder die andere Abgabevorrichtung dann entfernt, wenn ca. 90% der Falte mit wirkstoffenthaltender Zusammensetzung befüllt sind. Der optimale Zeitpunkt zur Entfernung der Spritze oder Pipette oder der anderen Abgabevorrichtung kann durch wenige Versuche genau bestimmt werden und ist auch reproduzierbar.

Unter dem Begriff "eine andere zur punktförmigen Abgabe der wirkstoffenthaltenden Zusammensetzung befähigte Vorrichtung" bezeichnet eine Vorrichtung, welche ähnlich einer Pipette befähig ist, einen gleichmäßigen und kontinuierlichen Fluß an wirkstoffenthaltender Zusammensetzung zu liefern, so dass darunter auch eine Pumpe, Mikropumpe oder ein anderes Reservoir verstanden werden kann, welches diese gleichmäßige und kontinuierliche Abgabe an wirkstoffenthaltender Zusammensetzung gewährleistet.

Nach dem Befüllen einer Falte wird der Katheterballon gedreht, so dass die nächste zu beschichtende Falte nach oben und vorzugsweise horizontal liegt. Der Faltenbefüllungsvorgang wird nun wiederholt.

Je nach Konsistenz der wirkstoffenthaltenden Zusammensetzung kann es notwendig sein, die vorher befüllte Falte zu trocknen bevor der Ballon gedreht wird, um die nächste Falte zu befüllen. Die Trocknung erfolgt vorzugsweise durch Verdunstung des Lösungsmittels.

Ferner ist es bei diesem Verfahren auch möglich zwei, mehr als zwei oder alle Falten eines Katheterballons gleichzeitig zu befüllen oder zu beschichten, falls die Konsistenz der wirkstoffenthaltenden Zusammensetzung dies zulässt, d.h. die Konsistenz nicht derart dünnflüssig ist, dass die Zusammensetzung aus den nicht horizontal liegenden Falten ausläuft.

Insbesondere das Pipettierverfahren eignet sich zur gleichzeitigen Befüllung mehrerer oder aller Falten eines Katheterballons. Dabei kann der Katheterballon waagerecht oder vorzugsweise senkrecht gelagert werden und die Abgabevorrichtungen werden von oben an die Enden der Falten vorzugsweise im Winkel von 10 bis 70 Grad angesetzt, so dass die wirkstoffenthaltende Zusammensetzung in die Falten fließen kann.

Wurden alle Falten des Ballons befüllt, so erfolgt die Endtrocknung. Grundsätzlich ist es natürlich nicht erforderlich, dass alle Falten des Katheterfaltenballons befüllt werden, wobei jedoch die Befüllung aller Falten die gängige und bevorzugte Ausführungsform ist, da bei der Dilatation in möglichst kurzer Zeit eine größtmögliche Menge an Wirkstoff auf die Gefäßwand übertragen werden soll.

Bei den erfindungsgemäßen Faltenballons dauert die Dilatation vorzugsweise maximal 60 Sekunden und insbesondere bevorzugt maximal 30 Sekunden.

Nach der Befüllung der letzten Falte erfolgt die Trocknung der letzten Falten, d.h. des Inhalts der letzten Falte vorzugsweise ohne Vakuum unter Normaldruck durch Verdunstung des Lösungsmittels.

An diese Vortrocknung kann sich eine Endtrocknung anschließen, welche erfindungsgemäß bei rotierendem Katheterballon durchgeführt wird. Falls erforderlich oder gewünscht kann zudem noch Vakuum während der Rotation angelegt werden. Dieses besondere Trocknungsverfahren wird im Anschluß an die erfindungsgemäßen Beschichtungsverfahren eingehender beschrieben.

<u>Spritzverfahren oder Spritzenverfahren:</u>

Bei diesem erfindungsgemäßen Verfahren wird eine feine Spritze, spritzenförmige Öffnung, spritzenförmiger Auslaß oder Nadel oder Düse am proximalen oder distalen Ende einer Falte angesetzt und diese Abgabevorrichtung in Form einer Spritze, Nadel oder Düse entlang der Längsrichtung der Falte relativ zur Falte bewegt und pro zurückgelegter Teilstrecke eine bestimmte Menge einer wirkstoffenthaltenden Zusammensetzung oder ein definierter Fluss an Beschichtungslösung abgegeben.

Dabei ist es unerheblich, ob der Katheterballon fixiert ist und sich die Abgabevorrichtung entlang der Falte bewegt oder die Abgabevorrichtung fixiert ist und sich relativ dazu der Katheterballon bewegt oder sich sogar Katheterballon und Abgabevorrichtung beide relativ zueinander bewegen. Sollten sich Katheterballon und Abgabevorrichtung relativ zueinander bewegen, so ist eine Bewegung auf einer Geraden in entgegengesetzter Richtung bevorzugt.

Von der Abgabevorrichtung, d.h. der Spritze, Nadel oder Düse oder ähnlichem wird vorzugsweise eine mittel- bis dickviskose wirkstoffenthaltende Zusammensetzung vorzugsweise in Form einer Paste oder eines Gels oder eines Öls in das Falteninnere abgegeben. Die Viskositäten bevorzugter Lösungen liegen zwischen $10^1$ bis $10^6$ mPa·s, vorzugsweise zwischen $10^2$ bis $10^5$ mPa·s und insbesondere bevorzugt zwischen $10^3$ bis $10^4$ mPa·s.

Somit eignen sich als wirkstoffenthaltende Zusammensetzungen insbesondere solche mit den oben bezeichneten Ölen, Alkoholen (insbesondere Diolen und Polyolen), Fettsäuren, Fettsäureestern, Aminosäuren, Polyaminosäuren, membranbildenden Substanzen, liposomalen Formulierungen und/oder Salzen.

Bei dem Beschichtungsvorgang reicht die Spitze der Spritze, Düse oder Nadel bis ca. in die Mitte des Falteninneren, also bis ca. in die Mitte der Falte, d.h. die Düse oder der Auslaß befindet sich relativ zentral im durch die Falte gebildeten Hohlraum. Dort wird ein kontinuierlicher Fluß der wirkstoffenthaltenden Zusammensetzung derart abgegeben, dass Abgabegeschwindigkeit und Abgabemenge in Bezug auf die relative Bewegungsgeschwindigkeit von Abgabevorrichtung und Katheterballon geeignet sind, die Falte bzw. das Falteninnere zu mindestens 50 Vol.-%, bevorzugt zu mindestens 70 Vol.-% und insbesondere bevorzugt zu mindestens 85 Vol.-% mit der wirkstoffenthaltenden Zusammensetzung zu füllen.

Die Befüllung einer Falte dauert ca. von 5 bis 80 Sekunden, vorzugsweise ca. 15 bis 60 Sekunden und insbesondere bevorzugt ca. 25 bis 45 Sekunden bei einer Faltenlänge von 10 mm.

Der Katheterballon befindet sich beim Befüllungsvorgang der Falten im komprimierten, d.h. deflatierten Zustand. Eine auch nur teilweise oder geringfügige Inflation des Katheterballons ist in der Regel nicht erforderlich um die Falten ein wenig zu öffnen. Dennoch kann die Befüllung der Falten bei einer geringfügigen Inflation des Katheterballons von maximal 10% des bei der Dilatation vorgesehenen Durchmessers erfolgen. Die Befüllung der Falten kann zudem bei einer leichten Aufweitung der Falten erfolgen indem 100 kPa (1 bar) Überdruck, vorzugsweise 50 kPa (0,5 bar) Überdruck angelegt werden, um die Falten leicht aufzuweiten.

Dieses Beschichtungsverfahren kann natürlich auch mit dünnflüssigen wirkstoffenthaltenden Zusammensetzungen durchgeführt werden ist aber eher für ölige Zusammensetzungen geeignet als auch für hochkonzentrierte Salzlösungen.

Ferner bietet dieses Verfahren noch den Vorteil, dass mehr als eine Falte und insbesondere alle falten gleichzeitig beschichtet oder befüllt werden können. Dabei wird eine ringförmige Anordnung von Abgabevorrichtungen entsprechend der Anzahl an Falten derart angeordnet, dass pro Falte ein Abgabevorrichtung bereitsteht. Durch eine geringfügige Drehung werden die Spitzen der Abgabevorrichtungen in die Falten eingeführt und ca. mittig im Falteninneren platziert. Durch eine relative und gleichzeitige Bewegung der Abgabevorrichtungen relativ zur Längsrichtung der Falten, können alle Falten gleichzeitig mit einem kontinuierlichen und gleichmäßigen Fluß an wirkstoffenthaltender Zusammensetzung befüllt werden.

Beim Beschichten oder Befüllen einer oder aller Falten kann sich der Katheterballon in senkrechter oder horizontaler Lage oder einer schrägen Lage befinden.

Wurden in der wirkstoffenthaltenden Zusammensetzung flüchtige Lösungsmittel verwendet, so kann eine Trocknung des Falteninhalts oder eine Entfernung der flüchtigen Lösungsmittel mit Siedepunkten von unter 150°C erforderlich sein. Bei flüchtigen Lösungsmitteln erfolgt dies bevorzugt zuerst aufgrund Verdunstung des oder der flüchtigen Lösungsmittel.

Danach kann eine Endtrocknung erfolgen, wobei der Katheterballon in Richtung der Faltenöffnungen gesehen vom Falterinnenraum gedreht wird. Auf dieses Verfahren wird weiter unter ausführlich eingegangen. Wurden Beschichtungslösungen verwendet, welche nach Entfernung des eventuell vorhandenen Lösungsmittels ölig oder pastös bleiben, so kann die Rotationstrocknung zum einen der Entfernung von Resten an Lösungsmitteln mit Siedepunkten vorzugsweise unter 150°C dienen und zum anderen der gleichmäßigen Verteilung der öligen oder pastösen Schicht in den Falten.

Die Drehung oder Rotation des Katheterballons in Richtung der Faltenöffnungen kann zudem auch dazu dienen, die in den Falten bzw. unter den Falten befindlichen Zusammensetzungen gleichmäßig in der jeweiligen Falte zu verteilen.

Diese Rotation des Faltenballons kann insbesondere bei der Verwendung von öligen oder pastenförmigen wirkstoffenthaltenden Zusammensetzungen vorteilhaft sein, um eine gleichmäßige Verteilung der wirkstoffenthaltenden Zusammensetzung in den Falten als auch auf der Innenoberfläche der Falten zu gewährleisten.

Wie hierin verwendet bezieht sich der Begriff "Beschichtung" daher auch vorrangig auf die Beschichtung der Falteninnenoberflächen, wobei der gesamte Falteninnenraum in der Regel nicht mit wirkstoffenthaltender Zusammensetzung bzw. nach der Trocknung mit der verbliebenen Zusammensetzung ausgefüllt ist.

Hingegen bezeichnet der Begriff "Befüllung" eher die vollständige Ausfüllung des Falteninnenraumes mit wirkstoffenthaltender Zusammensetzung.

Werden Lösungsmittel verwendet, welche durch Trocknung entfernt werden, so ist eine Befüllung in der Regel nicht zu erreichen und es wird eher von Beschichtung der inneren Oberflächen der Falten gesprochen.

Werden hingegen als Trägerstoffe oder Hilfsstoffe Substanzen mit hohen Siedepunkten wie beispielsweise Öle verwendet, so ist auch eine mehr oder weniger vollständige Befüllung der Falten möglich, sofern keine nennenswerten Mengen von flüchtigen Substanzen in der wirkstoffenthaltenden Zusammensetzung anwesend sind.

Dieses Spritzverfahren oder Spritzenverfahren eignet sich insbesondere zur Einbringung von wirkstoffenthaltenden Zusammensetzungen in die Falten von Katheterfaltenballons, welche mittels herkömmlicher Tauch- oder Sprühverfahren nicht auf einen Katheterballon aufgetragen geschweige denn in die Falten eingebracht werden können.

Im Gegensatz zu den herkömmlich verwendeten festen Beschichtungen auf Stents oder auf Katheterballons bieten die öligen und pastenförmigen Beschichtungen und Befüllungen den Vorteil, dass diese wirkstoffenthaltenden Zusammensetzungen eben nicht vollständig trocknen, sondern ihre Konsistenz weitgehend beibehalten. Daher werden bevorzugt Beschichtungslösungen verwendet, welche an der Luft oder unter Schutzgasatmosphäre bei Normaldruck nicht vollständig hart werden, d.h. nach weitgehender Entfernung eines eventuell verwendeten Lösungsmittels der Beschichtungslösung verbleibt in den Falten des Katheterballons eine ölige oder pastenförmige Beschichtung nachdem das Lösungsmittel durch Verdunstung oder unter vermindertem Druck entfernt worden ist. Daher sind Beschichtungslösungen bevorzugt, welche nach Entfernung des optional verwendeten Lösungsmittels oder Lösungsmittelgemisches einen Schmelzpunkt oder Erstarrungspunkt kleiner 20°C bevorzugt kleiner 30°C besitzten und zudem eine dickviskose, ölige oder pastenförmige Konsistenz haben, so dass auch bei Lagerung des beschichteten Katheterballons über mehrere Monate bis zu einem Jahr die Beschichtung nicht aus den Falten herausläuft.

Die Verwendung eines zu entfernenden Lösungsmittels ist jedoch nicht zwingend, so dass auch ein physiologisch verträgliches Lösungsmittel oder eine physiologisch verträgliche Komponente der Beschichtungslösung verwendet werden kann, wie beispielsweise Polyethylenglykol, Glycerin, Propylenglykol oder ähnliches, welche nicht entfernt wird und in der Beschichtung verbleibt und die Beschichtung in den Falten während der Dauer der Haltbarkeit der beschichteten Medizinprodukte ölig oder pastenförmig hält.

Die enormen Vorteile derartiger öliger oder pastenförmiger Beschichtungen sind evident. Wird am Ort der stenosierten Stelle der Katheterballon inflatiert bzw. dilatiert, so wird diese ölige oder pastenförmige Zusammensetzung zumindest teilweise in der Regel aber weitgehend auf die Gefäßwand übertragen und dient als Wirkstoffreservoir für eine verzögerte Wirkstoffabgabe von mehreren Stunden bis Tagen an das umliegende Gewebe und hat zudem die

positive Eigenschaft Plaque aufzulösen bzw. der Ablagerung von Plaque entgegenzuwirken und wird selber danach biologisch abgebaut, ohne physiologisch bedenkliche Abbauprodukte freizusetzen. Dieses System löst in perfekter Weise das Problem, zum einen eine Beschichtung derart sicher an einen Katheterballon anzubringen, dass sie während des Einführens nicht durch den Blutstrom abgewaschen oder durch Berührungen der Gefäßwand auf diese übertragen wird und zum anderen dass am Wirkort während der Dilatation in relativ kurzer Zeit, d.h. innerhalb von 30 bis 300 Sekunden genügend Wirkstoff auf die Gefäßwand übertragen wird, d.h. möglichst wenig Beschichtung auf dem Katheterballon verbleibt und möglichst viel, d.h. mindests 50% der Beschichtung auf die Gefäßwand übertragen wird, um der Restenose-Entstehung effektiv entgegenzuwirken.

Solche erfindungsgemäßen Systeme lassen sich nicht nur durch das Spritzverfahren herstellen, sondern auch durch die anderen hierin beschriebenen Beschichtungsverfahren.

<u>Sprühverfahren oder Faltensprühverfahren:</u>

Bei diesem erfindungsgemäßen Verfahren wird eine Vielzahl in Reihe befindlicher Abgabeöffnungen unter die Falte des Faltenballons geschoben oder gebracht und gleichzeitig wird aus der Vielzahl von Abgabeöffnungen eine wirkstoffenthaltende Zusammensetzung in die jeweilige Falte abgegeben.

Die Abgabevorrichtung besteht vorzugsweise aus 2 bis 10 Düsen oder Abgabeöffnungen, welche in vorzugsweise gleichmäßigen Abständen entlang der Längsrichtung der Falten angeordnet sind.

Diese Abgabevorrichtung wird dann unter die Falte des Katheterballons eingeführt und die jeweilige Falte wird durch gleichzeitige Abgabe der wirkstoffenthaltenden Zusammensetzung aus den Düsen oder anderen Abgabeöffnungen befüllt oder beschichtet.

Ähnlich wie beim vorgenannten Spritzenverfahren dauert die Befüllung einer Falte ca. 5 bis 80 Sekunden, vorzugsweise ca. 15 bis 60 Sekunden und insbesondere bevorzugt ca. 25 bis 45 Sekunden bei einer Faltenlänge von 10 mm und der Verwendung von 4 Abgabeöffnungen. Die Abgabeöffnungen befinden sich dabei vorzugsweise auch im wesentlichen in der Mitte des Hohlraumes unter den Falten.

Bei dieser Beschichtungs- oder Befüllungsvariante ist es nicht erforderlich, dass die Abgabevorrichtung in der Falte des Katheterballons relativ zur Faltenlängsrichtung bewegt wird. In der Regel sind Katheterballon und Abgabevorrichtung während der Befüllung oder Beschichtung fixiert, wobei jedoch eine Bewegung entlang der Längsrichtung der Falte möglich ist. Ist eine relative Bewegung vorgesehen, so ist die Distanz für die Bewegung vorzugsweise nicht größer als der Abstand zwischen zwei Düsen oder Abgabeöffnungen der Abgabevorrichtung.

Die Abgabevorrichtung umfasst oder besteht aus mindestens 2 und maximal 10 Abgabeöffnungen oder Düsen oder ähnlichem und vorzugsweise aus 3 bis 6 und insbesondere bevorzugt aus 4 oder 5 Abgabeöffnungen oder Düsen oder ähnlichem, welche über eine Distanz von 10 mm vorzugsweise gleichmäßig verteilt sind.

Die Abgabevorrichtung weist 2 bis 10 Düsen oder ähnliche Öffnungen auf, welche befähigt sind, die wirkstoffenthaltende Zusammensetzung gleichmäßig abzugeben oder gleichmäßig in die Falte zu sprühen.

Für dieses Befüllungs- oder Beschichtungsverfahren werden vorzugsweise mittel- bis dünnviskose Zusammensetzungen oder Lösungen eines Wirkstoffs oder einer Wirkstoffkombination eingesetzt, welche insbesondere ein alkoholisches Lösungsmittel enthalten. Ferner sind Beschichtungslösungen bevorzugt, welche nicht vollständig hart werden, sondern eine gelartige, viskose, ölige oder pastenförmige Konsistenz beibehalten. Es gelten auch hier die oben für das Spritzenverfahren gemachten Aussagen insbesondere zur Beschichtungslösung und Trocknung.

Bei diesem Faltensprühverfahren befindet sich der Katheterballon im komprimierten, d.h. deflatierten Zustand. Eine auch nur teilweise oder geringfügige Inflation des Katheterballons ist in der Regel nicht erforderlich um die Falten ein wenig zu öffnen. Dennoch kann die Befüllung der Falten bei einer geringfügigen Inflation des Katheterballons von maximal 10% des bei der Dilatation vorgesehenen Durchmessers erfolgen. Die Befüllung der Falten kann zudem bei einer leichten Aufweitung der Falten erfolgen indem 100 kPa (1 bar) Überdruck, vorzugsweise 50 kPa (0,5 bar) Überdruck angelegt werden, um die Falten leicht aufzuweiten.

Nach dem Befüllen einer Falte wird der Katheterballon gedreht, so dass die nächste zu beschichtende Falte vorzugsweise nach oben und vorzugsweise horizontal liegt. Der Faltenbefüllungs- oder Faltenbeschichtungsvorgang wird nun wiederholt.

Je nach Konsistenz der wirkstoffenthaltenden Zusammensetzung kann es notwendig sein, die vorher befüllte Falte zu trocknen bevor der Ballon gedreht wird, um die nächste Falte zu befüllen. Die Trocknung erfolgt vorzugsweise durch Verdunstung des Lösungsmittels.

Ferner ist es bei diesem Verfahren auch möglich zwei, mehr als zwei oder alle Falten eines Katheterballons gleichzeitig zu befüllen oder zu beschichten, falls die Konsistenz der wirkstoffenthaltenden Zusammensetzung dies zulässt, d.h. die Konsistenz nicht derart dünnflüssig ist, dass die Zusammensetzung aus den nicht horizontal liegenden Falten ausläuft. Zur Befüllung oder Beschichtung mehrerer oder aller Falten wird eine entsprechende kreisförmige Anordnung von Abgabevorrichtungen entsprechende der Anzahl an falten bereitgestellt, um den vorzugsweise senkrecht angeordneten Katheterballon platziert und durch Drehung werden die Abgabeöffnungen unter die Falten geführt, wo

dann gleichzeitige die Abgabe der wirkstoffenthaltenden Zusammensetzung stattfindet.

Wurden alle Falten des Ballons befüllt, so erfolgt die Endtrocknung. Grundsätzlich ist es natürlich nicht erforderlich, dass alle Falten des Katheterfaltenballons befüllt werden, wobei jedoch die Befüllung aller Falten die gängige und bevorzugte Ausführungsform ist, da bei der Dilatation in möglichst kurzer Zeit eine größtmögliche Menge an Wirkstoff auf die Gefäßwand übertragen werden soll.

Nach der Befüllung der letzten Falte erfolgt die Trocknung der letzten Falten, d.h. des Inhalts der letzten Falte vorzugsweise ohne Vakuum unter Normaldruck durch Verdunstung des Lösungsmittels.

An diese Vortrocknung kann sich eine Endtrocknung anschließen, welche erfindungsgemäß bei rotierendem Katheterballon durchgeführt wird. Falls erforderlich oder gewünscht kann zudem noch Vakuum während der Rotation angelegt werden. Dieses besondere Trocknungsverfahren wird im Anschluß an die erfindungsgemäßen Beschichtungsverfahren eingehender beschrieben.

<u>Schleppverfahren oder Tropfenschleppverfahren:</u>

Ein besonders bevorzugtes Verfahren für die ganzfläche Beschichtung als auch für die gezielte Beschichtung bzw. Befüllung der Falten ist das sogenannte Schleppverfahren oder Tropfenschleppverfahren.

Dieses Verfahren ermöglicht einen Katheterballon im gefalteten Zustand mit einer flüssigen wirkstoffenthaltenden Zusammensetzung über seinen gesamten Umfang innerhalb und außerhalb der Faltung zu beschichten.

Bei diesem Verfahren wird eine Abgabevorrichtung in Form einer Spritze, Nadel, Pipette oder Düse an den, vorzugsweise waagerecht aufgehängten, feststehenden oder vorzugsweise rotierenden Ballon angenähert und dann ein Volumen der wirkstoffenthaltenden Zusammensetzung dosiert, dass sich ein Tropfen an der Spitze der Abgabevorrichtung ausbildet, der sowohl Kontakt zur Dosiervorrichtung als auch zum Ballon hat.

Zur besseren Verfahrensführung kann vorzugsweise die Dosiervorrichtung mit einem dünnen Draht, Faden oder schwammähnlichem Hilfsmittel am auslassenden Ende verlängert sein, dass bei Annäherung der Flüssigkeitskontakt zwischen Dosiervorrichtung und Ballon über dieses Hilfsmittel hergestellt und beibehalten wird.

Wahlweise besteht auch die Möglichkeit, eine Dosiernadel mit seitlicher Öffnung oder eine gabelförmige Verlängerung zu verwenden.

Durch Seitwärtsbewegung der Dosiervorrichtung entlang der Längsrichtung des Ballons relativ zum rotierenden Ballon wird der Tropfen mitgeschleppt und. pro zurückgelegter Teilstrecke trocknet eine bestimmte Menge der wirkstoffenthaltenden Zusammensetzung als dünner Film auf der überstrichenen Fläche. Dabei wird durch Nachdosieren der wirkstoffenthaltenden Zusammensetzung die Tropfengröße beibehalten, bis die Zieldosierung erreicht ist.

Die Bewegung wird so lange aufrechterhalten, bis die gesamte Zielfläche beschichtet und keine Flüssigkeit mehr auf der Ballonoberfläche vorhanden ist.

Um bei der anfänglichen Dosierung, die dem Aufbau des Tropfens zwischen Ballonoberfläche und Dosiervorrichtung dient, dem Kapillareffekt der Faltung entgegenzuwirken, kann der Ballon mit geeignetem Lösemittel vorher benetzt werden, da so die Falten bereits mit Flüssigkeit gefüllt sind und der Kapülareffekt den Tropfen nicht absaugt.

Da die meisten Spitzen von Abgabevorrichtungen aus einem härteren oder harten Material sind bzw. aus einem Material sind, welches geeignet ist, das Ballonmaterial zu beschädigen, was bei der Dilatation zu gefährlichen Komplikationen führen kann, besteht eine insbesondere bevorzugte Ausführungsform darin, an der Spitze der Abgabevorrichtung oder durch die Abgabevorrichtung oder zumindest durch die terminale Öffnung der Abgabevorrichtung einen Faden oder Draht zu führen oder zu befestigen, welcher dann für den Kontakt zur Ballonoberfläche dient, ohne dass die Spitze der Abgabevorrichtung den Ballon berührt. Dieser Faden oder Draht ist aus einem Material, welches das Ballonmaterial nicht beschädigen kann.

Anstelle eines Fadens oder Drahtes kann auch ein Schwamm oder schwammartiges Gebinde, ein Stück Textil oder ein entsprechend dünn dimensioniertes Stück Leder, oder ein Bündel Haare oder Borsten eingesetzt werden. Voraussetzung ist jedoch, dass diese Mittel aus Materialien bestehen, welche den Katheterballon nicht beschädigen, d.h. weder scharf noch eckig sind oder ätzende, basische, saure oder klebrige Substanzen oder Chemikalien abgeben, welche das Polymer des Katheterballons auflösen, anlösen, zersetzen, versteifen, zerkratzen oder schneiden können.

Somit sind als Materialien für diese Mittel insbesondere Stoffe und Polymere bevorzugt, aus denen auch Textilien, Fäden, Garne, Borsten für Pinsel hergestellt werden können.

Erfindungsgemäß wird dadurch erreicht, dass die Spitze der Abgabevorrichtung in einem gewissen Abstand zur Ballonoberfläche gehalten werden kann und dennoch über die Kontaktvorrichtung in Form von Faden, Draht, Schwamm, Lederstreifen, Borste oder Textilstück der Tropfen und die Bewegung des Tropfens relativ zur Ballonoberfläche kontrolliert und gesteuert werden kann.

Grundsätzlich ist es unerheblich, ob die Abgabevorrichtung bei ortsfestem Ballon bewegt wird oder der Ballon bei ortsfester Abgabevorrichtung sich bewegt. Eine bevorzugte Ausführungsform besteht aus einem sich in horizontaler Lage befindenden und sich drehenden Ballon mit einer von oben angeordneten und sich entlang der Längsachse des Ballons bewegenden Abgabevorrichtung. Bei dieser Ausführungsform erfolgt eine spiralförmige Beschichtung der gesamten Oberfläche des Katheterballons.

Bei einer anderen bevorzugten Ausführungsform erfolgt die Bewegung des Katheterballons in horizontaler Lage intervallförmig. Bei feststehendem Ballon bewegt sich die Abgabevorrichtung entlang der Längsrichtung des Katheterballons in einer annähernd geraden Linie von einem Ende zum anderen und wieder zurück, wobei der Ballon um einige Grad gedreht wird, wenn die Abgabevorrichtung das distale oder proximale Ende des Katheterballons erreicht. Durch diese Ausführungsform ergibt sich eine linienförmige Beschichtung der gesamten Ballonoberfläche.

Setzt man hingegen die Abgabevorrichtung an einer Falte an und bewegt diese entlang der Falte und wiederholt diesen Vorgang nach Drehung des Ballons mit den weiteren Falten, so erhält man einen gezielt faltenbefüllten Katheterballon.

Fadenschleppverfahren:

Bei diesem Verfahren wird kein Tropfen über die Oberfläche des Katheterballons bewegt sondern ein mit der Abgabevorrichtung verbundener Faden bzw. als Abgabevorrichtung dienender Faden wird über die Ballonoberfläche gezogen oder auf die Ballonoberfläche aufgesetzt oder getüpfelt oder kann auch in Ruhestellung zur Abgabe einer Wirkstoff-enthaltenden Lösung dienen.

Bei diesem Verfahren fließt die Wirkstoff-enthaltende Lösung entlang des Fadens, wobei vorzugsweise keine Tropfenbildung stattfindet. Der Faden ist ständig mit der Wirkstoff-enthaltenden Lösung benetzt und gibt diese Lösung an die Ballonoberfläche ab, sobald der Faden diese berührt.

Auch dieses Verfahren hat den großen Vorteil, dass die zumeist aus hartem Material bestehende Spitze der Abgabevorrichtung ähnlich wie beim Tropfenschleppverfahren das Ballonmaterial nicht berührt und daher auch keine Beschädigung des Katheterballons erfolgt.

Vorzugsweise wird der Faden bei sich drehendem Katheterballon horizontal entlang der Längsrichtung der Ballonachse über die Ballonoberfläche gezogen, wobei er eine rasch trocknende Spur an Wirkstoff-enthaltender Lösung abgibt.

Dieses Verfahren ist jedoch nicht auf eine Ausführungsform mit einem Faden beschränkt, sondern es können auch gleichzeitig mehrere Fäden über die Ballonoberfläche bewegt werden, wobei in diesem Fall der Ballon vorzugsweise senkrecht angeordnet ist. Die Fäden können zudem auch miteinander verbunden sein oder ein Netz ausbilden. Dabei sind die Fäden mit mindestens einer Abgabevorrichtung verbunden, welche die Fäden oder das Netz kontinuierlich mit einer Wirkstoff-enthaltenden Lösung versorgt.

Dieses Verfahren eignet sich somit zur vollständigen und teilweisen Beschichtung der Ballonoberfläche. Sollen hingegen nur die Falten befüllt oder beschichtet werden, so besteht die Möglichkeit, einen Faden in die Falte zumindest teilweise einzuführen oder beim Falten des Ballons in die Falte einzulegen und die Wirkstoff-enthaltende Lösung über diesen Faden in die Falte einfließen zu lassen, wobei nach Befüllung der Falte der Faden vorzugsweise wieder entfernt wird.

Bei der gezielten Befüllung der Falten eignet sich ferner insbesondere eine Kombination aus Pipettierverfahren und Fadenschleppverfahren, wobei von der Abgabevorrichtung über den Faden eine derart große Menge an Wirkstoff-enthaltender Lösung am proximalen oder distalen Ende in die unbefüllte Falte eines inflatierten Katheterballons abgegeben wird, dass der Kapillareffekt die Lösung in die Falte hineinzieht.

Das Tropfenschleppverfahren als auch das Fadenschleppverfahren lösen beide in eleganter Weise das Problem gezielt die Ballonoberfläche als auch gezielt die Falten des Ballons mit einer definierten Menge an Wirkstoff zu beschichten bzw. zu befüllen, ohne das Ballonmaterial zu beschädigen. Die Abgabevorrichtung kann dabei über eine Messeinrichtung verfügen, welche die abgegebene Menge an Wirkstoff-enthaltender Lösung aufzeichnet oder angibt.

Ferner eignen sich diese Verfahren insbesondere zur Beschichtung und/oder zum Befüllen der Falten eines Ballons im deflatierten (gefaltenen) Zustand, was besonders anspruchsvoll ist, da die Ballonoberfläche eines zusammengefalteten Ballons nicht gleichmäßig ausgebildet ist und die üblichen Beschichtungstechniken für gleichmäßige Körper nur mit entsprechenden Problemen angewendet werden können. Hingegen werden Abstandsunterschiede zwischen Ballonoberfläche und Abgabevorrichtung beim Tropfenschlepp- und Fadenschleppverfahren durch die Kontaktvorrichtung in Form von Faden, Draht, Schwamm, Lederstreifen, Borste oder Textilstück elegant ausgeglichen.

Kugelschreiberverfahren oder Rollverfahren:

Eine bevorzugte Abwandlung des Tropfenschleppverfahrens besteht darin, einen Beschichtungskopf zu nutzen, der kugelförmig ist. Die Kugel hat einen Durchmesser, so dass sie gerade nicht aus der Ausslassöffnung des Beschichtungsbehälters fallen kann. Sie verschliesst dabei das Gefäss vollständig, so dass keine Beschichtungslösung zwischen Kugel und Gefässwand austreten kann. Wird auf diese Kugel durch den Kontakt mit dem zu beschichteten Gegenstand Druck ausgeübt, schiebt sich die Kugel entsprechend dem variabel ausübbaren Druck in den Behälter und die Beschichtungslösung kann zwischen Kugel und Gefässwand des Beschichtungsbehälters austreten. Bei gleichzeitiger Bewegung

entweder des Beschichtungsbehälters oder des zu beschichtenden Gegenstands und gewünschtem Winkel zueinander rollt die Kugel auf der Oberfläche und gewährleistet eine besonders gleichmässige Beschichtung der Oberfläche. Auf diese Art und Weise können die verschiedenartigen Gegenstände formgetreu beschichtet werden, da die Kugel über die Funktion des einstellbaren Druckes und Winkels die Oberfläche wie ein Sensor abrollen kann und damit besonders hohe Variabilität bzgl. der zu beschichtenden Oberflächen und auch der Beschichtungsmöglichkeiten bietet.

Diese Art der Beschichtung ist insbesondere bei Katheterballons sehr gut anwendbar, da jeder Katheterballon eine andere Oberflächenausgestaltung hat, uneben ist und keine Ballonoberfläche der anderen gleicht. Ein vorzugsweise optisch gesteuertes Kugelschreiberbeschichtungsverfahren bietet die Möglichkeit beliebig unterschiedliche und unebene sowie ungleichmäßige Oberflächen gleichmäßig zu beschichten. Ferner bietet der Kugelkopf für die Übertragung der Beschichtungslösung den Vorteil, dass er die Katheterballonoberfläche nicht beschädigt und der Kugelkopf bzw. die Kugel aus einem weichen oder gummiartigen Material wie z.B. Kautschuk hergestellt werden kann, welches noch schonender für die Ballonoberfläche im Vergleich zu einer Metallkugel ist.

Da der Kugelkopf ferner sehr genau platziert werden kann, ergeben sich kontrollierte Start- und Endpunkte für die Beschichtung. Ferner kann die Beschichtungsvorrichtung derart ausgestaltet sein, dass eine dreidimensionale Bewegung möglich ist, so dass der gesamte Katheterballon beschichtet werden kann, ohne den Kugelkopf auch nur einmal abzusetzen oder neu anzusetzen. Nach einem serpentinenartigen Abfahren der zu beschichtenden Ballonoberfläche erreicht der Kugelkopf der Beschichtungsvorrichtung wieder den Startpunkt, wobei inzwischen die anfangs beschichteten Bahnen getrocknet sind und auf die erste Beschichtungsschicht eine weitere aufgetragen werden kann.

Ferner ergibt sich durch die Rollbewegung des Kugelkopfes eine gut kontrollierbare und gleichmäßige Beschichtung, wobei über den auf die Kugel ausgeübten Druck und die Bewegungsgeschwindigkeit die Schichtdicke der Beschichtung gesteuert werden kann.

<u>Rotationstrocknung:</u>

Wie oben erwähnt können die beschichteten oder befüllten Katheterballons nach dem Befüllen oder Beschichten jeder Falte oder nach der Beschichtung oder Befüllung aller Falten oder der zu beschichtenden bzw. zu befüllenden Falten, falls nicht alle Falten beschichtet oder befüllt werden sollen, im rotierenden Zustand getrocknet werden. Dies wird bei den erfindungsgemäßen Verfahren zumeist als Schritt f) angegeben.

Diese Rotationstrocknung hat mehrere Vorteile. Zum einen wird dadurch die wirkstoffenthaltende Zusammensetzung getrocknet und zudem gleichmäßig in den Falten als auch auf der Oberfläche innerhalb der Falten verteilt.

Die Rotationstrocknung ist insbesondere bei öligen oder zähflüssigen wirkstoffenthaltenden Zusammensetzung geeignet, eine gleichmäßige Verteilung der Zusammensetzung in der jeweiligen Falte zu erreichen, wobei diese Beschichtungen in der Regel nicht trocken werden, sondern ihre viskose, ölige, gelartige oder pastöse Konsistenz beibehalten, was auch gewünscht und sehr bevorzugt ist.

Zudem kann bei der Rotation des Katheterballons Vakuum angelegt werden, um eine intensive Trocknung der wirkstoffenthaltenden Zusammensetzung zu erreichen.

Bei der Trocknung im Vakuum treten gerade bei zähflüssigen, hochviskosen oder in den festen Zustand übergehenden Lösungen Siedeverzüge auf, d.h. in dem Öl oder Feststoff eingeschlossene Lösungsmittelreste werden spontan freigesetzt und zerreißen oder sprengen die Beschichtung oder Befüllung. Durch eine Trocknung im Vakuum bei gleichzeitiger Rotation werden diese Siedeverzüge vermieden und es wird eine getrocknete und/oder ölige, viskose, gelartige oder pastenartige gleichmäßige Beschichtung der inneren Oberfläche der Falten erfalten.

Zudem ist die Drehrichtung der Rotation entscheidend. Die Drehrichtung erfolgt in Richtung der Faltenöffnungen, wenn man dies aus dem Inneren der Falte betrachtet. Der Katheterballon wird so wie ein Schaufelrad eines Schaufelradbaggers gedreht, damit die wirkstoffenthaltende Zusammensetzung aufgrund der Rotationskraft in das Falteninnere gedrückt wird.

Vorzugsweise wird der Faltenballon mit einer Rotationsgeschwindigkeit von 50 bis 500, vorzugsweise 150 bis 300 Umdrehungen pro Minute gedreht.

Je nach in die Falten einzubringendem Wirkstoff oder je nach Konsistenz der wirkstoffenthaltenden Zusammensetzung, welche unter die Falten eines Katheterfaltenballons eingebracht werden soll, kann das geeignete erfindungsgemäße Beschichtungsverfahren ausgewählt werden.

Alle erfindungsgemäßen Beschichtungsverfahren, welche gezielt die Beschichtung oder die Befüllung der Falten ermöglichen, sind geeignet, optional zusammen mit dem Rotationstrocknungsverfahren, eine nicht feste sondern ölige, gelartige, pastenartige oder dickviskose Beschichtung oder Befüllung der Falten zu ermöglichen.

Das <u>Faltensprühverfahren</u> eignet sich bevorzugt für dünnviskose bis mittelviskose wirkstoffenthaltende Zusammensetzungen, während das <u>Pipettierverfahren</u> sich bevorzugt für leichtviskose, mittelviskose bis leicht zähviskose Zusammensetzungen eignet und das <u>Spritzverfahren</u> besonders gut für mittelviskose, viskose bis hochviskose Zusammensetzungen einsetzbar ist.

Der Begriff Viskosität bezieht sich auf die dynamische Viskosität [η]:

$$[\eta] = \frac{kg}{m \cdot s} = Pa \cdot s = \frac{Ns}{m^2}$$

Das Spritzverfahren kann vorzugsweise bei dickviskosen Zusammensetzungen eingesetzt werden. Bevorzugt sind Viskositäten bei Raumtemperatur im Bereich von Ölen (Olivenöl: $10^2$ mPa s), Honig ($10^3$ mPa s), Glycerin (1480 mPa s) oder Sirup ($10^5$ mPa s). Selbstverständlich funktioniert dieses Verfahren auch mit dünnviskosen Lösungen mit $\eta \leq 10^2$ mPa s.
Das Pipettierverfahren kann vorzugsweise bei mittelviskosen Zusammensetzungen eingesetzt werden. Bevorzugt sind Viskositäten bei Raumtemperatur im Bereich von vorzugsweise 0,5 mPa s bis 5000 mPa s, weiter bevorzugt im Bereicht von 0,7 mPa s bis 1000 mPa s, noch weiter bevorzugt im Bereicht von 0,9 mPa s bis 200 mPa s und insbesondere bevorzugt im Bereicht von 1,0 mPa s bis 100 mPa s. In diesem Viskositätsbereich liegen Zusammensetzungen aus Ölen, Kontrastmitteln und/oder Salzen, welche mit üblichen Lösungsmitteln insbesondere Alkoholen verdünnt sind. Das Pipettierverfahren kann über einen sehr breiten Viskositätsbereich angewendet werden.
Das Faltensprühverfahren kann vorzugsweise bei dünnviskosen Zusammensetzungen eingesetzt werden. Bevorzugt sind Viskositäten bei Raumtemperatur im Bereich von vorzugsweise 0,1 mPa s bis 400 mPa s, weiter bevorzugt im Bereicht von 0,2 mPa s bis 100 mPa s und insbesondere bevorzugt im Bereicht von 0,3 mPa s bis 50 mPa s (Wasser: 1,0 mPa s; Petrolium: 0,65 mPa s; Pentan: 0,22 mPa s; Hexan: 0,32 mPa s; Heptan: 0,41 mPa s; Oktan: 0,54 mPa s; Nonan: 0,71 mPa s; Chloroform: 0,56 mPa s; Ethanol 1,2 mPa s; Propanol 2,3 mPa s; Isopropanol: 2,43 mPa s; Isobutanol: 3,95 mPa s; Isotridecanol: 42 mPa s).

**Beschichtete Katheterballons**

Gemäß den hierin offenbarten Verfahren lassen sich Katheterballons ohne Stent und teilweise auch mit Stent beschichten, so dass die vorliegende Erfindung beschichtete Katheterballons betrifft, welche gemäß den hierin beschriebenen Verfahren erhalten werden können.
Eine besonders bevorzugte Ausführungsform verwendet einen Katheterballon mit gecrimptem Stent. Bei dem Stent kann es sich um einen unbeschichteten (bare) Stent oder vorzugsweise einen nur mit einer hämokompatiblen Schicht überzogenen Stent handeln. Als hämokompatible Beschichtung sind insbesondere die hierin offenbarten Heparin-Derivate oder Chitosan-Derivate bevorzugt und vorrangig defulfatiertes und reacetyliertes oder repropionyliertes Heparin.
Es besteht zudem die Möglichkeit, dass unter und/oder auf der den Transportvermittler enthaltenden Schicht noch eine oder mehrere Schichten aus einem reinen Wirkstoff oder einem Polymer oder einem wirkstoffenthaltenden Polymer aufgetragen wird/werden.
Bei Verwendung von Faltenballons, welche im komprimierten Zustand Falten bilden, lassen sich diese mit Wirkstoff und Transportvermittler befüllen. Hierzu eignet sich besonders das Pipettierverfahren.
Ein eventuell verwendetes Lösungsmittel kann unter vermindertem Druck entfernt und die sich in den Falten befindende Mischung dadurch getrocknet werden. Bei der Dilatation eines solchen Faltenballons, der in der Regel ohne Stent verwendet wird, kehren oder wölben sich die Falten nach außen und geben dadurch ihren Inhalt an die Gefäßwand ab.
Die erfindungsgemäßen Verfahren sind zur Beschichtung von Führungsdrähten, Spiralen, Katheter, Kanülen, Schläuche sowie allgemein röhrenförmigen Implantaten oder Teilen der vorgenannten Medizinprodukte geeignet, wenn ein mit einem Stent vergleichbares Strukturelement in einem solchen Medizinprodukt enthalten ist, welches beschichtet oder befüllt werden soll. Es lassen sich auch beispielsweise Gefäßstützen und insbesondere Stents wie beispielsweise Koronarstents, Vaskularstents, Tracheastents (Luftröhrenstent), Bronchialstents, Harnröhrenstents, Speiseröhrenstents (Ösophagusstent), Gallenstents, Nierenstents, Dünndarmstents, Dickdarmstents beschichten.
Die beschichteten Medizinprodukte werden insbesondere zur Offenhaltung aller gangartigen Strukturen eingesetzt, beispielsweise von Hamwegen, Speiseröhren, Luftröhre, Gallenwegen, Nierenwegen, Blutgefäßen im gesamten Körper einschließlich Gehirn, Duodenum, Pilorus, Dünn- und Dickdarm aber auch zum Offenhalten künstlicher Ausgänge wie sie für den Darm oder für die Luftröhre verwendet werden.
Somit sind die beschichteten Medizinprodukte zur Verhinderung, Verminderung oder Behandlung von Stenosen, Restenosen, Arteriosklerose, Atherosklerose, und allen anderen Formen des Gefäßverschlusses oder Gefäßverengungen von Durchgängen oder Ausgängen geeignet.
Die erfindungsgemäß beschichteten Katheterballons ohne Stent eignen sich insbesondere zur Behandlung von In-Stent-Restenose, d.h. zur Behandlung einer erneuten Gefäßverengung innerhalb eines bereits implantierten Stent, der vorzugsweise nicht bioresorbierbar ist. Bei derartigen In-Stent-Restenosen ist das Setzen eines weiteren Stent innerhalb eines bereits liegenden Stent besonders problematisch, da das Gefäß in der Regel durch den zweiten Stent nur unzureichend aufgeweitet werden kann. Hier bietet die Wirkstoffapplikation mittels Ballondilatation eine ideale

Behandlungsmöglichkeit, da diese Behandlung mehrmals wiederholt werden kann, falls erforderlich und therapeutisch gesehen dieselben oder deutlich bessere Erfolge erzielen kann als die erneute Stentimplantation.

Ferner sind die erfindungsgemäß beschichteten Katheterballons ohne gekrimpten Stent insbesondere zur Behandlung kleiner Gefäße, vorzugsweise kleiner Blutgefäße geeignet. Als kleine Gefäße werden solche mit einem Gefäßdurchmesser kleiner als 2,5 mm, vorzugsweise kleiner als 2,2 mm bezeichnet.

Zusammenassend gilt für die Nutzung der ausgewählten Matrices und Hilfsmittel:

Die vorgenannten Matrices und Hilfsmittel wie deren Mischungen und Kombinationen besitzen bevorzugt mindestens eine der folgenden Eigenschaften zur erfolgreichen lokalen Applikation eines oder mehrerer Wirkstoffe:

1) die Kontaktzeit des Kurzzeitimplantates reicht zur Übertragung einer geeigneten therapeutischen Wirkstoffmenge in die Zellen aus,
2) während des Kontaktes bleibt genügend wirkstoffenthaltendes Beschichtungsmaterial an der Gefässwand haften, um den gewünschten therapeutischen Effekt zu gewährleisten
und besonders bevorzugt ist,
3) dass die auf dem Kurzzeitimplantat vorhandene wirkstoffenthaltende Beschichtung eine höhere Affinität zur Gefässwand als zur Implantatoberfläche aufweist, so dass eine optimale Übertragung des Wirkstoffes auf den Zielort erfolgen kann. Dies erfolgt for allem bei den pastösen, gelartigen oder öligen Beschichtungen sehr gut.

Selbstverständlich kann in allen Fällen ein beschichteter oder unbeschichteter Stent mit dem Ballonkatheter abhängig vom individuellen Bedarfsfall ein System bilden. Ebenso sind die weiteren Hilfsmittel wie z.B. die bildgebenden Mittel im Bedarfsfall zusetzbar.

Beispielsweise ist die Kontaktzeit bei der besonders bevorzugten Ausführungsform eines mit Paclitaxel im Sprühverfahren beschichteten Ballonkatheters bereits ausreichend, um eine therapeutische Menge des mittels der Sprühtechnik in amorpher Form abgelagerte Paclitaxel an und in die Zellwand zu applizieren. Ein mit dem semisynthetischen Oligosaccharid hämokompatibel gemachter und ebenfalls mit Paclitaxel beschichteter Stent dient hier als Reservoir für eine über einen längeren Zeitraum vorgesehene Elution weiterer Wirkstoffmengen.

Aufgrund der amorphen Konsistenz von Paclitaxel auf dem Stent und Katheterballon, erhalten durch das spezielle Sprühverfahren, wird Paclitaxel während der Einführung des Katheters nicht von der Oberfläche des Ballons gespült oder abgewaschen, so dass die gewünschte Wirkstoffmenge an den Zielort gelangt und hier über die Dilatation an die Gefässwand abgegeben wird. Aufgrund der gleichzeitigen Beschichtung von Stent und Katheterballon erfolgt zudem eine vollständige Gefäßabdeckung mit Wirkstoff. Ferner ist bevorzugt, wenn der Katheterballon auch in dem über die Stentenden hinausgehenden Bereich mit Paclitaxel beschichtet ist, so dass eine Gefaßversorgung mit Paclitaxel (oder anstelle von Paclitaxel auch mit beliebigen anderen Wirkstoffen) auch im Bereich der Stentenden und 1 bis 3 mm in proximaler und distaler Richtung darüber hinaus stattfindet. Auch hier ist die amorphe Struktur von besonderer Wichtigkeit, da nur so die Oberfläche der Wirkstoffschicht derart vergrössert ist, dass eine optimale Menge des Wirkstoffes an der Zellwand haften und in die Zellwand bzw. die Zellen gelangen kann.

Die Zugabe eines direkt auf die Zellwand wirkenden Vasodilators oder leicht membrangängigen Trägers (z.B. DMSO, PETN, Lecithin) kann die Aufnahme in die Zellen während der kumulierten Kontaktzeit von vorzugsweise 30 bis 300 Sekunden noch erheblich steigern.

In einer weiteren besonders bevorzugten Ausführungsform eines wirkstoffeluierenden Ballonkatheters wird der Wirkstoff zusammen mit einer hydrophoben längerkettigen Fettsäure z.B. Isopropylmyristat in einem geeigneten Lösungsmittel gelöst und auf die Ballonkatheteroberfläche aufgebracht. Zur Beschichtung eignen sich alle im folgenden beschriebenen Beschichtungsverfahren. Die Zugabe der Fettsäure ermöglicht die Übertragung von Beschichtungsmaterial von der Katheteroberfläche auf die Gefässwand, wobei die Menge der übertragenen wirkstoffeluierenden Matrix ausreicht, um den Wirkstoff in ausreichender Konzentration zur Verfügung zu stellen wie auch die sofortige Abspülung der Matrix im Blutstrom zu verhindern.

Eine weitere besonders bevorzugte Ausführungsform besteht in der Verwendung einer zur Zellwand hochaffinen Mischung aus dem Polysaccharid Carrageenan, Phosphatidylcholin, eine der Hauptkomponenten von Zellmembranen, als membrangängige Substanz und Glycerin, die aufgrund der sehr guten Haftungseigenschaft eine verzögerte Wirkstoffabgabe von bis zu 12 Stunden nach Dilatation des Gefässes ermöglicht. Alle Beschichtungsverfahren sind für diese Ausführungsform geeignet, besonders bevorzugt werden die hierin beschriebenen Verfahren des Pipettier-, Fadenschleppverfahrens und des Kugelschreiberverfahrens.

**Figurenbeschreibung**

Figur 1 zeigt einen mit Paclitaxel in PEG beschichteren Ballonkatheter (Vergrößerung 80-fach)

Figur 2 zeigt einen mit Paclitaxel in Ethanol beschichteten Ballonkatheter (Vergrößerung 40-fach)
Figur 3 zeigt einen mit Paclitaxel und PVP beschichteten Ballonkatheter nach Expansion (Vergrößerung 80-fach)
Figur 4 zeigt einen 4*20 mm Ballon mit Paclitaxel in Chloroform low dose nach Expansion (Vergrößerung 40-fach)
Figur 5 zeigt eine Beschichtungsvorrichtung gemäß dem Kugelschreiberverfahren, wobei sich im Inneren der Beschichtungsvorrichtung die Beschichtungslösung befindet, welche über eine rotierbare Kugel auf die zu beschichtende Oberfläche abgegeben wird.

**Beispiele**

**Beispiel 1**

Ein für die Hemoxigenase HO-2 kodierendes Gen wird in einen pAH 9-Vektor eingebettet. Die Plasmide werden unter Verwendung von Diethem oder Tetraethern in Lipidvesikel eingelagert. Die erhaltene Emulsion wird mit einem Biopolymer und mit Paclitaxel oder Rapamycin versetzt. Als Biopolymere werden beispielsweise Heparin, Heparansulfate oder Derivate von Heparin oder Heparansulfaten wie beispielsweise desulfatiertes Heparin eingesetzt.
Nach Zugabe des desulfatierten Heparins wird die dünnviskose Mischung zunächst mittels Tauchverfahren auf einen Katheterballon in komprimierter Form aufgetragen. Hierzu wird der Ballon senkrecht in die Tauchlösung eingebracht und so langsam (v < 1 mm/s) aus der Lösung wieder senkrecht herausgezogen, dass sich ein gleichmässiger blasenfreier Film auf der Oberfläche des Katheters bilden kann.
Nach einer kurzen Trockenzeit von max. 30 Minuten werden zur Gewährleistung einer vollständigen Beschichtung und zur optimalen Beladung des Ballonkatheters mit Rapamycin mittels Pipettierverfahren speziell die Falten nachbefüllt. Dazu wird der beschichtete Ballonkatheter auf einen Rotationsmotor mit einem Neigungswinkel von 25° derart angebracht, dass der Ballonkatheter nicht abknicken kann. Die Dosierspritze, die in einer stumpfen Kanüle endet, wird so positioniert, dass sie von dem erhöhten Faltenende derart in die Falte eingeschoben wird und eine definierte Menge der Beschichtungslösung in die Falte abgeben kann.
Nach Befüllen der Falte wird der Ballonkatheter wird nach einer Wartezeit von bis zu 30 sec. um seine Längsachse gedreht, so dass die nächste Falte befüllt werden kann.
Mit Hilfe des Neigungswinkel lässt sich die Kapillarwirkung und Schwerkraft nutzen, um die Falte vollständig oder teilweise je nach gewünschter Rapamycin-Dosierung zu befüllen.
Zum Zeitpunkt der Dilatation des Ballons im Gefäßinneren kommen die Liposomenkomplexe in Kontakt mit der Zellwand und fusionieren mit der lipophilen Zellmembran. In der Zelle transportieren Endosome die Lipoplexe zum Zellkern. Die induzierbare DNA wird nicht in die chromosomale DNA der Zelle eingebaut, sondern bleibt als eigenständige sogenannte episomale Plasmid-DNA im Zellkern aktiv. Ein als Promotor ausgestalteter Abschnitt der Plasmid-DNA startet die Synthese der Hemoxigenase 1, welche dann CO produziert.

**Beispiel 1a)**

Die vollständige und gleichmässige Beschichtung der Falten ist möglich, indem der Ballonkatheter an dem Rotationsmotor derart montiert wird, dass er horizontal und ohne zu Knicken oder Durchhängen befestigt wird. Die zu beschichtende Falte liegt oben, so dass sie nicht zur Seite wegklappen kann.
Nun wird die Beschichtungskanüle so positioniert, dass sie während der Bewegung vom proximalen zum distalem Ende der Falte und umgekehrt, diese derart erfasst, dass sich nur der Teil des Faltenmaterials hebt, der während der Bewegung der Kanüle entlang der Falte gleichzeitig mit Beschichtungslösung befüllt wird.
Auf diese Weise wird eine gleichmässige Verteilung der Beschichtungslösung vom Faltenanfang bis zum Faltenende erreicht.
Die Geschwindigkeit mit der die Kanüle sich entlang der Falte horizontal bewegt und die Eindringtiefe in die Falte sind derart eingestellt, dass die Falte sich gleichmässig nach dem Befüllungsschritt schliesst.
Die Trocknung der derart beschichteten Ballonkatheter erfolgt mittels Rotationstrocknung bei Raumtemperatur.

**Beispiel 2**

Die NO-Synthase III wird analog der Vorschrift gemäß Biochemistry 2002, 30, 41 (30), 9286 - 9292 und MPMI Band 16, Nr. 12, 2003, Seiten 1094 - 11054 rekombinant hergestellt.
Die rekombinante NOS III wird in einem überwiegend wässrigen Medium gelöst. Der wäßrigen Lösung können Cosolventien bis zu 15Vol.-%, bevorzugt bis zu 9Vol.-% zugesetzt werden. Als Cosolventien eignen sich Tetrahydrofuran (THF), Propanol, IsoPropanol, Ethanol, Methanol, Dimethylformamid (DMF), Dimethylsulfamid (DMSO), Aceton oder Essigsäureethylester.
Zu der wäßrigen Lösung mit 10Vol.-% DMSO werden ferner L-Arginin im Überschuß sowie 15 mg Simvastatin

pro ml Lösung gegeben.

Zu der erhaltenen Lösung wird ein biologisch abbaubares Polymer hinzugefügt. Bevorzugte resorbierbare Polymere sind Polymethylmethacrylate (PMMA), Polytetralluoroethylene (PTFE), Polyurethane, Polyvinylchloride (PVC), Polyvinylpyrrolidone, Polyethylenglykole, Polydimethylsiloxane (PDMS), Polyester, Nylons Polyethylenoxid, und Polylactide. Insbesondere bevorzugt sind Polyvinylpyrrolidone, Polyethylenglykole Polyester, Polylactide sowie Copoymere aus Diolen und Estern bzw. Diolen und Lactiden. Als Diole werden beispielsweise Ethan-1,2-diol, Propan-1,3-diol oder Butan-1,4-diol eingesetzt.

Zu der wäßrigen Lösung mit DMSO wird im vorliegenden Fall Polyvinylpyrrolidon und Fasudil gegeben, so dass eine 1 %ige polymerhaltige viskose Lösung entsteht. Ein Katheterballon mit gekrimptem Stent wird mehrmals mit dieser Lösung mit Hilfe des Fadenschleppverfahrens vollständig beschichtet.

Der Ballonkatheter mit aufgecrimpten Stent wird über einen Adapter an die Antriebswelle des Rotationsmotor aufgesteckt und so fixiert, dass er in der Horizontalen ohne Durchknicken zu liegen kommt.

Durch die Dosiernadel und den angeschweißten Schleppdraht wird ein Tropfen Lösung über den rotierenden Ballon geschleppt, bis sich eine zusammenhängende Beschichtung gebildet hat. Anschließend wird der immer noch rotierende Katheter/Stent-System zum Vortrocknen einem leichten warmen Luftstrom ausgesetzt, so dass sich eine hochviskose nicht mehr fliessfähige Oberfläche bildet. Anschliessend wird bei Raumtemperatur getrocknet.

Sowohl Stent als auch Beschichtung können resorbierbar sein und können nach dem Einwachsen in die Zellwand langsam abgebaut werden. Insbesondere während der ersten 10 Tage nach der Implantation stellt die NOS III genügend NO bereit, was den Heilungsprozess der Zellwand sowie das Zellwachstum positiv beeinflußt und reguliert.

**Beispiel 3**

Ein Katheterballon wird mit einer biostabilen Beschichtung aus Cellulosenitrat mittels Tropfenschleppverfahren beschichtet.

Hierzu wird der Katheter derart in den Adapter des Rotationsmotors fixiert, dass er in horizontaler Lage fixiert ist, ohne dass ein Abknicken oder Durchhängen möglich ist. Die Abgabevorrichung ist so über dem Ballon fixiert, dass der Abstand der Pipette, aus der die Beschichtungslösung tritt, gerade so gross ist, dass der austretende Tropfen Kontakt zur Ballonoberfläche erhält, ohne dass er sich von der Pipettenspitze gelöst hat. Die Geschwindigkeit , mit der die Beschichtungslösung ist derart eingestellt, dass der Tropfen während der längsgerichteten Bewegung des Katheterballons nicht abreissen kann. Ist auf diese Weise die oben liegende Fläche des Ballons vollständig beschichtet, wird der Ballon soweit gedreht, dass der Nachbarbereich in gleicher Längsrichtung beschichtet erden kann. Der Vorgang wird solange wiederholt, bis der Ballonkatheter eine vollständige

Umdrehung durchgeführt hat.

Auf diese Schicht wird das Enzym NOS III oder HO-1 immobilisiert, indem es nach der Auftragung mittels Glutardialdehyd quervernetzt wird. Dennoch behält das Enzym ein ausreichendes Maß an Aktivität, um nach der Implantation des Stent CO bzw. NO zu bilden.

Auf diese Schicht wird eine reine Wirkstoffschicht aus Paclitaxel aufgetragen.

Sofern erforderlich kann die Paclitaxel-Wirkstoffschicht mit einer Barriereschicht aus Polylactiden, Polyglykoliden, Polyanhydriden, Polyphosphazenen, Polyorthoestern, Polysacchariden, Polynukleotiden, Polypeptiden, Polyolefinen, Vinylchloridpolymeren, Fluor-enthaltenden Polymeren, Teflon, Polyvinylacetaten, Polyvinylalkoholen, Polyvinylacetalen, Polyacrylaten, Polymethacrylaten, Polystyrol, Polyamiden, Polyimiden, Polyacetalen, Polycarbonaten, Polyestern, Polyurethanen, Polyisocyanaten, Polysilikonen sowie Copolymere und Mischungen dieser Polymere überzogen werden.

**Beispiel 4**

Ein Hämoglobinderivat wird gemäß Ausführungsbeispiel 1 oder 2 von WO 02/00230 A1 hergestellt. Das erhaltene Hämoglobinpolymer wurde in drei Versuchsreihen eingesetzt.

Ein Teil der Hämoglobinpolymere wurde mit CO gesättigt. Ein weiterer Teil wurde mit NO gesättigt und der restliche Teil wurde mit einer Mischung aus CO und NO gesättigt. Danach wurde jedem Teil der Wirkstoff Paclitaxel zugesetzt.

Ein Katheterballon wurde mit einer biostabilen Polymerbeschichtung überzogen. Im vorliegenden Fall wurde als biostabiles Polymer ein Polyvinylester verwendet. Auf diese polymere Schicht wurde dann mittels Sprühverfahren unter CO-Atmosphäre die mit CO gesättigten Hämoglobinpolymere aufgetragen, getrocknet und unter CO-Atmosphäre aufbewahrt.

Die mit NO gesättigten Hämoglobinpolymere wurden zur Beschichtung eines Katheterballons samt gekrimptem Cobalt-Chrom-Stent eingesetzt. Dazu wurden die mit NO gesättigten Hämoglobinpolymere in einer wäßrigen Lösung zusammen mit einem Polylactid vermischt, das Paclitaxel zugesetzt und durch Rollverfahren auf den Ballon samt Stent

aufgetragen, wobei das Roll- und Trocknungsvorgang dreimal wiederholt worden ist. Der Beschichtungsvorgang wurde unter Argon als Inertgas durchgeführt und die beschichteten Katheterballons samt Stents danach unter Argon gelagert.

Der Ballonkatheter mit aufgecrimpten Stent wird in horizontaler Lage fixiert. Die Abgabevorrichtung für die Beschichtungslösung ist derart angebracht, dass sie entlang der Längsrichtung des Katheters und senkrecht dazu beweglich ist. Dabei wird die senkrechte Bewegung über die feste Vorgabe des Druckes auf die Kugel derart kontrolliert, dass der durch den Kontakt mit der zu beschichtenden Oberfläche ausgeübte Druck auf die Kugel des Auslasses immer gleichmässig ausgeübt wird und so immer die gleiche Menge der Beschichtungslösung austritt. Damit wird gewährleistet, dass in der gleichen Zeit immer die gleiche Menge an Beschichtungslösung auf der Oberfläche des Ballonkatheter wie auch des Stents und der Stentzwischenräume aufgebracht wird.

Während der Beschichtung wird die Kugel um den entsprechend dem eingestellten Druck durch den Kontakt mit der Oberfläche so weit eingedrückt, dass Lösung entlang der Kugel

Aus der Öffnung tritt. Durch die gleichzeitige gleichmässige Bewegung des Katheters/Stent in Längsrichtung wird die Kugel bewegt und verteilt mit der Rollbewegung die Beschichtungslösung gleichmässig auf der Oberfläche.

Das Abrollen der Oberfläche wird bei gleichzeitiger leichter Drehbewegung des Katheters um seine Längsachse durchgeführt, so dass die gesamte Oberfläche des Katheters ohne Abbrechen der Rollbewegung des kugelförmigen Auslasses durchgeführt werden kann.

Die mit NO und CO gesättigten Hämoglobinpolymere wurden in einer wäßrigen Lösung zusammen mit einem Polyglykolid und Paclitaxel vermischt und danach als hochviskose Sprühlösung für die gezielte Beschichtung der Falten eines Katheterballons eingesetzt. Hierzu wird der Ballon horizontal fixiert und so gering deflatiert, dass die Falten sich beginnen zu öffnen. Mit Hilfe einer Düse kann nun die Beschichtungslösung in eingestellter Abgabemenge entlang der Falte am Faltengrund eingebracht werden, während sich der Ballonkatheter um seine Längsachse dreht. Da die Beschichtungspaste im Faltengrund klebt, kann der Ballonkatheter sofort nach Befüllen jeder Falte gefahrlos sofort gedreht werden, um die nächste Falte zu befüllen.

Nach Enfernen des leichten Überdruckes können die Falten wieder in ihren Ausgangzustand zurückgebracht werden. Ein Trocknungsvorgang ist in diesem Beispiel nicht vorzunehmen.

### Beispiel 5

Bei einer Ausführungsform der vorliegenden Erfindung wird bei der Dilatation CO oder NO oder ein Gemisch aus CO und NO aus dem Inneren der Katheterballons durch eine Vielzahl von Mikro- oder Nanoporen freigesetzt und unterstützt zum einen bei der Dilatation die Ablösung der sich auf dem Katheterballon befindenden Beschichtung von der Ballonoberfläche als auch die Aufnahme des sich in der Beschichtung auf der Ballonoberfläche befindenden Wirkstoffs in die Gefäßwand als Vasodilator. Auf der Ballonoberfläche befindet sich eine vorzugsweise polymere Beschichtung, welche einen oder mehrere Wirkstoffe enthält, welche einem Wiederverschluss oder einer Wiederverengung des Gefäßes entgegenwirken bzw. dies verhindern.

### Beispiel 6 a

Der Ballonkatheter wird mit einer alkoholischen Lösung aus einem iodhaltigen Kontrastmittel und Paclitaxel (bzw. ein anderer Wirkstoff bzw. Wirkstoffkombination) mittels Fadenschleppverfahren vollflächig beschichtet.

Dazu wird eine 2%ige Kontrastmittellösung hergestellt, in der soviel Paclitaxel gelöst ist, dass eine 30%ige Wirkstofflösung erhalten wird.

Mit dieser Lösung wird der Ballon vollständig und beschichtet und danach in d unter langsamen Drehen um die Längsachse mindestens drei Stunden bei Raumtemperatur getrocknet. Dieser Vorgang wird mindestens einmal wiederholt. Nach vollständigen Trocknen kann der derart Wirkstoff-beschichtete Ballonkatheter mit einer 1%igen PVA-Lösung beispielsweise mit einem Topcoat in gleicher Weise oder mit einem anderen geeigneten Verfahren wie z.B. das Rollverfahren beschichtet werden.

### Beispiel 7a

Der auf Nominaldruck expandierte Faltenballon wird in einer 1%igen Paclitaxel/Chloroform-Tauchlösung für 5-10 s eingetaucht und anschliessend unter Drehen um die Längsachse soweit angetrocknet, dass der Grossteil des Chloroform sich verflüchtigt hat. Vor vollständiger Trocknung wird der Ballon im Luftstrom wieder deflatiert.

### Beispiel 7b

Der Faltenballon wird in horizontaler Lage auf einer drehbaren Achse fixiert, so dass die zu befüllende Falte immer auf der oberen Seite zu liegen kommt. So wird Schritt für Schritt jede Falte mit einer honig- bis sirupähnlich

viskosen (Viskositäten von $10^2$ bis $10^5$ mPa·s) wirkstoffenthaltenden Lösung (z.B. aus Beispiel 17) mit einer vom Faltenanfang zum Faltenende langsam durchgeführten Teflonkanüle als Erweiterung einer Nadelspritze befüllt.
Hierzu wird die Teflonkanüle bis in die Mitte des durch die Falte gebildeten Hohlraums geführt und während der Bewegung des horizontal fixierten Katheters in seine Längsrichtung wird eine definierte Menge der hochviskosen Lösung in den Faltenhohlraum abgegeben (Spritzenverfahren). Die Menge des eingefüllten Materials ist derart limitiert, dass sich die Falte nach dem Befüllen nicht vom Ballonkörper abhebt und varriert entsprechend der unterschiedlichen Ballonmaße und Hersteller.

**Beispiel 7c**

Der in Beispiel 7a wirkstoffbeladene und wieder deflatierte Ballon wie der in Beispiel 7b partiell wirkstoffbeladene Faltenballon lässt sich in einem zweiten Schritt mittels Sprühverfahren mit einer polymeren äusseren Schicht als Barriere überziehen. Dazu muss die Konzentration der polymeren Sprühlösung so gering gehalten werden, dass die nach dem Trocknen erhaltene Polymerschicht die gleichmässige Entfaltung nicht behindert. Beispielsweise ist hier bereits eine 0,5%ige PVP-Lösung geeignet.

**Beispiel 8**

Ein Katheterballon wird mit einer reiner Wirkstoffschicht aus Paclitaxel beschichtet. Nun wird zum Schutz des Wirkstoff vor vorzeitiger Ablösung der Katheterballon mit einer Schützhülle versehen, wie sie bei selbstexpandierenden Nitinolstents angewandt wird. Die Schutzhülle ich in vivo unmittelbar vor der Dilatation entfernbar.

**Beispiel 9**

Eine Lösung aus desulfatiertern Heparin in einem Methanol-Ethanol-Gemisch wird angesetzt und mit Essigsäure angesäuert, so dass sich ein pH-Wert von 3 bis 5 ergibt. Zu dieser Lösung wird Paclitaxel gegeben. Ein Katheterballon wird mit dieser Lösung beschichtet und anschließend erfolgt eine leichte Vernetzung der getrockneten Beschichtung auf dem Ballon mittels Glutaraldehyd.

**Beispiel 10**

Ein herkömmlicher Katheterballon wird vorzugsweise in einem ersten Schritt mit einem Gleitmittel wie z.B. Graphit oder einem Stearat beschichtet und danach vorzugsweise mittels eines Spritzverfahrens mit einer viskosen Mischung eines Öls oder Fetts und eines Wirkstoffs wie z.B. Rapamycin oder Paclitaxel beschichtet. Falls erforderlich kann danach eine geringe Aushärtung durch Autopolymerisation initiiert durch Sauerstoffmoleküle oder durch Strahlung und/oder Radikalbildner erfolgen. Dadurch ergibt sich auf der Katheterballonoberfläche eine glatte Oberfläche, welche in der Regel keines weiteren Schutzes vor frühzeitigen Ablösung bedarf. Der Katheterballon kann in der vorliegenden Form bis zur verengten Gefäßstelle vorgeschoben werden und dort kann die Übertragung der Beschichtung auf die Gefäßwand mittels Dilatation des Ballons durchgeführt werden, wobei das Gleitmittel direkt auf der Ballonoberfläche die Ablösung der öligen Beschichtung unterstützt.

**Beispiel 11**

Magnetische Partikel im Nanometer- bis Mikrometerbereich mit einem eisenhaltigen Kern werden gemäß bekannter Verfahren mit einer Carboxylgruppen-enthaltenden Außenhülle versehen. Zu diesen magnetischen Partikeln in einem Methanol-Ethanol-Lösungsmittelgemisch wird Paclitaxel gegeben und danach die alkoholische Lösung zur Beschichtung des Katheterballons verwendet.
Diese Beschichtungslösung lässt sich aufgrund seiner geringen Viskosität mit dem Sprühverfahren auftragen. Werden vorzugweise die Falten des Ballons mit der Lösung beschichtet eignet sich das Faltensprühverfahren besonders. Wird eine Dosierung über mehrere Düsen gleichzeitig vorgenommen, so dass die Falte über die gesamte Faltenlänge gleichzeitig besprüht wird, kann bei Arbeiten im warmen langsamen Luftstrom bereits eine Vortrockung erfolgen, so dass alle Falten des Ballons in kürzester Zeit beschichtet werden können. Darauf folgt noch die Rotationstrocknung.
Bei der Dilatation des beschichteten Katheterballons wird ein äußeres Magnetfeld angelegt, welches die Magnetpartikel an der stenotisierten Stellt fixiert und dadurch die Aufnahme in die glatten Muskelzellen begünstigt.

**Beispiel 12**

Magnetische Ferritpartikel werden mit einer organischen Hülle versehen, welche den Wirkstoff Paclitaxel enthält. Die Magnetpartikel werden auf einen Katheterballon aufgebracht in dessen Innerem ein Magnetfeld zur Fixierung der Magnetpartikel erzeugt werden kann.

Bei der Dilatation des Katheterballons wird das Magnetfeld umgepolt und führt somit zur Abstoßung der Magnetpartikel von der Ballonoberfläche und zur verstärkten Aufnahme in die glatten Muskelzellen.

**Beispiel 13**

Paclitaxel wird in DMSO gelöst, welches ca. 10Vol.% Wasser enthält. Kaliumoxalat, Natriumchlorid, Glutaminsäure und Oxalsäure werden dieser Lösung zugesetzt und der Katheterballon wird mehrmals mit dieser Lösung unter Anwendung des Fadenschleppverfahrens bestrichen t und nach Streichvorgang getrocknet. Danach wird der beschichtete Katheterballon mit einer biologisch abbaubaren Schicht aus einem Lactam versehen.

**Beispiel 14**

Eine Mischung aus Natriumstearat, Kaliumvalerat, Malonsäure und Paclitaxel in Ethylenglykol, Ethanol, Wasser wird angesetzt, in eine Pipette gefüllt und mittels der Pipette unter die Falten eines Faltenballon gespritzt. Nach der Trocknung erhält man eine pulverige Beschichtung der Faltenzwischenräume, welche sich bei der Dilatation des Ballons leicht ablöst.

**Beispiel 15**

Paclitaxel wird mit Magnesiumsulfat, Kaliumchlorid, Lithiumchlorid und Natriumacetat vermischt und durch Zugabe eines alkoholischen Lösungsmittels und zur Verdünnung eventuell eines Kontrastmittels zu einer Paste verarbeitet, welche dann in eine Spritze gefüllt wird und unter die Falten eines Faltenballon gespritzt wird und dort an der Luft trocknen kann, bis sich eine spröde Beschichtung ergibt. Bei der Beschichtung fährt die Spitze der Spritzdüse entlang der Falte und legt eine Schicht der Paste in die Falte entlang der Faltenlängsrichtung.

**Beispiel 16**

Eine dünnflüssige alkoholische Lösung von Paclitaxel wird angesetzt, welche derart dünnviskos ist, dass sich diese Lösung aufgrund von Kapillarkräften selber in die Falten eines Faltenballons hineinzieht. Mittels einer Kapillare, welche an einem Ende der Falte ansetzt, lässt man die alkoholische Paclitaxellösung in die Falte fließen, bis sich der Falteninnenraum aufgrund von Kapillarkräften vollständig gefüllt hat. Man lässt den Falteninhalt trocknen, dreht den Ballon und befüllt die nächste Falte. Jede Falte wird nur einmal befüllt.

**Beispiel 17**

Eine Mischung aus 70% Leinöl und 30% Olivenöl wird hergestellt. Diese Mischung wird im Mischungsverhältnis 1:1 in Chloroform gelöst und nach Zugabe von Paclitaxel (25Gew.%) auf den gleichmäßig rotierenden Ballonkathetermittels Rollverfahren aufgebracht. Nach Abdampfen des Chloroforms im leichten Luftstrom wird der Ballonkatheter im Trockenschrank bei 70°C gelagert, so dass eine bereits an der Oberfläche haftende aber weiche, hochviskose und damit bei der Expansion des Ballons nicht behindernde Oberfläche bereit gestellt wird.

Beispiel 18

Auf einen Katheterballon aus Polyamid wird ein Cobalt-Chrom-Stent gekrimpt.

Nun wird mittels einer Spritze auf den Stent eine Lösung aus Paclitaxel in DMSO aufgetragen. Die Lösung ist derart dünnviskos, dass sie zwischen die eng anliegenden Stentstreben läuft und die Zwischenräume zwischen Ballonoberfläche und Stentinnenseite sowie zwischen den einzelnen Stentstreben ausfällt. Das Lösungsmittel verdunstet und der reine Wirkstoff lagert sich als Feststoff auf den Katheterballon unter dem Stent, in den Stentzwischenräurnen und auf der Stent sowie Ballonoberfläche ab. Der Katheterballon wird an beiden Enden des Stent ca. 2 bis 3mm über das Stentende hinaus mit dem Wirkstoff beschichtet.

**Beispiel 19**

Eine Lösung aus Rapamycin in Ethanol wird hergestellt und diese Lösung wird auf einen Katheterballon ohne Stent mehrmals aufgesprüht und der Katheterballon zwischendurch getrocknet, indem man das Lösungsmittel verdunsten lässt.

Nach dreimaliger Wiederholung der Sprühbeschichtung wird der Katheterballons letztmalig getrocknet und ein unbeschichteter Stent aus Metall wird auf den Ballon gekrimpt.

**Beispiel 20**

Ein kommerziell erhältlicher Katheterballon wird mit einer Menge von 3 μg Paclitaxel pro $mm^2$ Ballonoberfläche beschichtet. Die Beschichtung erfolgt mittels Pipettierverfahren unter Verwendung einer Lösung von Paclitaxel in DMSO. Die DMSO-Lösung kann des weiteren bis zu 1 mg pro ml an Salzen wie beispielsweise Natriumacetat und vorzugsweise sauren als auch neutralen Aminosäuren enthalten. Auf den beschichteten Katheterballon wird dann ein unbeschichteter Metallstent aus Cobalt-Chrom gekrimpt.

**Beispiel 21**

Ein Katheterballon wird mit gekrimptem unbeschichtetem Metallstent mit einer Lösung aus Paclitaxel in DMSO mit Hilfe des Tropfenschleppverfahrens beschichtet. Der Beschichtungsvorgang wird drei- bis viermal wiederholt, bis erkennbar die Zwischenräume zwischen Ballonoberfläche und Stentinnenseite als auch die Zwischenräume zwischen den einzelnen Stentstreben mit Wirkstoff befüllt sind.
Falls gewünscht, kann nun noch eine Schutzschicht aus beispielsweise einem Polylactid auf die Wirkstoffschicht aus Paclitaxel aufgebracht werden.

**Beispiel 22**

Ein kommerziell erhältlicher Katheterballon wird mit einer Dispersion von Paclitaxel in Essigsäureethylester mit 5 Vol.% Essigsäure beschichtet, so dass eine Menge von 2-3 μg Paclitaxel pro $mm^2$ Ballonoberfläche erhalten wird. Auf die beschichtete Ballonoberfläche wird ein bioresorbierbarer Stent aus Polyhydroxybutyrat gekrimpt.

**Beispiel 23**

Auf einen in den Falten mittels Kapillarverfahren mit Paclitaxel beschichteten Katheterballon, der eine Menge von 1 - 2 μg Paclitaxel pro $mm^2$ Falte aufweist, wird ein Titanstent gekrimpt, der mit einem polymeren Trägersystem aus einem Polyethersulfon enthaltend den Wirkstoff Paclitaxel in einer vorzugsweise cyctostatischen Dosis beschichtet ist. Der Titanstent wurde mittels Pipettierverfahren mit einer Lösung von Paclitaxel und dem Polyethersulfon in Methylenchlorid vorher beschichtet. Auf dem Titanstent befinden sich ca. 0,5 μg Paclitaxel pro $mm^2$ Stentoberfläche.

**Beispiel 24**

Ein mit Rapamycin eingelagert in einem Polylactid-Polyglycolid-Copolymer beschichteter Katheterballon wird bereitgestellt. Auf diesen Katheterballon wird nun ein bioresorbierbarer Stent aus Polylactid gekrimpt, der mit einer Beschichtung aus Polylactid enthaltend Paclitaxel in einer Menge von ca. 1,0 μg Paclitaxel pro $mm^2$ Stentoberfläche beschichtet ist.

**Beispiel 25**

Ein nicht dilatierter Faltenballon wird mit Hilfe des beschriebenen Pipettierverfahrens vollflächig mit einem Wirkstoff und einem Hilfsstoff als Träger beschichtet.
Dazu werden 150mg Sirolimus in 4,5 ml Aceton gelöst und mit einer Lösung aus 100 μl Isopropylmyristat in 450 μl Ethanol vermischt. Nach dem Auftragen der Lösung wird der Faltenballon über Nacht getrocknet.

**Beispiel 26**

Der nach Beispiel 25 beschichete Faltenballon wird in einen mit PBS gefüllten Silikonschlauch eingeführt und darin auf Nominaldruck 60 sec expandiert. Anschliessend wird der auf dem Ballonkatheter verbliebene Sirolimusgehalt, der im PBS-Puffer gelöste Anteil und der an der Schlauchinnenwand haftende Wirkstoffgehalt nach Extraktion mit Acetonitril über HPLC-Messung bestimmt:

| Ermittlung des Sirolimusgehaltes nach Expansion des Faltenballons mittels HPLC-Messung [in %] | | |
|---|---|---|
| Auf Faltenballon | In PBS-Puffer | An Schlauchinnenwand |
| 35,2% | 17,3% | 47,5% |

**Beispiel 27**

Beschichtung eines Katheters mit dem Fadenschleppverfahren

Mit einsetzender Rotation des Katheters wird leichter Unterdruck auf den Ballon gezogen, so dass die Falten bei der Drehbewegung des Ballons um die eigene Längsachse nicht umklappen. Anschließend wird der Ballon mit der Benetzungslösung vorbenetzt. Unmittelbar danach läuft der Beschichtungsvorgang ab. Durch die Dosiernadel und den angeschweißten Schleppdraht wird ein Tropfen Lösung über den Ballon geschleppt bis das Lösungsmittel so weit verdunstet ist, dass sich eine feste Beschichtung gebildet hat.
Nach Beendigung der eingestellten Überstreichungen rotiert der Katheter noch ein paar Sekunden nach. Anschließend wird der Katheter von der Maschine genommen und bei Raumtemperatur getrocknet.

**Beispiel 28**

Kovalente hemokompatible Beschichtung von Stents

Nicht expandierte gereinigte Stents aus medizinischem Edelstahl LVM 316 werden für 5 Minuten in eine 2%ige Lösung von 3-Aminopropyltriethoxysilan in einem Gemisch Ethanol/Wasser (50/50 : (v/v)) getaucht und anschließend getrocknet. Anschließend wurden die Stents über Nacht mit demineralisiertem Wasser gewaschen.
3 mg desulfatiertes und reacetyliertes Heparin wird in 30 ml 0,1M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung werden die Stents über Nacht bei 4°C gerührt. Anschließend wird mit Wasser und 4M NaCl-Lösung ausgiebig gespült.

**Beispiel 29**

Die gereinigten bzw kovalent beschichteten Stents werden auf den Ballonkatheter gecrimpt und gemeinsam mit der wirkstoffhaltigen Sprühlösung mittels Fadenschleppverfahren beschichtet.
Herstellung der Sprühlösung : 44 mg Taxol werden in 6g Chloroform gelöst.

**Beispiel 30**

Beschichtung der hämokompatibel ausgerüsteten Stents mit wirkstoffbeladener Matrix mittels Rollverfahren
Beschichtungslösung : Polylaktid RG502/Taxol - Lösung wird aus 145,2 mg Polylaktid und 48,4 mg Taxol auf 22 g mit Chloroform aufgefüllt.

**Beispiel 31**

Beschichtung des Gesamtsystems Stent + Ballon mit wirkstoffbeladener Matrix als Basiscoat und Wirkstoff als Topcoat
Basiscoat : 19,8 mg Leinöl und 6,6 mg Taxol werden mit Chloroform auf 3 g aufgefüllt Topcoat : 8,8 mg Taxol werden mit Chloroform auf 2 g aufgefüllt.
Der Ballonkatheter mit aufgecrimpten Stent wird mit dem Basiscoat mit Hilfe des Tropfenschleppverfahrens beschichtet. Sobald dieses Basiscoat durch die Verdunstung des Lösungsmittels auf der Systemoberfläche zu einem hochviskosen Film wird, kann die zweite reine Wirkstoffschicht aufgesprüht werden.

**Beispiel 32**

Beschichtung eines Ballonkatheters mit einer zellmembranaffinen wirkstoffenthaltenden Matrix
Der Ballonkatheter wird über einen Adapter an die Antriebswelle des Rotationsmotor aufgesteckt und so fixiert, dass er in der Horizontalen ohne Durchknicken zu liegen kommt. Nachdem leichter Unterdruck auf den Ballon gezogen worden ist, wird der Ballon und der eingestellten Anzahl an Ballonüberstreichungen mit der Lösung beschichtet.

Beschichtungslösung: Carrageenan, Phosphatidylcholin und Glycerin (1:2:2) werden in Ethanot/Wasser (1:1; v:v)) gelöst.

Fadenschleppverfahren :

Durch die Dosiernadel und den angeschweißten Schleppdraht wird ein Tropfen Lösung über den rotierenden Ballon geschleppt bis das Lösungsmittel so weit verdunstet ist, dass sich eine feste Beschichtung gebildet hat. Anschließend wird der Katheter von der Maschine genommen und bei Raumtemperatur und weiterer Rotation über Nacht getrocknet.

## Patentansprüche

1. Verfahren zur Beschichtung eines Katheterballons mit einer definierten Menge eines pharmakologischen Wirkstoffs, wobei das Beschichtungsverfahren eine Beschichtungsvorrichtung mit einer Volumenmesseinrichtung zur Abgabe einer messbaren Menge einer Beschichtungslösung mittels einer Abgabevorrichtung gezielt auf die Oberfläche des Katheterballons verwendet, und wobei das Verfahren ein Fadenschleppverfahren mit einem Faden, einem Netz aus Fäden, einem Stück Textil, einem Lederstreifen oder einem Schwamm als Abgabevorrichtung ist, oder wobei das Verfahren ein Tropfenschleppverfahren mit einer Spritze oder einer Nadel als Abgabevorrichtung ist.

2. Verfahren gemäß Anspruch 1, wobei die Beschichtungslösung einen pharmakologischen Wirkstoff enthält, zusammen mit mindestens einem Transportvermittler, Citratester, Kontrastmittel, Polymer, Polysaccharid, Peptid, Nukleotid, Öl, Fett, Wachs, Fettsäure, Fettsäureester, Hydrogel, Salz, Lösungsmittel, pharmakologisch verträglichen Hilfsstoff oder einer Mischung der vorgenannten Stoffe.

3. Verfahren gemäß Anspruch 1 oder 2, umfassend die folgenden Schritte:

   a) Bereitstellung eines Katheterballons im gefalteten, teilweise inflatierten oder vollständig inflatierten Zustand,
   b) Bereitstellung einer Beschichtungsvorrichtung mit einer Abgabevorrichtung,
   c) Bildung eines Tropfens aus Beschichtungslösung an der Abgabevorrichtung,
   d) Schleppen des Tropfens über die zu beschichtende Oberfläche des Katheterballons, ohne dass die Abgabevorrichtung selbst die Oberfläche des Katheterballons berührt, und
   e) Nachdosieren der Beschichtungslösung, so dass der Tropfen im Wesentlichen seine Größe beibehält.

4. Verfahren gemäß Anspruch 3, wobei gezielt nur die Falten des Katheterballons befüllt werden, indem der Tropfen über die Öffnung der Falte geschleppt wird.

5. Verfahren gemäß Anspruch 3 oder 4, wobei die Oberfläche des Katheterballons vor der Beschichtung mit einem Lösungsmittel benetzt wird.

6. Verfahren gemäß einem der Ansprüche 1 oder 2, umfassend die folgenden Schritte:

   a) Bereitstellung eines Katheterballons im gefalteten, teilweise inflatierten oder vollständig inflatierten Zustand,
   b) Bereitstellung einer Beschichtungsvorrichtung mit einer Abgabevorrichtung in Form eines Fadens, Schwamms, Lederstreifens oder Textilstücks,
   c) Bereitstellung einer Beschichtungslösung,
   d) Tränkung der Abgabevorrichtung mit der Beschichtungslösung,
   e) Übertragung der Beschichtungslösung von der Abgabevorrichtung auf die zu beschichtende Oberfläche des Katheterballons, und
   f) Nachdosieren der Beschichtungslösung, so dass eine gleichmäßige Abgabe der Beschichtungslösung von der Abgabevorrichtung auf die zu beschichtende Oberfläche des Katheterballons erfolgt.

7. Verfahren gemäß Anspruch 6, wobei die Übertragung der Beschichtungslösung von der Abgabevorrichtung auf die zu beschichtende Oberfläche des Katheterballons gemäß Schritt e) durch das Schleppen der Abgabevorrichtung über die zu beschichtende Oberfläche des Katheterballons erfolgt.

8. Verfahren gemäß Anspruch 6 oder 7, wobei gezielt nur die Falten des Katheterballons befüllt werden, indem die Abgabevorrichtung über die Faltenöffnung oder durch die Falten geschleppt wird.

**9.** Verfahren gemäß einem der Ansprüche 6 - 8, wobei die Oberfläche des Katheterballons vor dem Beschichten mit einem Lösungsmittel benetzt wird.

**10.** Verfahren gemäß einem der Ansprüche 6 - 9, wobei die Abgabevorrichtung aus einem Material besteht, welches den Katheterballon nicht beschädigen kann.

**11.** Verfahren gemäß einem der Ansprüche 6 - 10, wobei alle Falten des Katheterballons gleichzeitig beschichtet oder befüllt werden.

**12.** Verfahren gemäß einem der Ansprüche 6 - 11, wobei die gesamte Oberfläche des Katheterballons gleichzeitig mit einer Vielzahl von Fäden, Lederstreifen, einem Stück Textil oder einem Netz aus Fäden beschichtet wird.

## Claims

**1.** Method for coating a catheter balloon with a defined amount of a pharmacologically active agent, wherein the coating method uses a coating device with a volume measuring device for releasing a measurable amount of a coating solution by means of a dispensing device specifically to the surface of the catheter balloon and wherein the method is a thread drag method with a thread, a mesh of threads, a piece of textile, a leather strip, or a sponge as dispensing device or wherein the method is a drop-drag method with a syringe or a needle as dispensing device.

**2.** Method according to claim 1, wherein the coating solution contains a pharmacologically active agent together with at least one transport mediator, citrate ester, contrast medium, polymer, polysaccharide, peptide, nucleotide, oil, fat, wax, fatty acid, fatty acid ester, hydrogel, salt, solvent, pharmacologically acceptable excipient or a mixture of the aforementioned substances.

**3.** Method according to claim 1 or 2, comprising the following steps:

a) providing a catheter balloon in a folded, partially inflated or completely inflated state,
b) providing a coating device with a dispensing device,
c) forming of a drop of the coating solution at the dispensing device,
d) dragging the drop over the surface to be coated of the catheter balloon without the dispensing device itself contacting the surface of the catheter balloon, and
e) redosing of the coating solution so that the drop substantially maintains its size.

**4.** Method according to claim 3, wherein specifically only the folds of the catheter balloon are filled by dragging the drop over the opening of the fold.

**5.** Method according to claim 3 or 4, wherein the surface of the catheter balloon is bedewed before coating with a solvent.

**6.** Method according to claim 1 or 2, comprising the following steps:

a) providing a catheter balloon in a folded, partially inflated or completely inflated state,
b) providing a coating device with a dispensing device in form of a thread, sponge, leather strip or piece of textile,
c) providing a coating solution,
d) soaking the dispensing device with the coating solution,
e) transferring the coating solution from the dispensing device onto the surface to be coated of the catheter balloon, and
f) redosing of the coating solution so that a consistent dispense of the coating solution from the dispensing device onto the surface to be coated of the catheter balloon occurs.

**7.** Method according to claim 6, wherein the transfer of the coating solution from the dispensing device onto the surface to be coated of the catheter balloon is effected according to step e) by dragging the dispensing device over the surface to be coated of the catheter balloon.

**8.** Method according to claim 6 or 7, wherein specifically only the folds of the catheter balloon are filled by dragging the dispensing device over the opening of the fold or through the folds.

9. Method according to one of the claims 6 - 8, wherein the surface of the catheter balloon is bedewed with a solvent before coating.

10. Method according to one of the claims 6 - 9, wherein the dispensing device consists of a material that does not damage the catheter balloon.

11. Method according to one of the claims 6 - 10, wherein all folds of the catheter balloon are coated or filled concomitantly.

12. Method according to one of the claims 6 - 11, wherein the complete surface of the catheter balloon is coated concomitantly by a plurality of threads, leather strips, a piece of textile or a mesh of threads.

**Revendications**

1. Procédé de revêtement d'un ballonnet de cathéter avec une quantité définie d'un principe actif pharmacologique, dans lequel le procédé de revêtement utilise un dispositif de revêtement avec un dispositif de métrage de volume pour le déchargement d'une quantité mesurable d'une solution de revêtement moyennant un dispositif de déchargement de manière ciblée sur la surface d'un ballonnet de cathéter, et dans lequel le procédé est un procédé de traînage de fil avec un fil, un filet de fils, une pièce de textile, un bracelet en cuir ou une éponge comme dispositif de déchargement, ou dans lequel le procédé est un procédé de traînage de goutte avec une seringue ou un épingle comme dispositif de déchargement.

2. Procédé selon la revendication 1, dans lequel la solution de revêtement contient un principe actif pharmacologique avec au moins un médiateur de transport, ester citrique, moyen de contraste, polymère, polysaccharide, peptide, nucléotide, huile, graisse, ester d'acide gras, hydrogel, sel, solvant, excipient pharmacologiquement acceptable ou une mélange des matières susmentionnées.

3. Procédé selon la revendication 1 ou 2, comprenant les pas suivants:

   a) provisionner un ballonnet de cathéter dans l'état plié, partiellement gonflé ou complètement gonflé,
   b) provisionner un dispositif de revêtement avec un dispositif de déchargement,
   c) former une goutte de la solution de revêtement au dispositif de déchargement,
   d) traîner la goutte sur la surface à revêtir du ballonnet de cathéter sans que le dispositif de déchargement même touche la surface du ballonnet de cathéter, et
   e) redoser la solution de revêtement de manière que la goutte substantiellement maintienne sa taille.

4. Procédé selon la revendication 3, dans lequel seulement les plis du ballonnet de cathéter sont remplis en traînant la goutte sur l'aperture du pli.

5. Procédé selon la revendication 3 ou 4, dans lequel la surface du ballonnet de cathéter est mouillée avec un solvant avant le revêtement.

6. Procédé selon la revendication 1 ou 2, comprenant les pas suivants:

   a) provisionner un ballonnet de cathéter dans l'état plié, partiellement gonflé ou complètement gonflé,
   b) provisionner un dispositif de revêtement avec un dispositif de déchargement en forme d'un fil, éponge, bracelet en cuir ou pièce de textile,
   c) provisionner une solution de revêtement,
   d) imprégner le dispositif de déchargement avec la solution de revêtement,
   e) transférer la solution de revêtement du dispositif de revêtement sur la surface à revêtir du ballonnet de cathéter, et
   f) redoser la solution de revêtement de manière que s'effectue un déchargement régulier de la solution de revêtement du dispositif de revêtement sur la surface à revêtir du ballonnet de cathéter.

7. Procédé selon la revendication 6, dans lequel le transfert de la solution de revêtement du dispositif de revêtement sur la surface à revêtir du ballonnet de cathéter s'effectue selon le pas e) en traînant le dispositif de déchargement sur la surface à revêtir du ballonnet de cathéter.

**8.** Procédé selon la revendication 6 ou 7, dans lequel de manière ciblée seulement les plis du ballonnet de cathéter sont remplis en traînant le dispositif de déchargement sur les apertures des plis ou au travers des plis.

**9.** Procédé selon une des revendications 6 - 8, dans lequel la surface du ballonnet de cathéter est mouillée avec un solvant avant le revêtement.

**10.** Procédé selon une des revendications 6 - 9, dans lequel le dispositif de déchargement consiste d'un matériau qui ne peut pas endommager le ballonnet de cathéter.

**11.** Procédé selon une des revendications 6 - 10, dans lequel tous les plis du ballonnet de cathéter sont revêtus ou remplis au même temps.

**12.** Procédé selon une des revendications 6 - 11, dans lequel l'entière surface du ballonnet de cathéter est revêtue au même temps avec une pluralité de fils, bracelets en cuir, une pièce de textile ou un filet de fils.

Figur 1 :

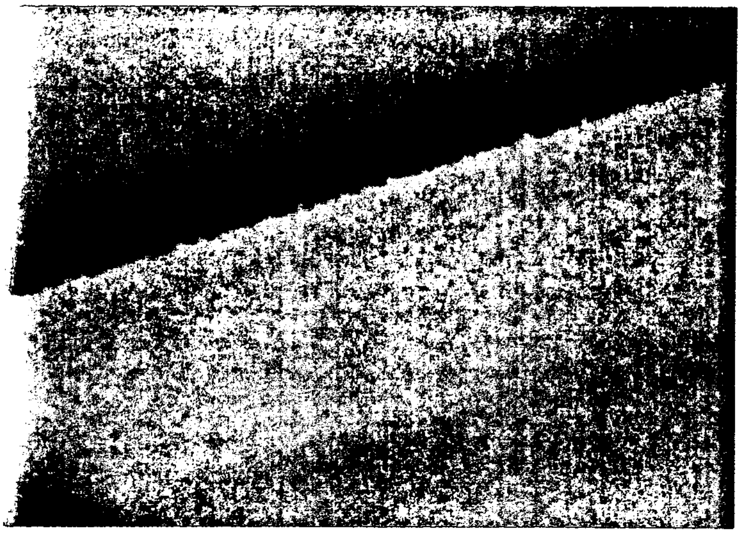

Figur 2 :

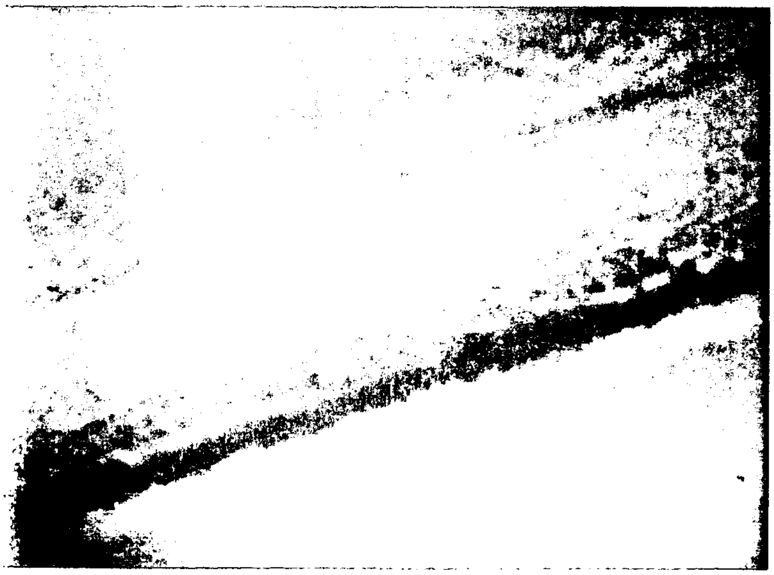

Figur 3 :

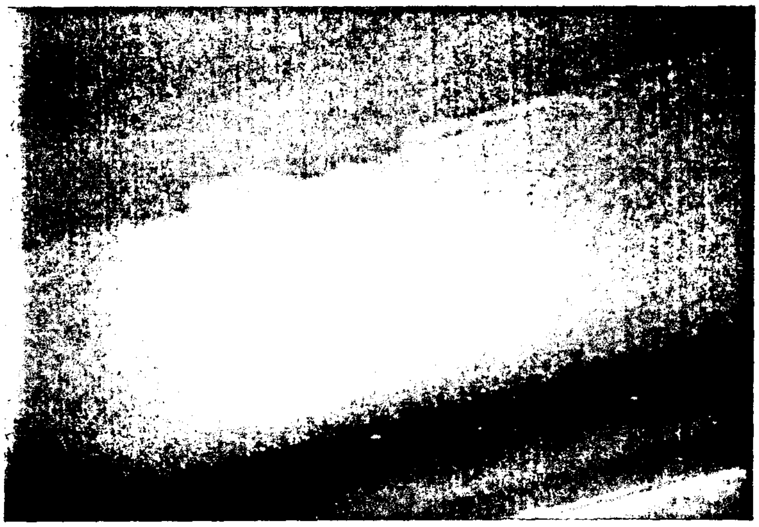

Figur 4 :

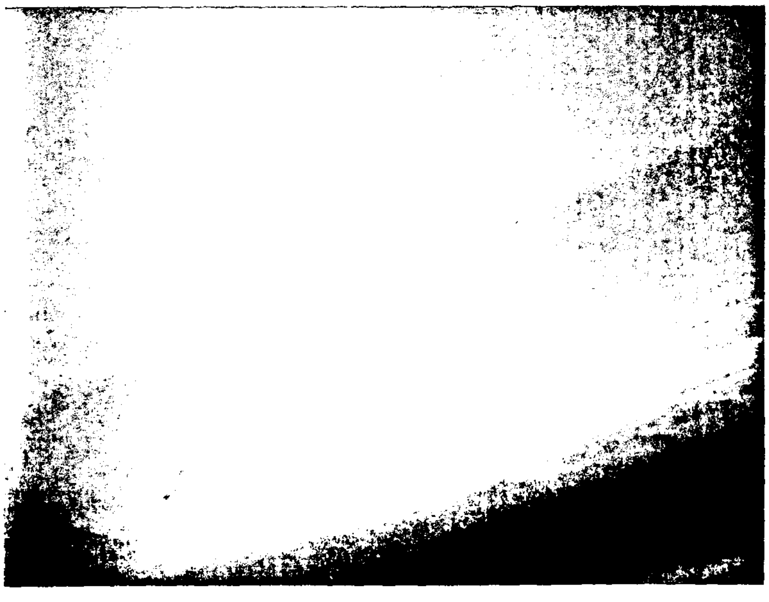

Figur 5

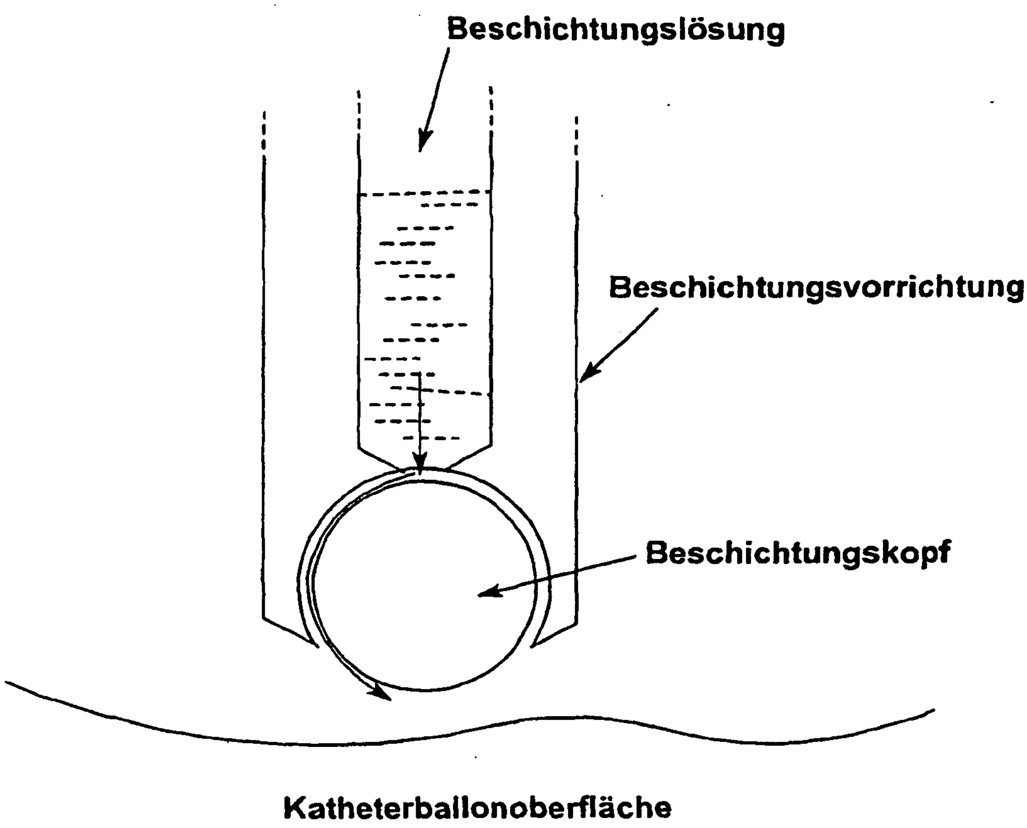
Beschichtungslösung
Beschichtungsvorrichtung
Beschichtungskopf
Katheterballonoberfläche

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5087244 A
- US 4994033 A
- US 4186745 A
- EP 0383429 A
- WO 2004028582 A1
- EP 1189553 B1
- EP 0519063 B1
- WO 03059430 A1
- WO 94123787 A1
- WO 02043796 A2
- WO 03026718 A1
- EP 1419793 B1
- DE 19856983 A1
- EP 0966979 A2
- US 6548569 B1
- US 5935506 A
- US 6623749 B2
- US 6838493 B2
- US 6867247 B2
- US 6245103 B1
- US 6991647 B2
- EP 1152778 B1
- EP 1501565 B1
- WO 03022265 A1
- DE 19726282 A
- DE 19726282
- DE 19912798 C1
- DE 10059151 A
- DE 19614136 A
- DE 19515820 A
- DE 4428851 A1
- EP 0516252 A2
- WO 9858673 A
- WO 0200230 A1


### In der Beschreibung aufgeführte Nicht-Patentliteratur


- *CardioNews Letter,* 21. April 2006
- *Wirkstoffliste,* 6-9
- *Biochemistry,* 2002, vol. 30, 41, 9286-9292
- *MPMI,* 2003, vol. 16 (12), 1094-11054